# EUROPÄISCHE PATENTSCHRIFT

(11) **EP 3 573 985 B1**
(45) Veröffentlichungstag und Bekanntmachung des Hinweises auf die Patenterteilung: **18.01.2023**
(21) Anmeldenummer: 18701051.7
(22) Anmeldetag: 29.01.2018
(51) Int. Cl.: C07D 471/04, C07D 471/14, H01L 51/00

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 30.01.2017 EP 17153706
(43) Veröffentlichungstag der Anmeldung: 04.12.2019
(73) Patentinhaber: Merck Patent GmbH, 64293 Darmstadt (DE)
(72) Erfinder: PARHAM, Amir, 60486 Frankfurt Am Main (DE); KROEBER, Jonas, 60311 Frankfurt Am Main (DE); JOOSTEN, Dominik, 60487 Frankfurt Am Main (DE); LUDEMANN, Aurélie, 60322 Frankfurt Am Main (DE); GROSSMANN, Tobias, 64297 Darmstadt (DE)
(86) Internationale Anmeldenummer: PCT/EP2018/052057
(87) Internationale Veröffentlichungsnummer: WO 2018/138306

(56) Entgegenhaltungen:
- CN-A- 103 183 638
- CN-A- 103 664 894
- CN-B- 103 589 420
- CN-B- 103 589 421
- CN-B- 103 614 133
- CN-B- 103 614 134
- KR-A- 20120 081 539
- US-A1- 2010 032 658
- Yulian Zagranyarski ET AL: "Supplementary Information Facile Synthesis of Annulated Heterocyclic Benzo[kl]acridine Derivatives via One-pot N-H/C- H Coupling Electronic Supplementary Material (ESI) for Organic Chemistry Frontiers. This journal is the Partner Organisations 2016", , 19. Mai 2016 (2016-05-19), XP055458771, Gefunden im Internet: URL:http://www.rsc.org/suppdata/c6/qo/c6qo 00371k/c6qo00371k1.pdf [gefunden am 2018-03-13]

## Beschreibung

Die vorliegende Erfindung betrifft Materialien für die Verwendung in elektronischen Vorrichtungen, insbesondere in organischen Elektrolumineszenzvorrichtungen, sowie elektronische Vorrichtungen, insbesondere organische Elektrolumineszenzvorrichtungen enthaltend diese Materialien.

In organischen Elektrolumineszenzvorrichtungen (OLEDs) werden als emittierende Materialien häufig metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Triplettemission (Phosphoreszenz) zeigen, immer noch Verbesserungsbedarf, beispielsweise im Hinblick auf Effizienz, Betriebsspannung und Lebensdauer. Die Eigenschaften phosphoreszierender OLEDs werden nicht nur von den eingesetzten Triplettemittern bestimmt. Hier sind insbesondere auch die anderen verwendeten Materialien, wie Matrixmaterialien, von besonderer Bedeutung. Verbesserungen dieser Materialien und deren Ladungstransporteigenschaften können somit auch zu deutlichen Verbesserungen der OLED-Eigenschaften führen.

Aufgabe der vorliegenden Erfindung ist die Bereitstellung von Verbindungen, welche sich für den Einsatz in einer OLED eignen, insbesondere als Matrixmaterial für phosphoreszierende Emitter. Eine weitere Aufgabe der vorliegenden Erfindung ist es, weitere organische Halbleiter für organische Elektrolumineszenzvorrichtungen bereitzustellen, um so dem Fachmann eine größere Wahlmöglichkeit an Materialien für die Herstellung von OLEDs zu ermöglichen.

Überraschend wurde gefunden, dass bestimmte, unten näher beschriebene Verbindungen diese Aufgabe lösen, sich gut für die Verwendung in OLEDs eignen und zu Verbesserungen der organischen Elektrolumineszenzvorrichtung führen, insbesondere in Bezug auf die Lebensdauer, die Effizienz und die Betriebsspannung. Diese Verbindungen sowie elektronische Vorrichtungen, insbesondere organische Elektrolumineszenzvorrichtungen, welche derartige Verbindungen enthalten, sind daher der Gegenstand der vorliegenden Erfindung.

Aus WO 2010/104047 sind N-Aryl-azabenzoanthracenderivate bekannt, welche zwischen der am Stickstoff gebundenen Arylgruppe und dem Azabenzoanthracen-Grundgerüst eine Einfachbindung aufweisen. Verbindungen gemäß der vorliegenden Erfindung sind nicht offenbart. In Y. Zagranyarski et al. "Facile Synthesesis of Annulated Heterocyclic Benzo[kl]acridine Derivatives via One-pot N-H/C-H Coupling", Org. Chem. Front., 2016, 3, 1520-1523 (DOI: 10.1039/c6qo00371k) ist die Synthese von zwei heterocyclischen Verbindungen beschrieben, die von der Erfindung ausgenommen sind. Es wird nicht deren Verwendung in Elektrolumineszenzvorrichtungen beschrieben. US 2010/032658, CN 103 664 894, CN 103 614 134, CN 103 614 133, CN 103 589 421, CN 103 589 420, CN 103 183 638 und KR 2012 0081539 offenbaren kondensierte Polyzyklen, welche in Elektrolumineszenzvorrichtungen eingesetzt werden.

Gegenstand der vorliegenden Erfindung ist eine Verbindung gemäß Formel (1), 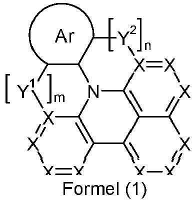 wobei für die verwendeten Symbole und Indizes gilt:
- X: ist bei jedem Auftreten gleich oder verschieden CR oder N, wobei X = C ist, wenn an dieses X eine Gruppe Y¹ bzw. Y² gebunden ist, mit der Maßgabe, dass kein, ein oder zwei Symbole X für N stehen;
- Ar: ist zusammen mit den explizit eingezeichneten Kohlenstoffatomen eine Aryl- oder Heteroarylgruppe mit 5 bis 14 aromatischen Ring-atomen, die durch einen oder mehrere Reste R substituiert sein kann;
- Y¹: ist C(R')₂, NR', O, S oder BR';
- Y²: ist eine Einfachbindung, C(R')₂, C(=C(R")₂), NR', O, S oder BR';
- R: ist bei jedem Auftreten gleich oder verschieden H, D, F, N(Ar')₂, CN, OR¹, eine geradkettige Alkylgruppe mit 1 bis 10 C-Atomen oder eine Alkenylgruppe mit 2 bis 10 C-Atomen oder eine verzweigte oder cyclische Alkylgruppe mit 3 bis 10 C-Atomen, wobei die Alkyl oder Alkenylgruppe jeweils mit einem oder mehreren Resten R¹ substituiert sein kann und wobei eine oder mehrere nicht benachbarte CH₂-Gruppen durch O ersetzt sein können, oder ein aromatisches oder heteroaromatisches Ringsystem mit 6 bis 30 aromatischen Ring-atomen, das jeweils durch einen oder mehrere Reste R¹ substituiert sein kann;
- R': ist für Y¹ bzw. Y² = NR' ein aromatisches oder heteroaromatisches Ringsystem mit 6 bis 24 aromatischen Ringatomen, das durch einen oder mehrere nicht-aromatische Reste R¹ substituiert sein kann; und ist für Y¹ bzw. Y² = C(R')₂ bei jedem Auftreten gleich oder verschieden eine geradkettige Alkylgruppe mit 1 bis 6 C-Atomen oder eine verzweigte oder cyclische Alkylgruppe mit 3 bis 6 C-Atomen, oder ein aromatisches oder heteroaromatisches Ringsystem mit 6 bis 24 aromatischen Ringatomen, das jeweils durch einen oder mehrere Reste R¹ substituiert sein kann; dabei können zwei Reste R' auch miteinander ein aromatisches oder aliphatisches Ringsystem bilden;
- R": ist für Y² = C(=C(R")₂) einmal H und das andere R" steht für CR¹=CR¹ und bildet zusammen mit Ar ein aromatisches oder heteroaromatisches Ringsystem;
- Ar': ist bei jedem Auftreten gleich oder verschieden ein aromatisches oder heteroaromatisches Ringsystem mit 5 bis 40 aromatischen Ring-atomen, das durch einen oder mehrere Reste R¹ substituiert sein kann;
- R¹: ist bei jedem Auftreten gleich oder verschieden H, D, F, CN, OR², eine geradkettige Alkylgruppe mit 1 bis 10 C-Atomen oder eine Alkenylgruppe mit 2 bis 10 C-Atomen oder eine verzweigte oder cyclische Alkylgruppe mit 3 bis 10 C-Atomen, wobei die Alkyl- oder Alkenylgruppe jeweils mit einem oder mehreren Resten R² substituiert sein kann, wobei eine oder mehrere nicht benachbarte CH₂-Gruppen durch O ersetzt sein können, oder ein aromatisches oder heteroaromatisches Ringsystem mit 6 bis 30 aromatischen Ring-atomen, das jeweils durch einen oder mehrere Reste R² substituiert sein kann;
- R²: ist bei jedem Auftreten gleich oder verschieden H, eine Alkylgruppe mit 1 bis 4 C-Atomen oder eine Arylgruppe mit 6 bis 10 C-Atomen, die mit einer Alkylgruppe mit 1 bis 4 C-Atomen substituiert sein kann;
- m, n: ist unabhängig voneinander 0 oder 1 mit der Maßgabe, dass m + n = 1 ist; dabei bedeutet m = 0, dass die Gruppe Y¹ nicht vorhanden ist und an das Kohlenstoffatom in Ar, an das Y¹ gebunden wäre, ein Rest R gebunden ist; weiterhin bedeutet n = 0, dass die Gruppe Y² nicht vorhanden ist und an das Kohlenstoffatom in Ar, an das Y² gebunden wäre, ein Rest R gebunden ist;
dabei sind die folgenden Verbindungen von der Erfindung ausgenommen:

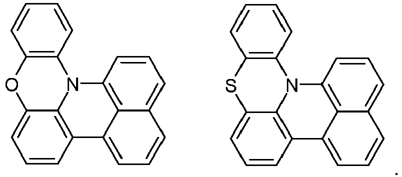

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte (anellierte) Aryl- oder Heteroarylgruppe, beispielsweise Naphthalin, Anthracen, Phenanthren, Chinolin, Isochinolin, etc., verstanden. Miteinander durch Einfachbindung verknüpfte Aromaten, wie zum Beispiel Biphenyl, werden dagegen nicht als Aryl- oder Heteroarylgruppe, sondern als aromatisches Ringsystem bezeichnet.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit, wie z. B. ein C-, N- oder O-Atom, verbunden sein können. So sollen beispielsweise auch Systeme wie Fluoren, 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine kurze Alkylgruppe verbunden sind. Bevorzugt ist das aromatische Ringsystem gewählt aus Fluoren, 9,9'-Spirobifluoren, 9,9-Diarylamin oder Gruppen, in denen zwei oder mehr Aryl- und/oder Heteroarylgruppen durch Einfachbindungen miteinander verknüpft sind.

Im Rahmen der vorliegenden Erfindung werden unter einem aliphatischen Kohlenwasserstoffrest bzw. einer Alkylgruppe bzw. einer Alkenyl- oder Alkinylgruppe, die 1 bis 40 C-Atome enthalten kann, und in der auch einzelne H-Atome oder CH₂-Gruppen durch die oben genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, neo-Pentyl, Cyclopentyl, n-Hexyl, neo-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl, Heptinyl oder Octinyl verstanden. Unter einer Alkoxygruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy und 2,2,2-Trifluorethoxy verstanden. Unter einer Thioalkylgruppe mit 1 bis 40 C-Atomen werden insbesondere Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden. Allgemein können Alkyl-, Alkoxy- oder Thioalkylgruppen gemäß der vorliegenden Erfindung geradkettig, verzweigt oder cyclisch sein, wobei eine oder mehrere nicht-benachbarte CH₂-Gruppen durch die oben genannten Gruppen ersetzt sein können; weiterhin können auch ein oder mehrere H-Atome durch D, F, Cl, Br, I, CN oder NO₂, bevorzugt F, Cl oder CN, weiter bevorzugt F oder CN, besonders bevorzugt CN ersetzt sein.

Unter einem aromatischen oder heteroaromatischen Ringsystem mit 5 - 60 aromatischen Ringatomen, welches noch jeweils mit den oben genannten Resten substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Pyren, Chrysen, Perylen, Fluoranthen, Naphthacen, Pentacen, Benzpyren, Biphenyl, Biphenylen, Terphenyl, Triphenylen, Fluoren, Spirobifluoren, Dihydrophenanthren, Dihydropyren, Tetrahydropyren, cis- oder trans-Indenofluoren, cis- oder trans-Indenocarbazol, cis- oder trans-Indolocarbazol, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Hexaazatriphenylen, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Gruppen, die abgeleitet sind von Kombinationen dieser Systeme.

Wenn zwei Reste R bzw. R' bzw. R¹ miteinander ein Ringsystem bilden, so kann dieses mono- oder polycyclisch sein. Dabei sind die Reste, die miteinander ein Ringsystem bilden, bevorzugt benachbart, d. h. dass diese Reste an dasselbe Kohlenstoffatom oder an Kohlenstoffatome, die direkt aneinander gebunden sind, gebunden sind. Wenn R" für CR¹=CR¹ steht und mit Ar ein aromatisches oder heteroaromatisches Ringsystem bildet, so entsteht durch den so gebildeten Cyclus eine an Ar ankondensierte Aryl- bzw. Heteroarylgruppe, wie weiter unten ausführlicher dargestellt ist.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

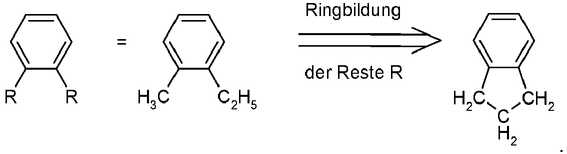

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

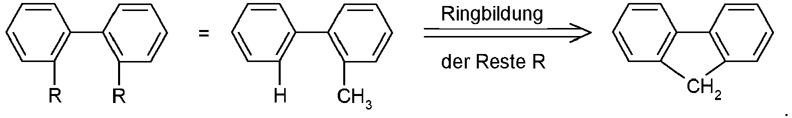

In einer bevorzugten Ausführungsform der Erfindung ist m = 1 und n = 0, und Y¹ steht für NR', O oder S, wobei R' die oben genannten Bedeutungen aufweist.

In einer weiteren bevorzugten Ausführungsform der Erfindung ist n = 1 und m = 0, und Y² steht für eine Einfachbindung, NR', O, S oder für C(=C(R")₂), wobei eine Gruppe R" für H steht und die andere Gruppe R" für CR¹=CR¹ steht und mit Ar eine kondensierte Arylgruppe bildet, und besonders bevorzugt für eine Einfachbindung, NR', O oder S.

Besonders bevorzugt ist n = 1 und m = 0.

Bevorzugt handelt es sich somit für m = 1 um eine Verbindung gemäß einer der folgenden Formeln (2a), (2b) oder (2c) und für n = 1 um eine Verbindung gemäß einer der folgenden Formeln (2d), (2e), (2f), (2g) oder (2h), 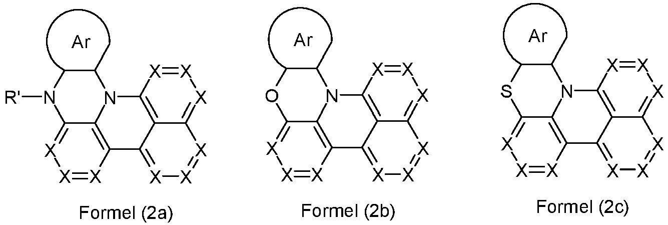 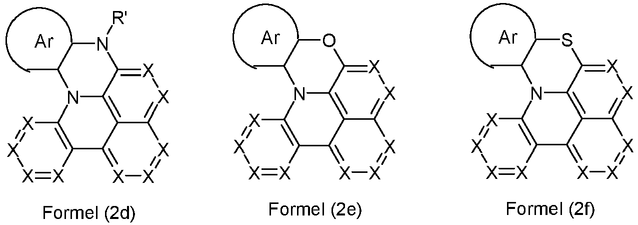 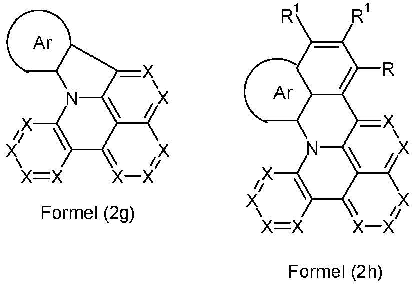 wobei die verwendeten Symbole die oben genannten Bedeutungen aufweisen.

In einer weiteren bevorzugten Ausführungsform der Erfindung steht Ar in Formel (1) und (2a) bis (2h) für eine Gruppe gemäß einer der folgenden Formeln (Ar-a), (Ar-b) oder (Ar-c), 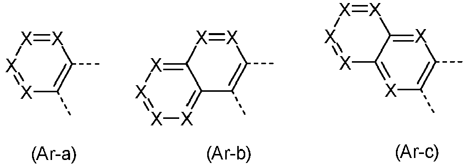 wobei eine der gestrichelten Bindungen die Bindung an das Stickstoffatom darstellt, die andere der gestrichelten Bindungen die Bindung an Y¹ bzw. Y² darstellt und X die oben genannten Bedeutungen aufweist. Dabei stehen bevorzugt maximal zwei Symbole X pro Gruppe Ar für N, und die restlichen Symbole X stehen gleich oder verschieden bei jedem Auftreten für CR. Besonders bevorzugt steht maximal ein Symbol X für N, und die restlichen Symbole X stehen gleich oder verschieden bei jedem Auftreten für CR, und ganz besonders bevorzugt stehen alle Symbole X gleich oder verschieden bei jedem Auftreten für CR.

Bevorzugt ist Ar eine Gruppe der Formel (Ar-a), insbesondere eine Gruppe der Formel (Ar-a), in der alle Symbole X für CR stehen. Bevorzugt sind die Verbindungen der Formeln (1) daher gewählt aus den Verbindungen der folgenden Formel (3), 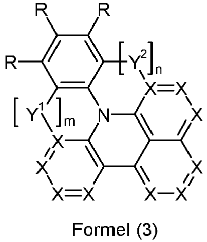 wobei die verwendeten Symbole und Indizes die oben genannten Bedeutungen aufweisen. Dabei ist an den Phenylring, der die Gruppe Ar darstellt, noch ein Rest R an das Kohlenstoffatom gebunden, wenn in dieser Position keine Gruppe Y¹ bzw. Y² gebunden ist.

Entsprechend sind die Verbindungen der Formeln (2a) bis (2h) bevorzugt gewählt aus den Strukturen der folgenden Formeln (4a) bis (4h), 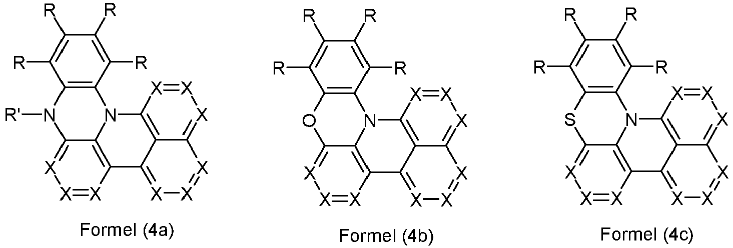 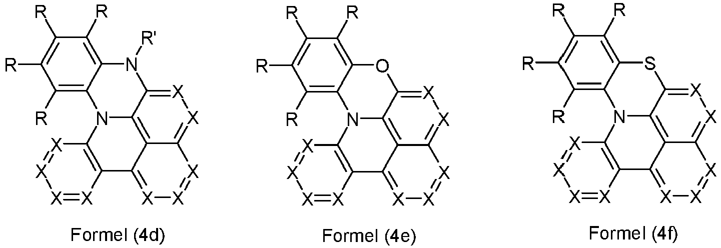 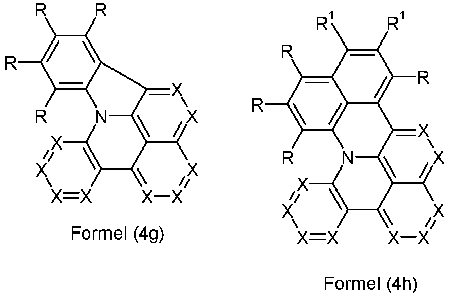 wobei die verwendeten Symbole die oben genannten Bedeutungen aufweisen.

In einer weiteren bevorzugten Ausführungsform enthalten Verbindungen der Formel (1) bzw. der oben genannten bevorzugten Ausführungsformen kein oder ein Symbol X im Grundgerüst für N. Ganz besonders bevorzugt stehen alle Symbole X im Grundgerüst für CR. Der Begriff "Grundgerüst" bezeichnet dabei in Formel (1) den Teil der Struktur, der die Symbole X enthält, ohne die Gruppe Ar. Dies gilt analog für die oben aufgeführten bevorzugten Strukturen.

Bevorzugte Verbindungen der Formel (1) sind für m = 1 die Verbindungen der folgenden Formeln (5a) bis (5k) und für n = 1 die Verbindungen der folgenden Formeln (5l) bis (5u), 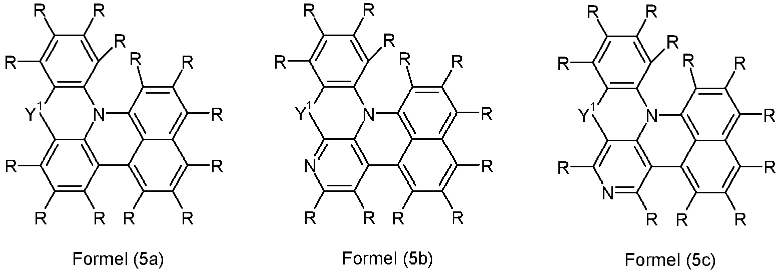 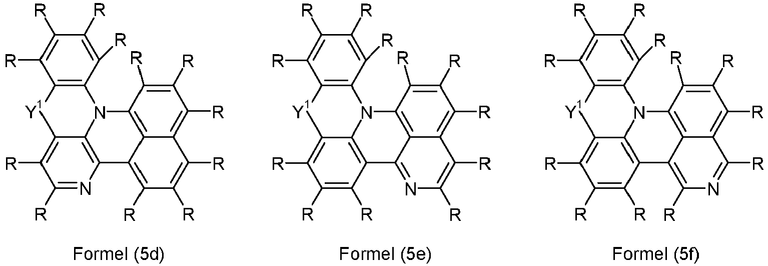 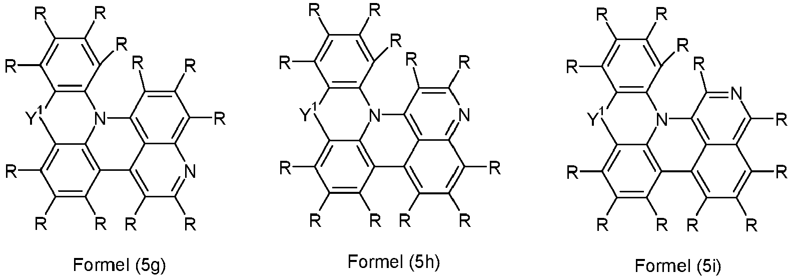 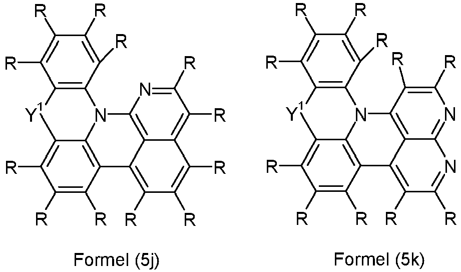 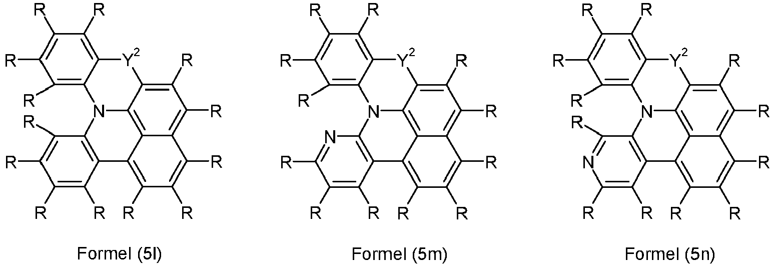 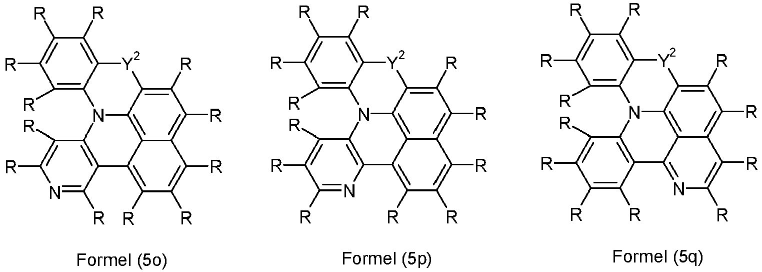 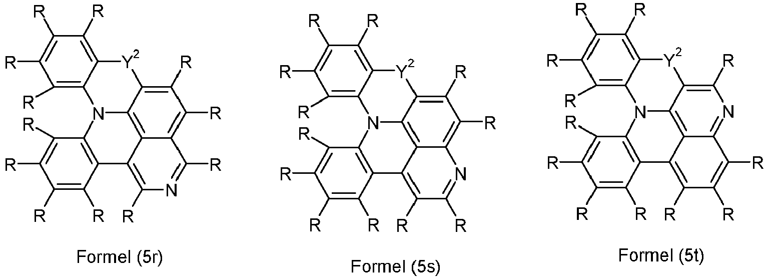 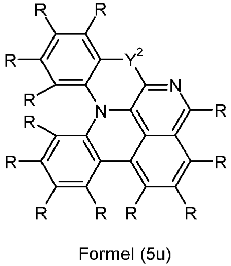 wobei die verwendeten Symbole die oben genannten Bedeutungen aufweisen. Dabei steht Y¹ bevorzugt für NR', O oder S, und Y² steht bevorzugt für eine Einfachbindung, NR', O, S oder C(=C(R")₂), wobei R' und R" die oben genannten Bedeutungen aufweisen

In einer besonders bevorzugten Ausführungsform der Erfindung sind die Verbindungen für m = 1 gewählt aus den Verbindungen der folgenden Formeln (6a) bis (6k) und für n = 1 aus den Verbindungen der folgenden Formeln (6l) bis (6u), 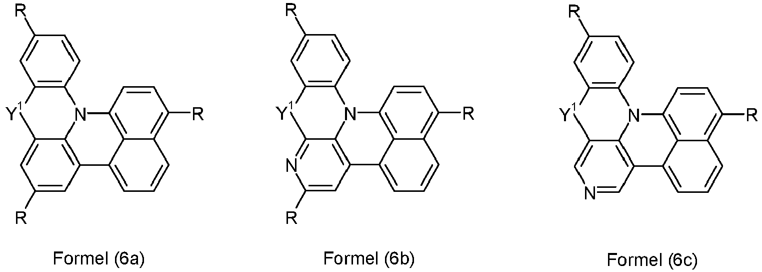 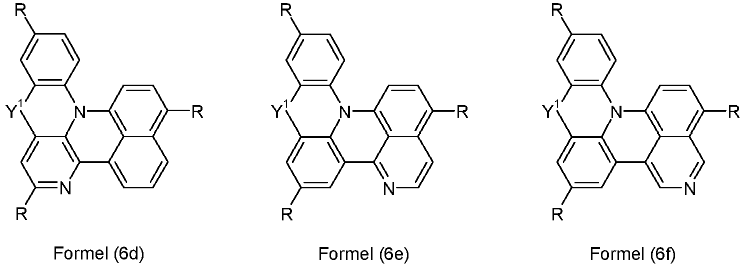 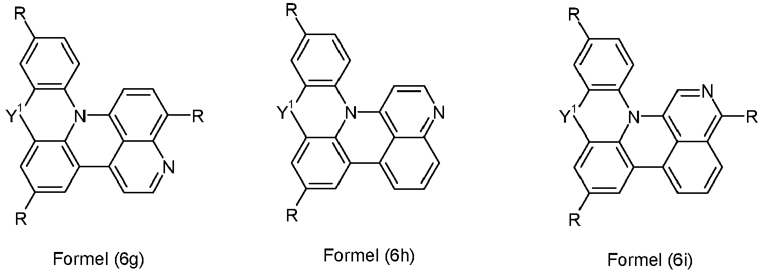 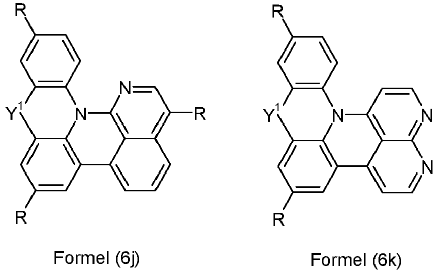 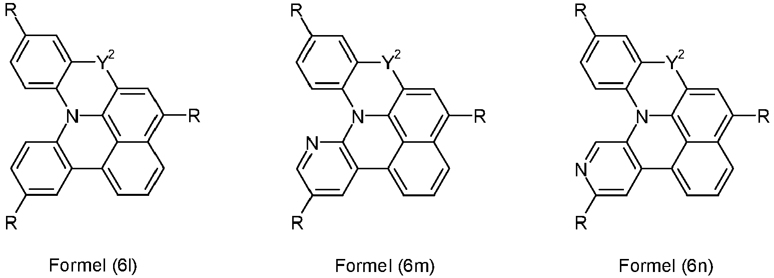 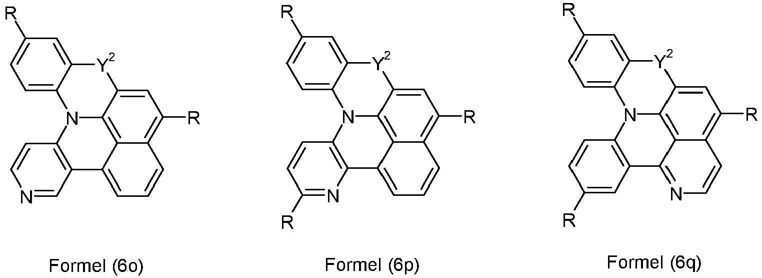 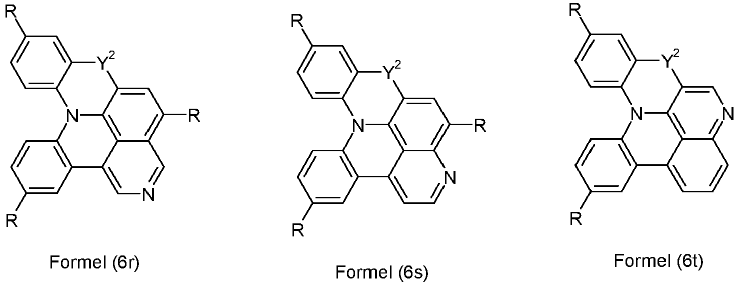 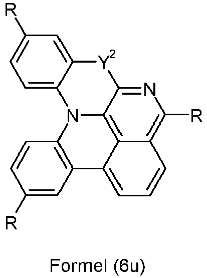 wobei die verwendeten Symbole die oben genannten Bedeutungen aufweisen. Dabei steht Y¹ bevorzugt für NR', O oder S, und Y² steht bevorzugt für eine Einfachbindung, NR', O, S oder C(=C(R")₂), wobei R' und R" die oben genannten Bedeutungen aufweisen
Besonders bevorzugt ist für m = 1 die Verbindung der Formel (5a) oder (5k) bzw. (6a) oder (6k) und für n = 1 die Verbindung der Formel (5l) bzw. (6l).

Ganz besonders bevorzugt sind die Verbindungen der folgenden Formeln (7a) bis (7h), 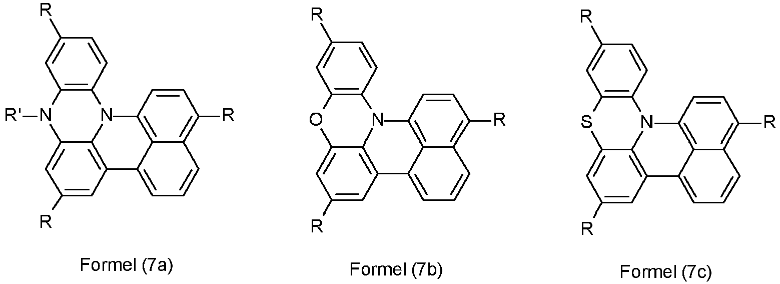 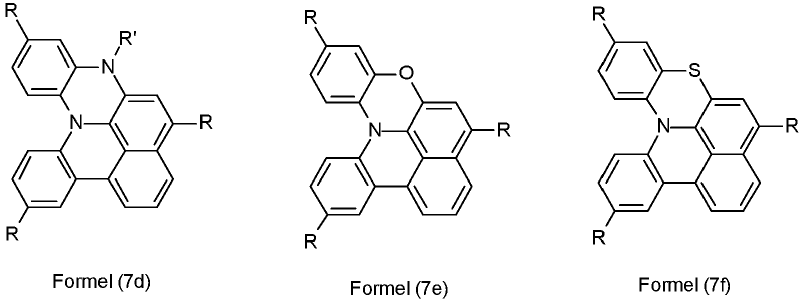 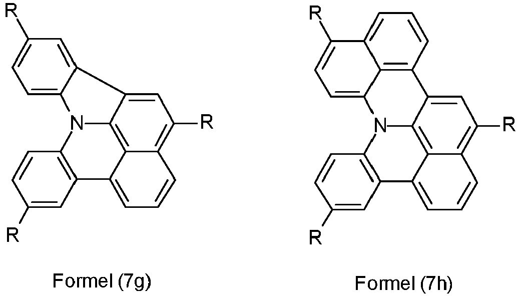 wobei die verwendeten Symbole die oben genannten Bedeutungen aufweisen.

Im Folgenden werden bevorzugte Substituenten R und R' an den erfindungsgemäßen Verbindungen beschrieben.

In einer bevorzugten Ausführungsform der Erfindung ist mindestens ein Substituent R, besonders bevorzugt genau ein Substituent R, verschieden von H, und die anderen Substituenten R stehen für H. In einer weiteren bevorzugten Ausführungsform der Erfindung sind zwei Substituenten R verschieden von H, und die anderen Substituenten R stehen für H. In einer weiteren bevorzugten Ausführungsform der Erfindung sind drei Substituenten R verschieden von H. Dabei ist bevorzugt mindestens einer der Substituenten R gewählt aus einem aromatischen oder heteroaromatischen Ringsystem. In einer weiteren bevorzugten Ausführungsform der Erfindung stehen alle Substituenten R für H, und die Gruppe Y¹ bzw. Y² steht für NR', wobei R' für ein aromatisches oder heteroaromatisches Ringsystem mit 6 bis 24 aromatischen Ringatomen steht, welches mit einem oder mehreren Resten R¹ substituiert sein kann.

In einer bevorzugten Ausführungsform der Erfindung ist R bei jedem Auftreten gleich oder verschieden ausgewählt aus der Gruppe bestehend aus H, N(Ar')₂, einer geradkettige Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe jeweils mit einem oder mehreren Resten R¹ substituiert sein kann, bevorzugt aber unsubstituiert ist, oder einem aromatischen oder heteroaromatischen Ringsystem mit 6 bis 24 aromatischen Ringatomen, das jeweils durch einen oder mehrere, bevorzugt nicht-aromatische, Reste R¹ substituiert sein kann. Ganz besonders bevorzugt ist R bei jedem Auftreten gleich oder verschieden ausgewählt aus der Gruppe bestehend aus H, N(Ar')₂ oder einem aromatischen oder heteroaromatischen Ringsystem mit 6 bis 24 aromatischen Ringatomen, das jeweils durch einen oder mehrere, bevorzugt nicht-aromatische, Reste R¹ substituiert sein kann.

Wenn Y¹ bzw. Y² für C(R')₂ steht, ist R' bevorzugt gleich oder verschieden bei jedem Auftreten gewählt aus der Gruppe bestehend aus einer geradkettige Alkylgruppe mit mit 1, 2, 3 oder 4 C-Atomen, oder einer verzweigten oder cyclischen Alkylgruppe mit 3 bis 6 C-Atomen, wobei die Alkylgruppe jeweils mit einem oder mehreren Resten R¹ substituiert sein kann, bevorzugt aber unsubstituiert ist, oder einem aromatischen oder heteroaromatischen Ringsystem mit 6 bis 12 aromatischen Ringatomen, das jeweils durch einen oder mehrere, bevorzugt nicht-aromatische, Reste R¹ substituiert sein kann. Dabei können zwei Reste R' auch miteinander ein aliphatisches oder aromatisches Ringsystem bilden. Ganz besonders bevorzugt ist R' gleich oder verschieden bei jedem Auftreten Methyl oder Phenyl, wobei zwei Phenylgruppen auch miteinander ein aromatisches Ringsystem bilden können.

In einer weiteren bevorzugten Ausführungsform der Erfindung steht für Y² = C(=C(R")₂) eine der beiden Gruppen R" für H und die andere der beiden Gruppen R" für CR¹=CR¹ und bildet mit Ar ein Ringsystem, wobei R¹ bevorzugt für H steht.

In einer weiteren bevorzugten Ausführungsform der Erfindung ist R¹ gleich oder verschieden bei jedem Auftreten ausgewählt aus der Gruppe bestehend aus H, einer geradkettigen Alkylgruppe mit 1 bis 6 C-Atomen, insbesondere mit 1, 2, 3 oder 4 C-Atomen, oder einer verzweigten oder cyclischen Alkylgruppe mit 3 bis 6 C-Atomen, wobei die Alkylgruppe mit einem oder mehreren Resten R² substituiert sein kann, bevorzugt aber unsubstituiert ist, oder einem aromatischen oder heteroaromatischen Ringsystem mit 6 bis 24 aromatischen Ringatomen, das jeweils durch einen oder mehrere Reste R² substituiert sein kann, bevorzugt aber unsubstituiert ist.

In einer weiteren bevorzugten Ausführungsform der Erfindung enthält die erfindungsgemäße Verbindung mindestens einen Substituenten R, der ausgewählt ist aus einem aromatischen oder heteroaromatischen Ringsystem oder N(Ar')₂, und/oder sie enthält mindestens eine Gruppe Y¹ bzw. Y², die für NR' steht, wobei R' ausgewählt ist aus einem aromatischen oder heteroaromatischen Ringsystem. Dabei kann dieser Substituent R für X = CR an das Grundgerüst der Verbindung oder an die Gruppe Ar gebunden sein.

Nachfolgend werden bevorzugte aromatische und heteroaromatische Ringsysteme beschrieben, welche als Substituent R oder R' oder als Gruppe Ar' innerhalb des Substituenten N(Ar')₂ in der erfindungsgemäßen Verbindung vorliegen können.

Geeignete aromatische bzw. heteroaromatische Ringsystem R, R' bzw. Ar" sind ausgewählt aus Phenyl, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, welches über die 1-, 2-, 3- oder 4-Position verknüpft sein kann, Spirobifluoren, welches über die 1-, 2-, 3- oder 4-Position verknüpft sein kann, Naphthalin, insbesondere 1- oder 2-verknüpftem Naphthalin, Indol, Benzofuran, Benzothiophen, Carbazol, welches über die 1-, 2-, 3- oder 4-Position verknüpft sein kann, Dibenzofuran, welches über die 1-, 2-, 3- oder 4-Position verknüpft sein kann, Dibenzothiophen, welches über die 1-, 2-, 3- oder 4-Position verknüpft sein kann, Indenocarbazol, Indolocarbazol, Pyridin, Pyrimidin, Pyrazin, Pyridazin, Triazin, Chinolin, Isochinolin, Chinazolin, Chinoxalin, Phenanthren, Triphenylen oder einer Kombination aus zwei oder drei dieser Gruppen, welche jeweils mit einem oder mehreren Resten R¹ substituiert sein können.

Dabei sind die Gruppen R, R' bzw. Ar' bevorzugt gewählt aus den Gruppen der folgenden Formeln Ar-1 bis Ar-75, 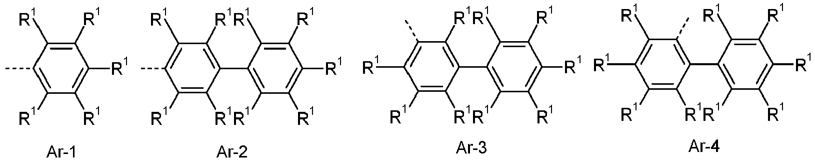 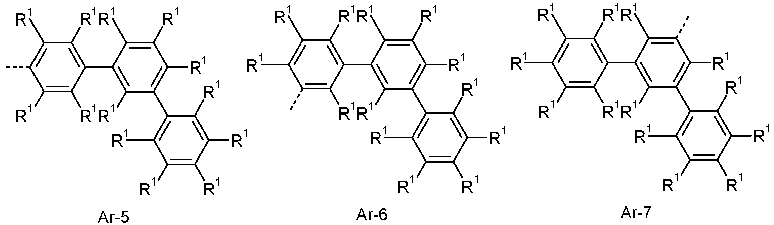 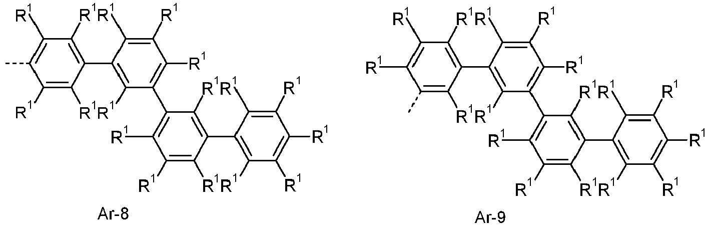 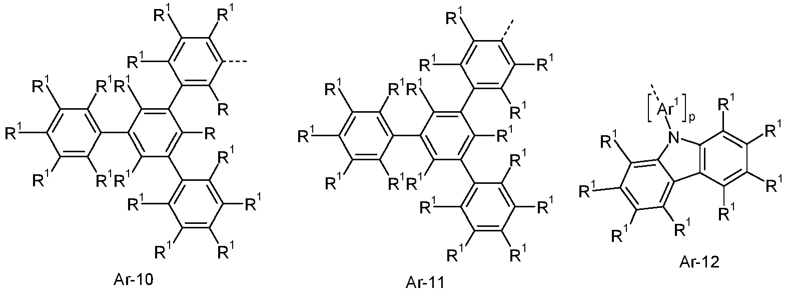 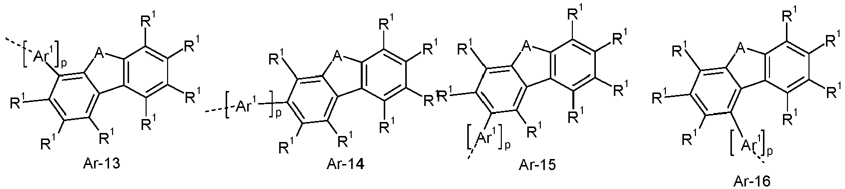 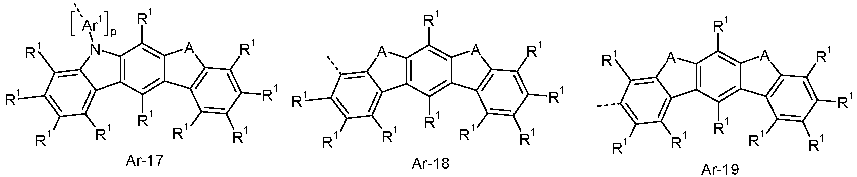 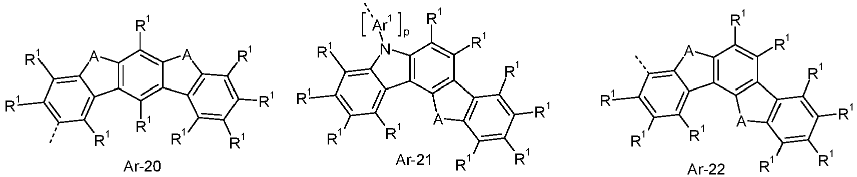 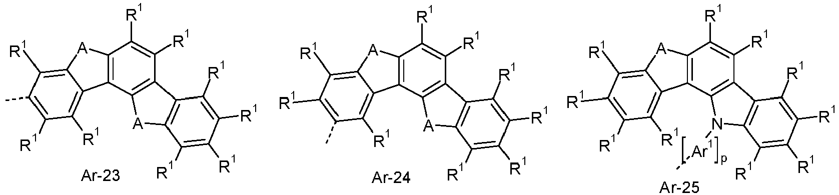 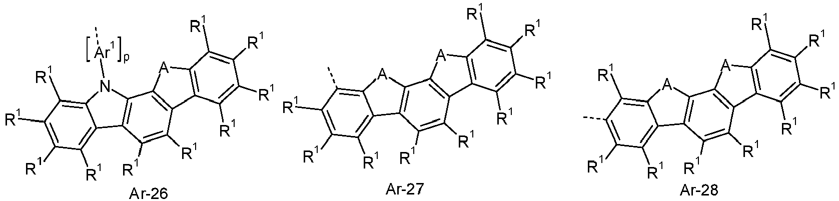 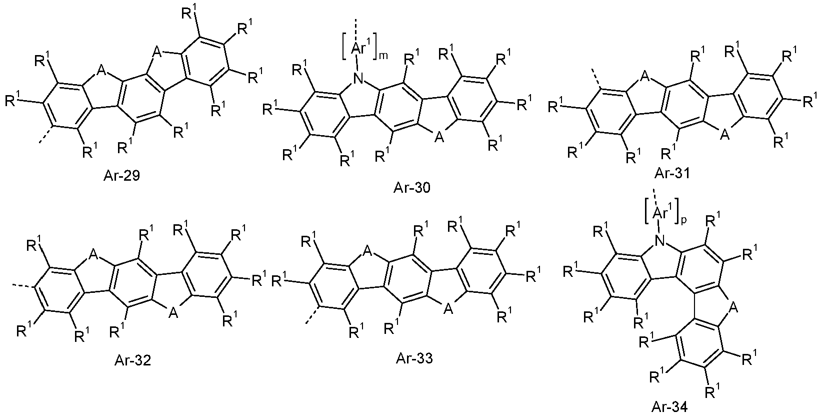 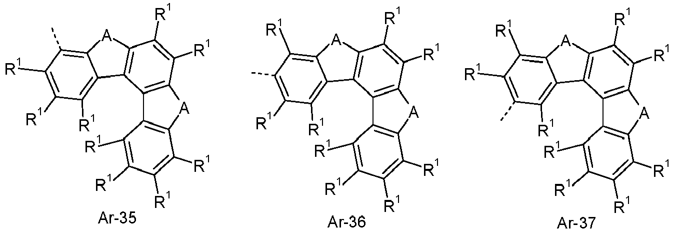 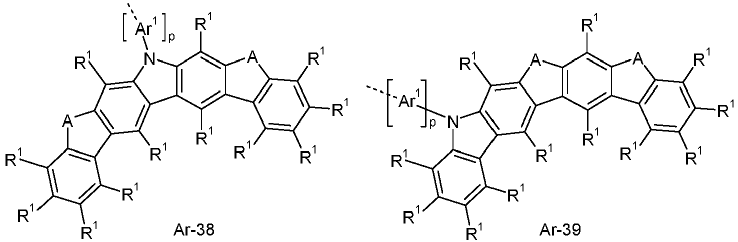 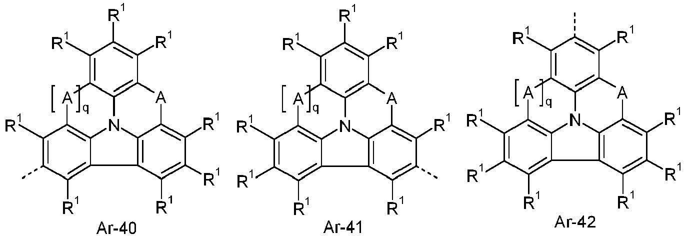 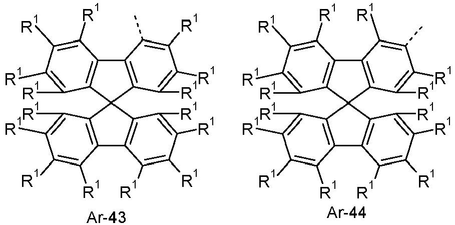 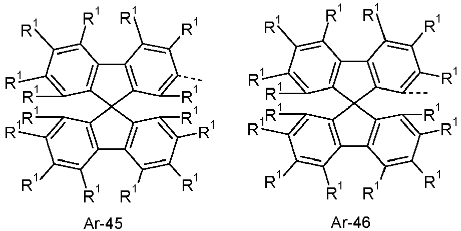 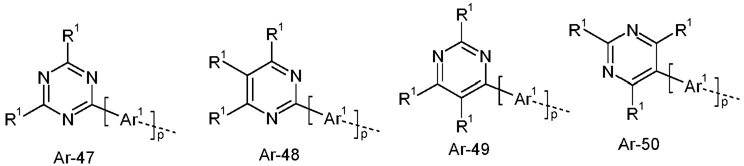 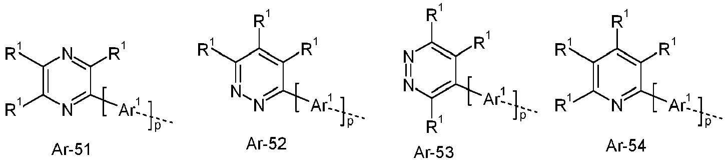 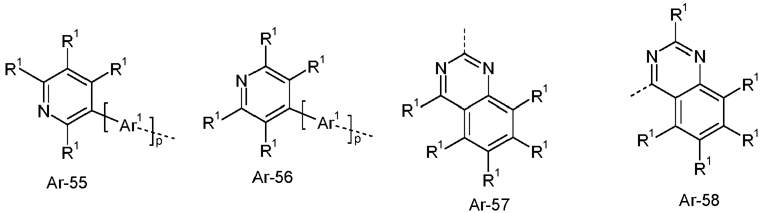 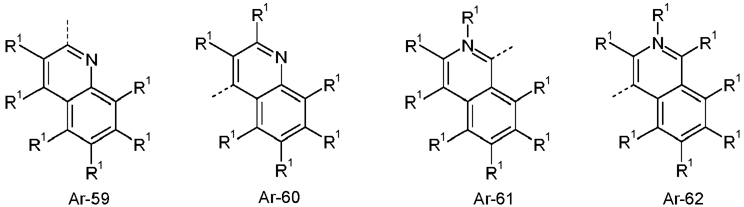 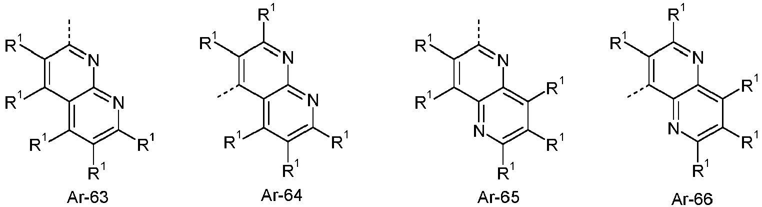 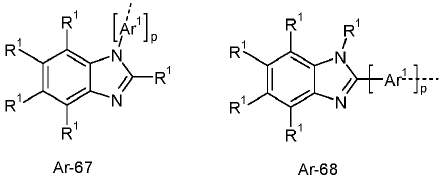 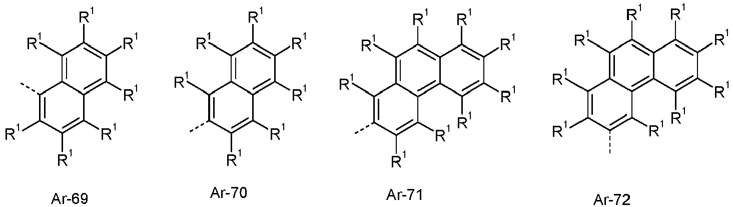 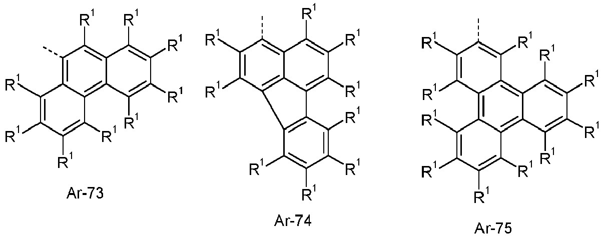 wobei R¹ die oben genannten Bedeutungen aufweist, die gestrichelte Bindung die Bindung an das Stickstoffatom in Y¹ bzw. Y² bzw. an ein Kohlenstoffatom des Grundgerüsts in Formel (1) bzw. an Ar bzw. an das Stickstoffatom in der Gruppe N(Ar')₂ darstellt und weiterhin gilt:
- Ar¹: ist bei jedem Auftreten gleich oder verschieden ein bivalentes aromatisches oder heteroaromatisches Ringsystem mit 6 bis 18 aromatischen Ringatomen, welches jeweils mit einem oder mehreren Resten R¹ substituiert sein kann;
- A: ist bei jedem Auftreten gleich oder verschieden C(R¹)₂, NR¹, O oder S;
- p: ist 0 oder 1, wobei p = 0 bedeutet, dass die Gruppe Ar¹ nicht vorhanden ist und dass die entsprechende aromatische bzw. heteroaromatische Gruppe direkt an ein Stickstoffatom in Y¹ bzw. Y² bzw. an ein Kohlenstoffatom des Grundgerüsts in Formel (1) bzw. in den bevorzugten Ausführungsformen bzw. an das Stickstoffatom in der Gruppe N(Ar')₂ gebunden ist; mit der Maßgabe, dass p = 1 ist für die Strukturen (Ar-12), (Ar-17), (Ar-21), (Ar-25), (Ar-26), (Ar-30), (Ar-34), (Ar-38) und (Ar-39), wenn es sich bei diesen Gruppen um Ausführungsformen von R' oder Ar' handelt;
- q: ist 0 oder 1, wobei q = 0 bedeutet, dass an dieser Position keine Gruppe A gebunden ist und an die entsprechenden Kohlenstoffatome statt dessen Reste R¹ gebunden sind.

Wenn die oben genannten Gruppen für R, R' bzw. Ar' mehrere Gruppen A aufweisen, so kommen hierfür alle Kombinationen aus der Definition von A in Frage. Bevorzugte Ausführungsformen sind dann solche, in denen eine Gruppe A für NR¹ und die andere Gruppe A für C(R¹)₂ steht oder in denen beide Gruppen A für NR¹ stehen oder in denen beide Gruppen A für O stehen. In einer besonders bevorzugten Ausführungsform der Erfindung steht in Gruppen R bzw. Ar, die mehrere Gruppen A aufweisen, mindestens eine Gruppe A für C(R¹)₂ oder für NR¹.

Wenn A für NR¹ steht, steht der Substituent R¹, der an das Stickstoffatom gebunden ist, bevorzugt für ein aromatisches oder heteroaromatisches Ringsystem mit 6 bis 24 aromatischen Ringatomen, welches auch durch einen oder mehrere Reste R² substituiert sein kann. In einer besonders bevorzugten Ausführungsform steht dieser Substituent R¹ gleich oder verschieden bei jedem Auftreten für ein aromatisches oder heteroaromatisches Ringsystem mit 6 bis 24 aromatischen Ringatomen, insbesondere mit 6 bis 18 aromatischen Ringatomen, welches keine kondensierten Arylgruppen aufweist und welches keine kondensierten Heteroarylgruppen, in denen zwei oder mehr aromatische bzw. heteroaromatische 6-RingGruppen direkt aneinander ankondensiert sind, aufweist, und welches jeweils auch durch einen oder mehrere Reste R² substituiert sein kann. Bevorzugt sind Phenyl, Biphenyl, Terphenyl und Quaterphenyl mit Verknüpfungsmustern, wie vorne für Ar-1 bis Ar-11 aufgeführt, wobei diese Strukturen statt durch R¹ durch einen oder mehrere Reste R² substituiert sein können, bevorzugt aber unsubstituiert sind. Bevorzugt sind weiterhin Triazin, Pyrimidin und Chinazolin, wie vorne für Ar-47 bis Ar-50, Ar-57 und Ar-58 aufgeführt, wobei diese Strukturen statt durch R¹ durch einen oder mehrere Reste R² substituiert sein können.

Wenn A für C(R¹)₂ steht, stehen die Substituenten R¹, die an dieses Kohlenstoffatom gebunden sind, bevorzugt gleich oder verschieden bei jedem Auftreten für eine lineare Alkylgruppe mit 1 bis 10 C-Atomen oder für eine verzweigte oder cyclische Alkylgruppe mit 3 bis 10 C-Atomen oder für ein aromatisches oder heteroaromatisches Ringsystem mit 6 bis 24 aromatischen Ringatomen, welches auch durch einen oder mehrere Reste R² substituiert sein kann. Ganz besonders bevorzugt steht R¹ für eine Methylgruppe oder für eine Phenylgruppe. Dabei können die Reste R¹ auch miteinander ein Ringsystem bilden, was zu einem Spirosystem führt.

Weitere geeignete Gruppen R, R' bzw. Ar sind Gruppen der Formel -Ar⁴-N(Ar²)(Ar³), wobei Ar², Ar³ und Ar⁴ gleich oder verschieden bei jedem Auftreten für ein aromatisches oder heteroaromatisches Ringsystem mit 5 bis 24 aromatischen Ringatomen stehen, welches jeweils mit einem oder mehreren Resten R¹ substituiert sein kann. Dabei beträgt die Gesamtzahl der aromatischen Ringatome von Ar², Ar³ und Ar⁴ maximal 30.

Dabei können Ar⁴ und Ar² miteinander und/oder Ar² und Ar³ miteinander auch durch eine Gruppe ausgewählt aus C(R¹)₂, NR¹, O oder S verbunden sein. Bevorzugt erfolgt die Verknüpfung von Ar⁴ und Ar² miteinander bzw. von Ar² und Ar³ miteinander jeweils ortho zur Position der Verknüpfung mit dem Stickstoffatom. In einer weiteren Ausführungsform der Erfindung sind keine der Gruppen Ar², Ar³ bzw. Ar⁴ miteinander verbunden.

Bevorzugt ist Ar⁴ ein aromatisches oder heteroaromatisches Ringsystem mit 6 bis 24 aromatischen Ringatomen, bevorzugt mit 6 bis 12 aromatischen Ringatomen, das jeweils mit einem oder mehreren Resten R¹ substituiert sein kann. Besonders bevorzugt ist Ar⁴ ausgewählt aus der Gruppe bestehend aus ortho-, meta- oder para-Phenylen oder ortho-, meta- oder para-Biphenyl, welche jeweils durch einen oder mehrere Reste R¹ substituiert sein können, bevorzugt aber unsubstituiert sind. Ganz besonders bevorzugt ist Ar⁴ eine unsubstituierte Phenylengruppe.

Bevorzugt sind Ar² und Ar³ gleich oder verschieden bei jedem Auftreten ein aromatisches oder heteroaromatisches Ringsystem mit 6 bis 24 aromatischen Ringatomen, das jeweils mit einem oder mehreren Resten R¹ substituiert sein kann. Besonders bevorzugte Gruppen Ar² bzw. Ar³ sind gleich oder verschieden bei jedem Auftreten ausgewählt aus der Gruppe bestehend aus Benzol, ortho-, meta- oder para-Biphenyl, ortho-, meta-, para- oder verzweigtem Terphenyl, ortho-, meta-, para- oder verzweigtem Quaterphenyl, 1-, 2-, 3- oder 4-Fluorenyl, 1-, 2-, 3- oder 4-Spirobifluorenyl, 1- oder 2-Naphthyl, Indol, Benzofuran, Benzothiophen, 1-, 2-, 3- oder 4-Carbazol, 1-, 2-, 3- oder 4-Dibenzofuran, 1-, 2-, 3- oder 4-Dibenzothiophen, Indenocarbazol, Indolocarbazol, 2-, 3- oder 4-Pyridin, 2-, 4- oder 5-Pyrimidin, Pyrazin, Pyridazin, Triazin, Phenanthren, Triphenylen oder Kombinationen aus zwei, drei oder vier dieser Gruppen, welche jeweils mit einem oder mehreren Resten R¹ substituiert sein können. Besonders bevorzugt stehen Ar² und Ar³ gleich oder verschieden bei jedem Auftreten für ein aromatisches Ringsystem mit 6 bis 24 aromatischen Ringatomen, welches durch einen oder mehrere Reste R¹ substituiert sein kann, insbesondere ausgewählt aus den Gruppen bestehend aus Benzol, Biphenyl, insbesondere ortho-, meta- oder para-Biphenyl, Terphenyl, insbesondere ortho-, meta-, para- oder verzweigtem Terphenyl, Quaterphenyl, insbesondere ortho-, meta-, para- oder verzweigtem Quaterphenyl, Fluoren, insbesondere 1-, 2-, 3- oder 4-Fluoren, oder Spirobifluoren, insbesondere 1-, 2-, 3- oder 4-Spirobifluoren.

Dabei haben in erfindungsgemäßen Verbindungen, die durch Vakuumverdampfung verarbeitet werden, die Alkylgruppen bevorzugt nicht mehr als fünf C-Atome, besonders bevorzugt nicht mehr als 4 C-Atome, ganz besonders bevorzugt nicht mehr als 1 C-Atom. Für Verbindungen, die aus Lösung verarbeitet werden, eignen sich auch Verbindungen, die mit Alkylgruppen, insbesondere verzweigten Alkylgruppen, mit bis zu 10 C-Atomen substituiert sind oder die mit Oligoarylengruppen, beispielsweise ortho-, meta-, para- oder verzweigten Terphenyl- oder Quaterphenylgruppen, substituiert sind.

Wenn die Verbindungen der Formel (1) bzw. die bevorzugten Ausführungsformen als Matrixmaterial für einen phosphoreszierenden Emitter oder in einer Schicht, die direkt an eine phosphoreszierende Schicht angrenzt, verwendet werden, ist es weiterhin bevorzugt, wenn die Verbindung keine kondensierten Aryl- bzw. Heteroarylgruppen enthält, in denen mehr als zwei Sechsringe direkt aneinander ankondensiert sind. Insbesondere ist es bevorzugt, dass die Reste R, R', Ar, R¹ und R² keine kondensierten Aryl- bzw. Heteroarylgruppen enthalten, in denen zwei oder mehr Sechsringe direkt aneinander ankondensiert sind. Eine Ausnahme hiervon bilden Phenanthren und Triphenylen, die aufgrund ihrer hohen Triplettenergie trotz der Anwesenheit kondensierter aromatischer Sechsringe bevorzugt sein können.

Die oben genannten bevorzugten Ausführungsformen können beliebig innerhalb der in Anspruch 1 definierten Einschränkungen miteinander kombiniert werden. In einer besonders bevorzugten Ausführungsform der Erfindung treten die oben genannten Bevorzugungen gleichzeitig auf.

Beispiele für bevorzugte Verbindungen gemäß den oben aufgeführten Ausführungsformen sind die in der folgenden Tabelle aufgeführten Verbindungen.

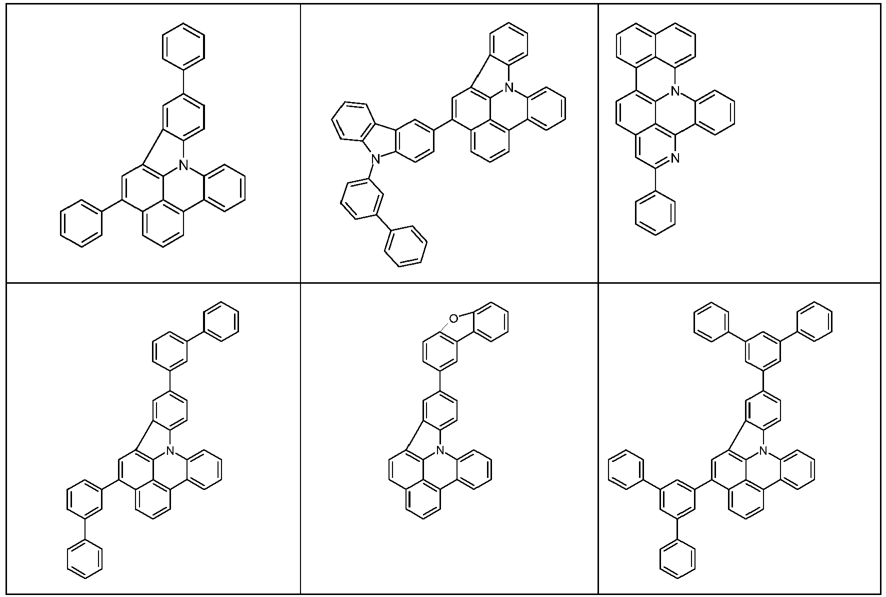

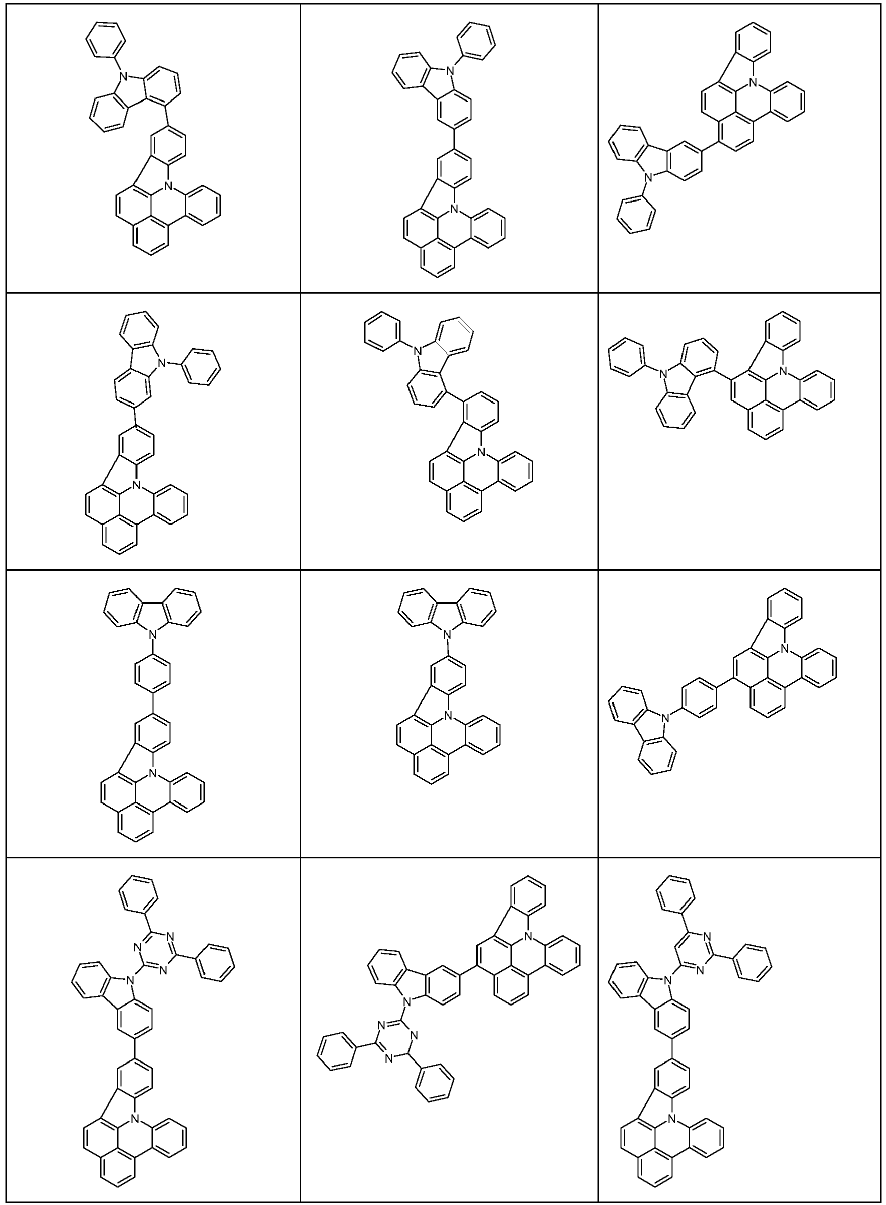

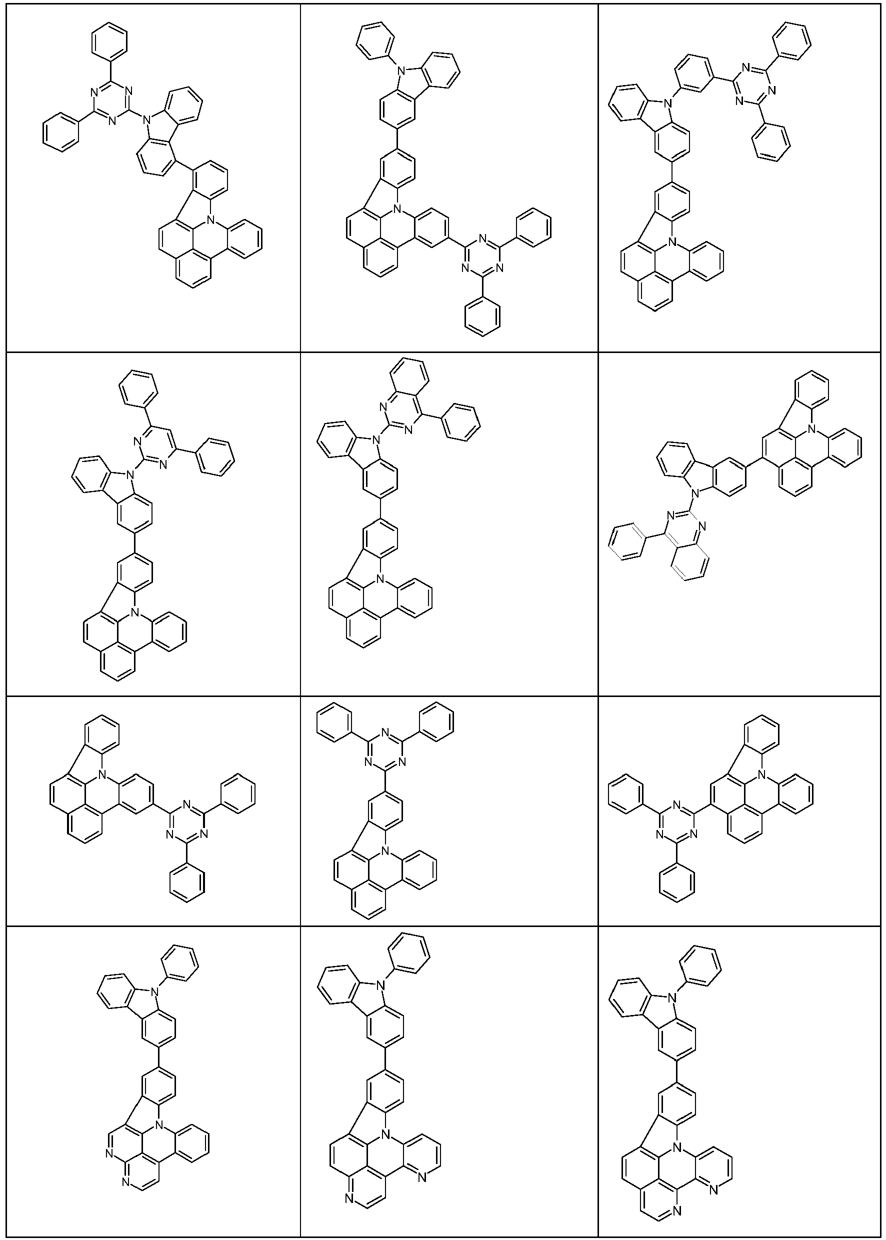

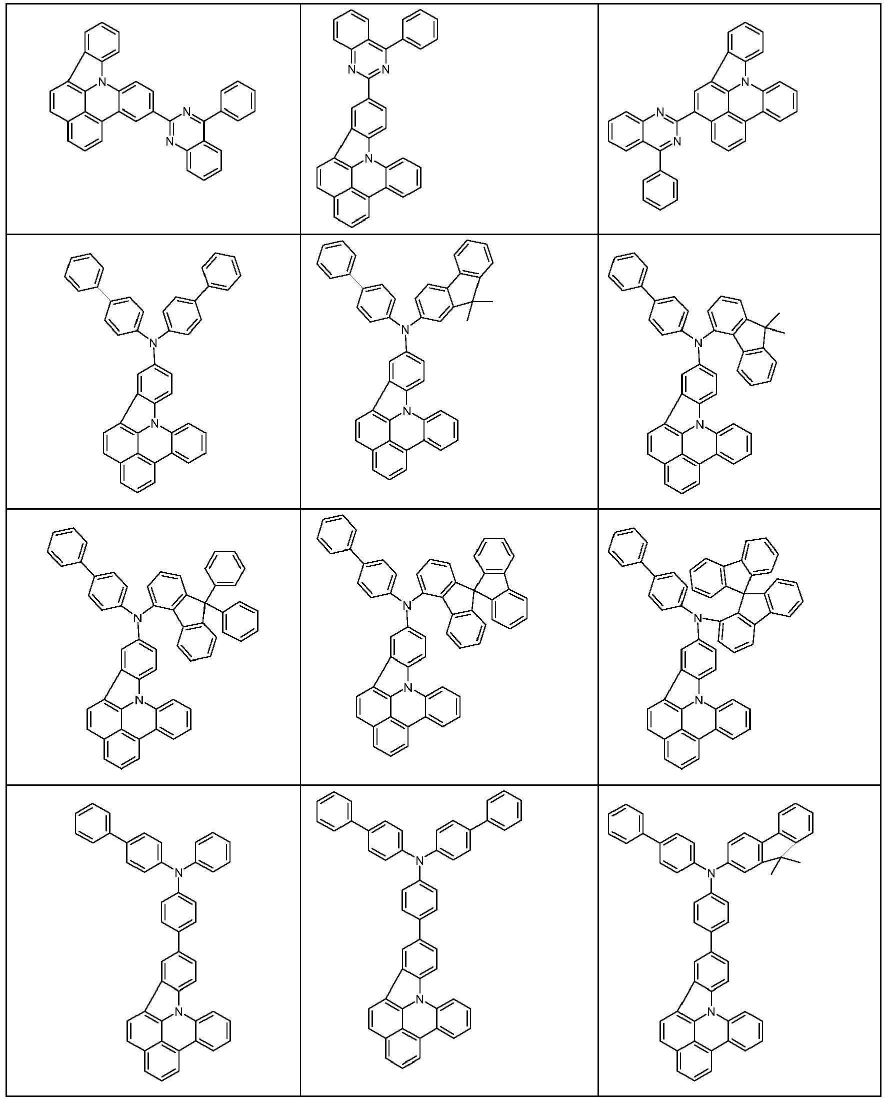

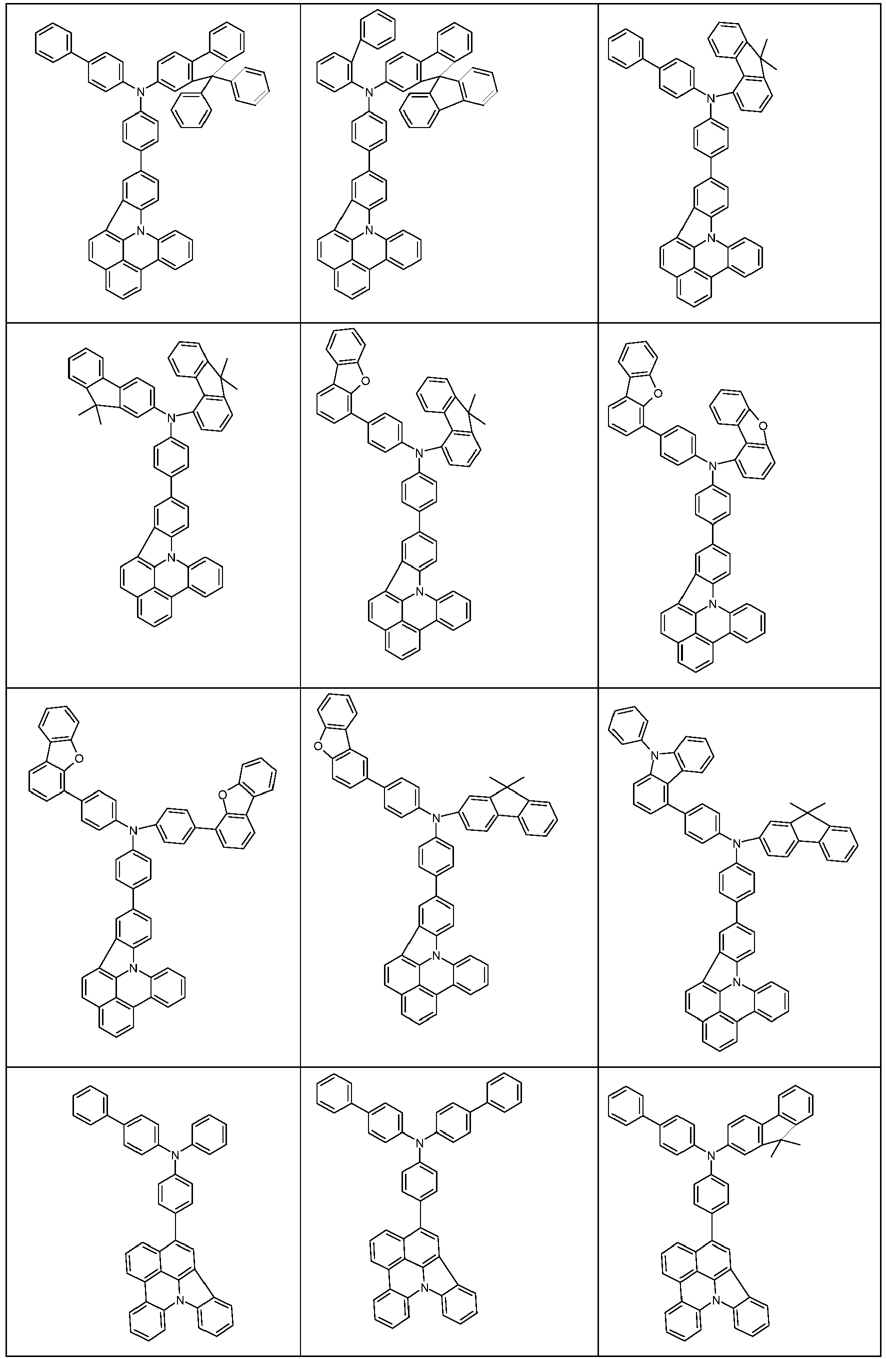

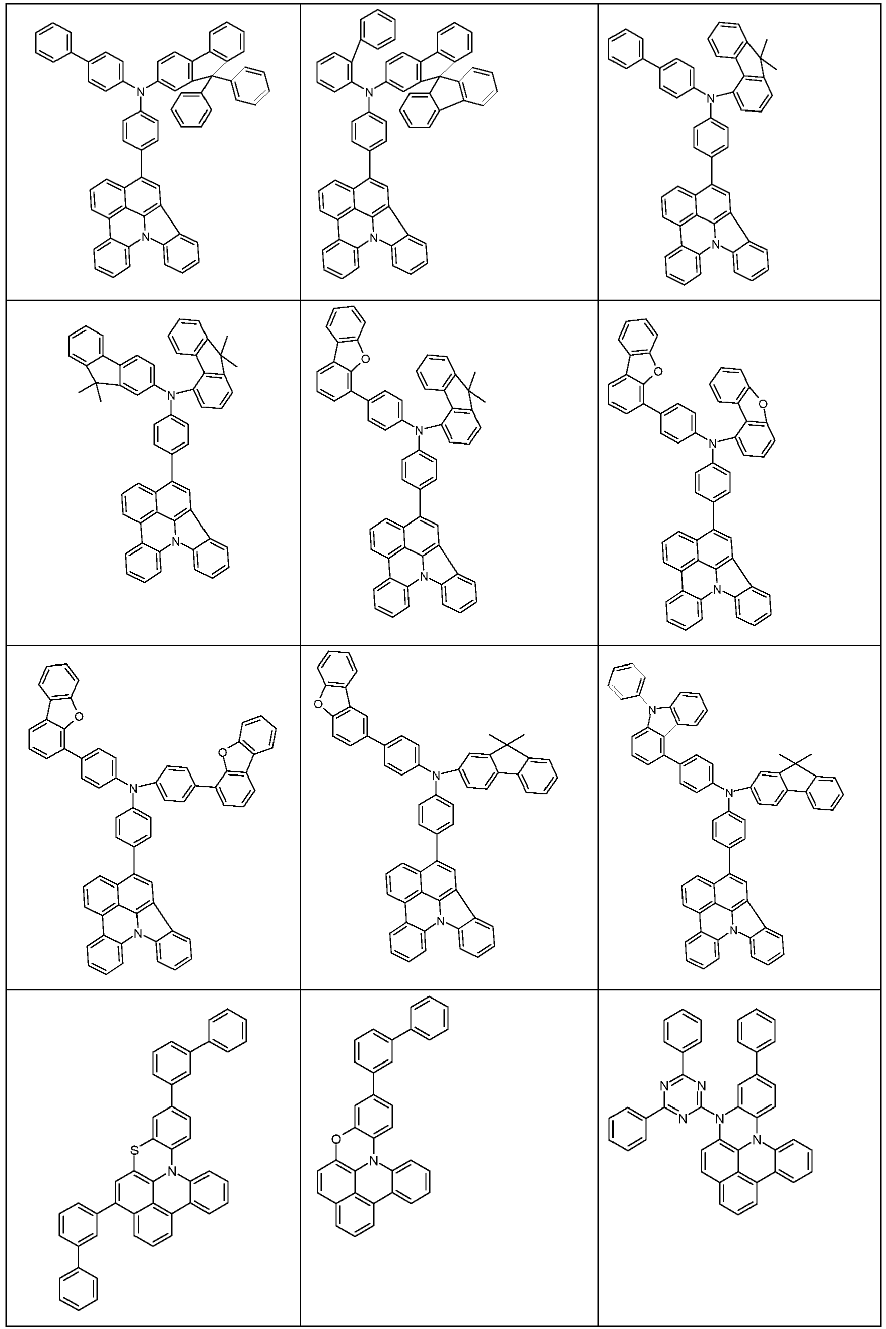

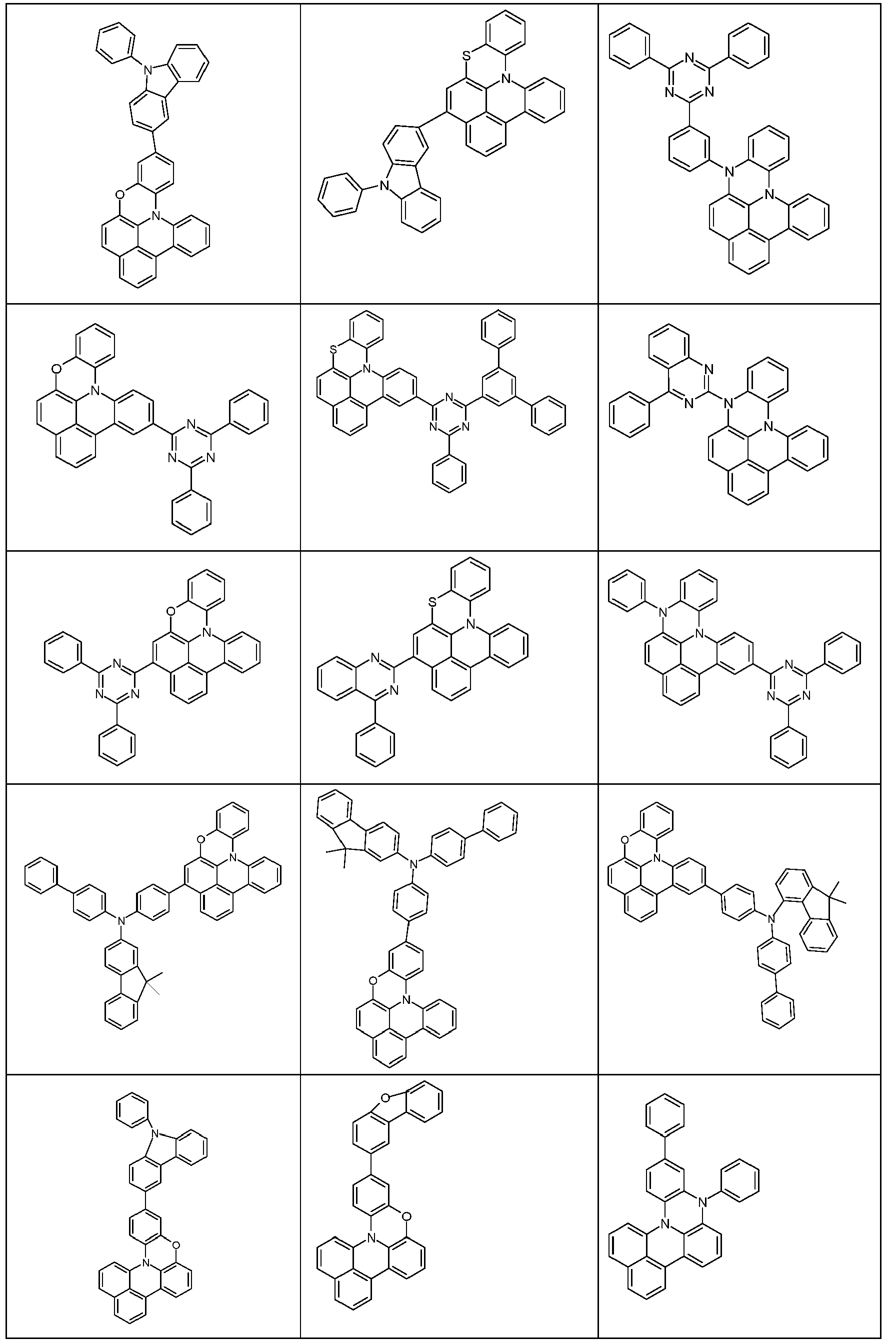

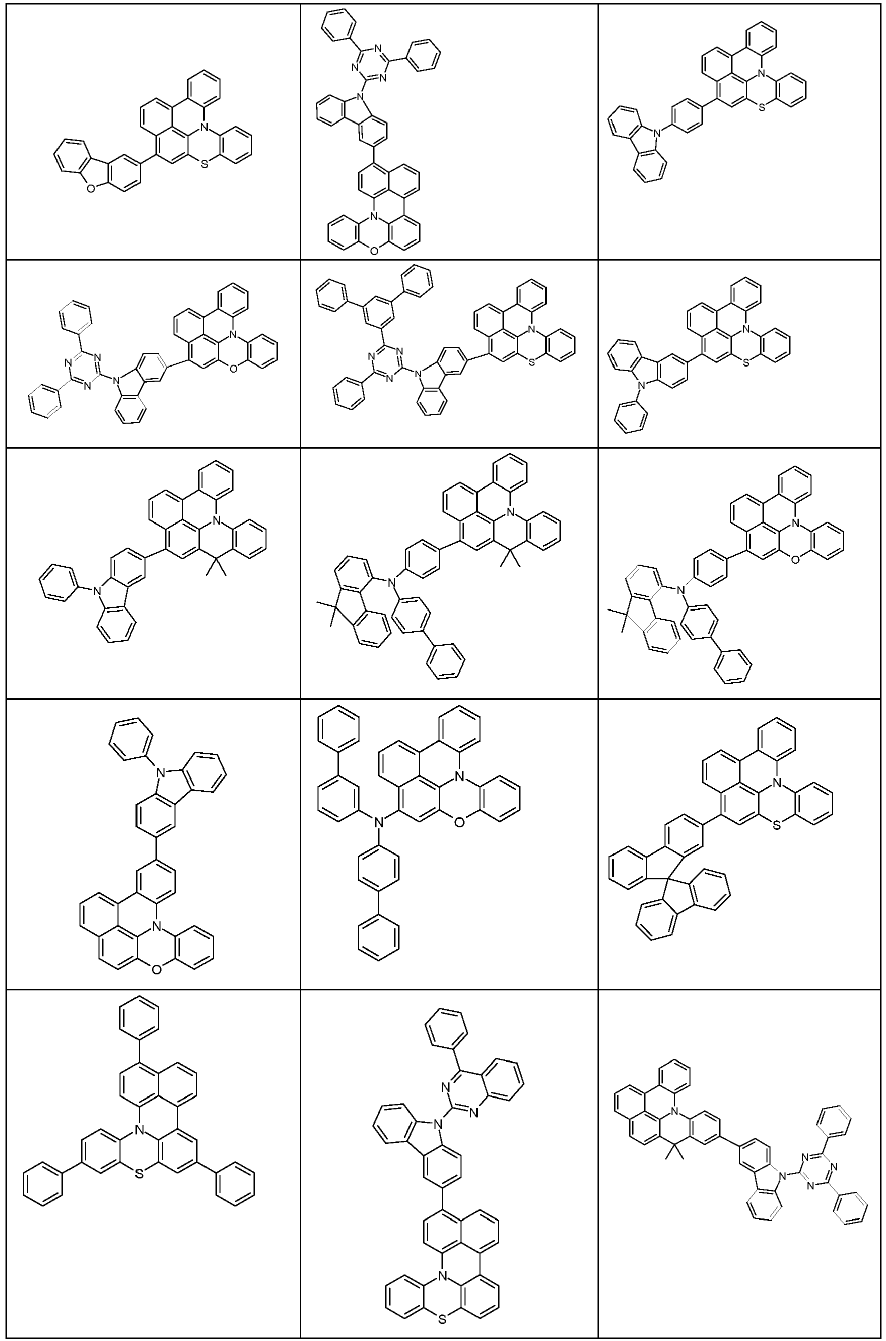

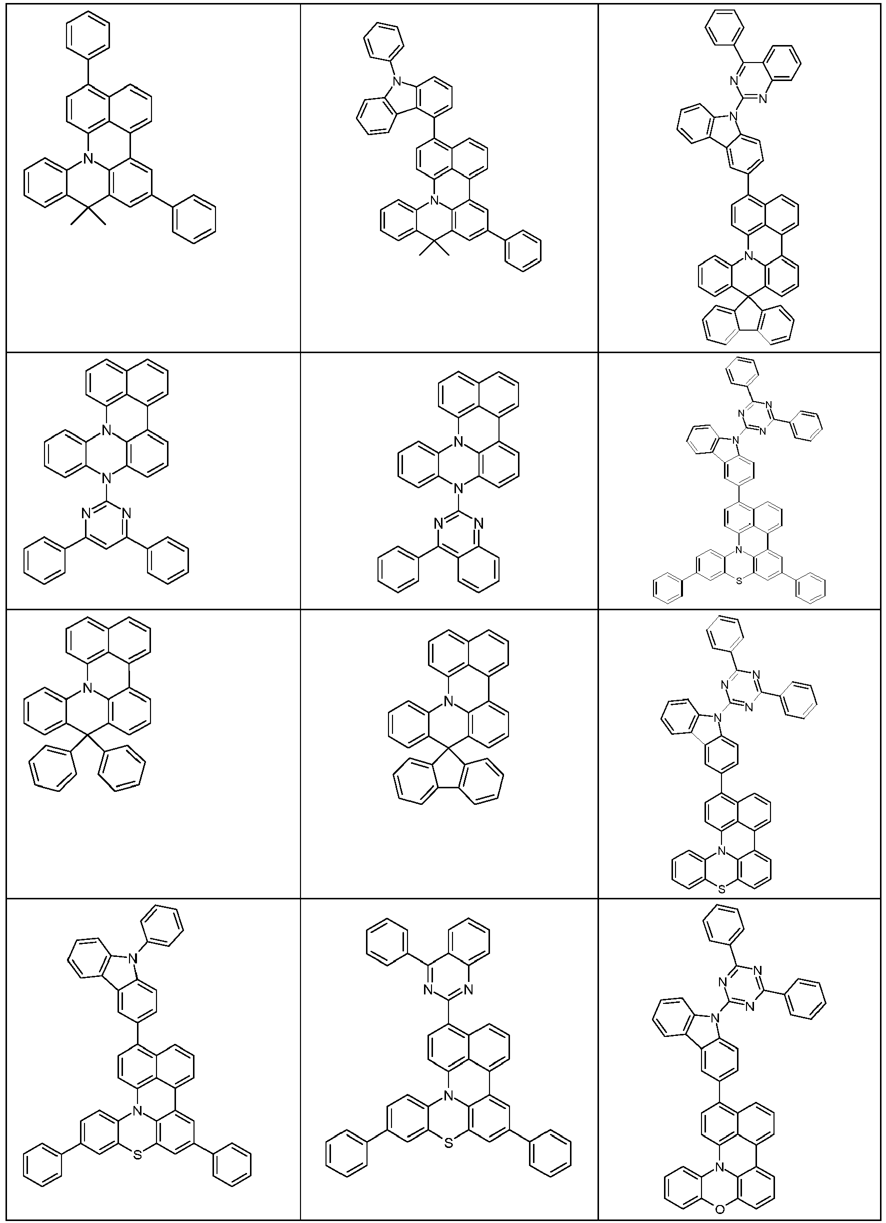

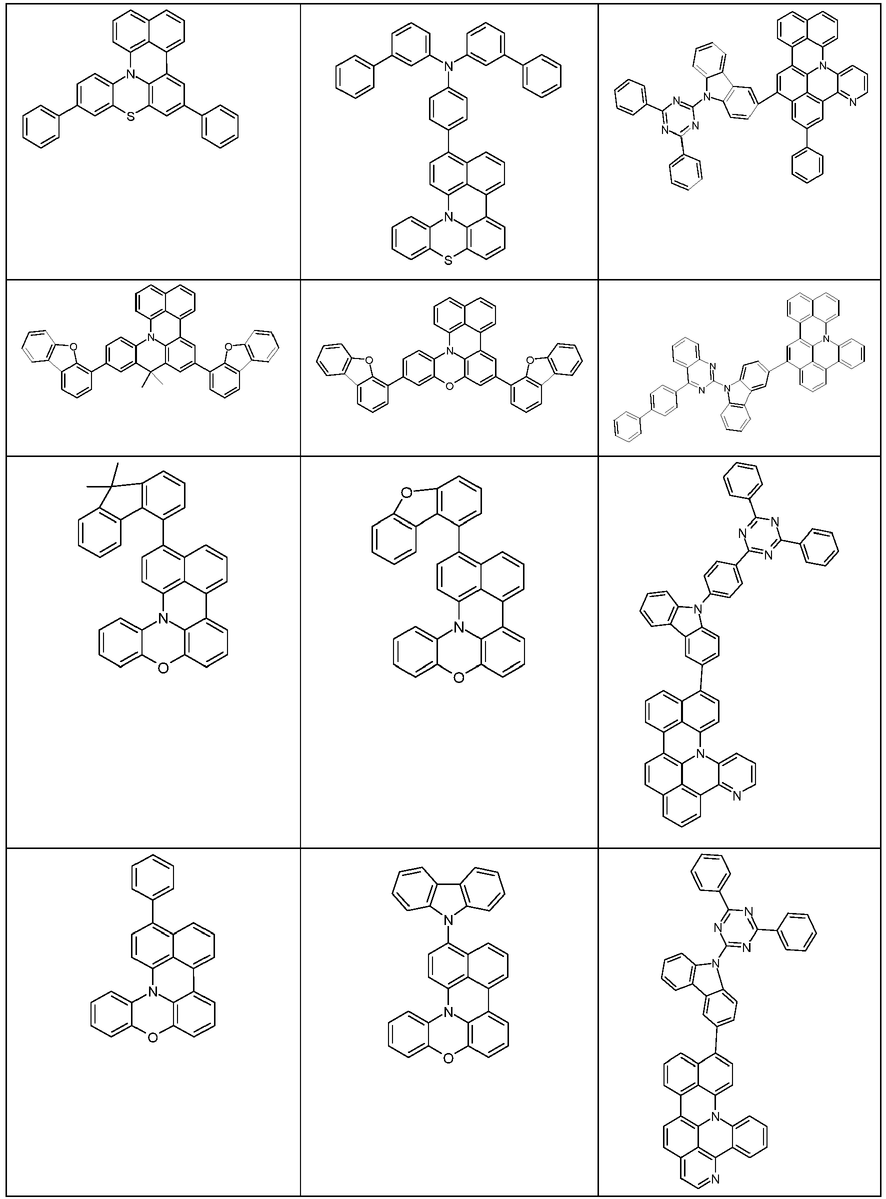

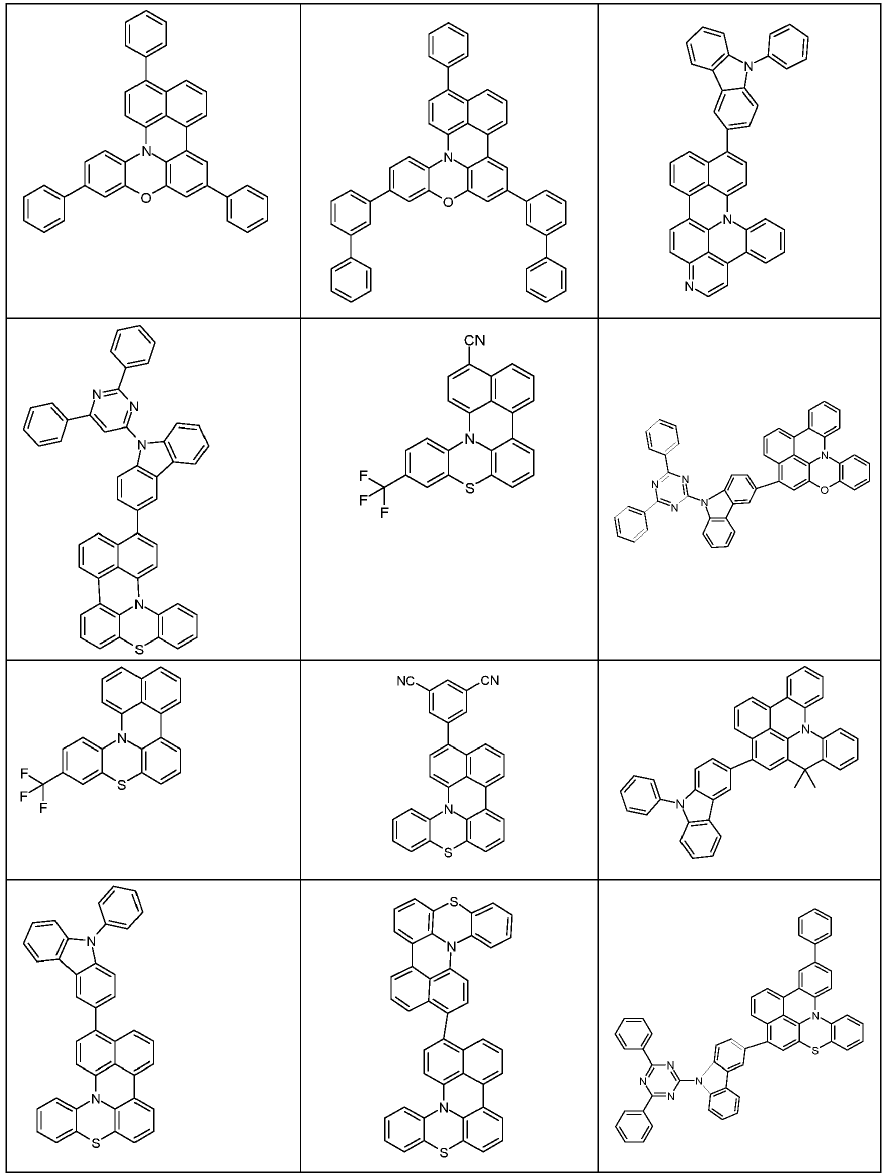

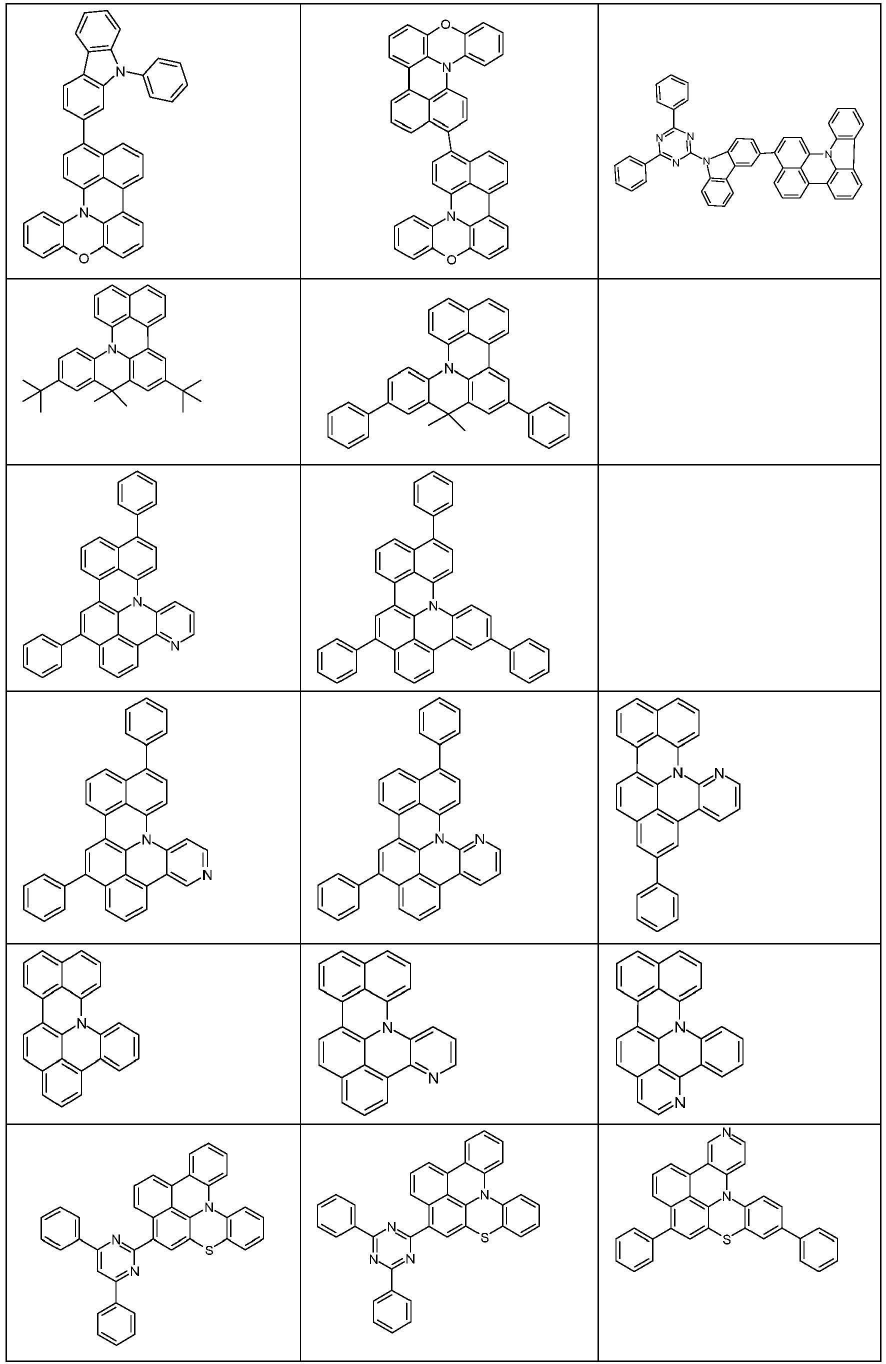

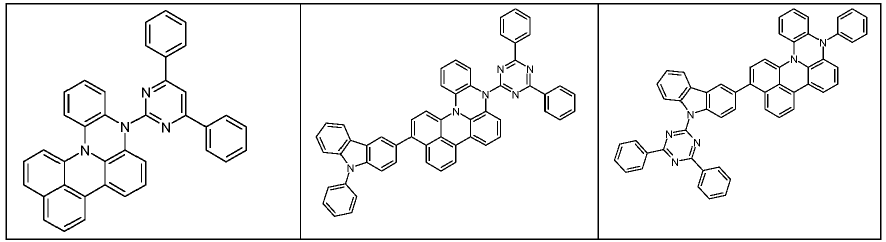

Die Grundstruktur der erfindungsgemäßen Verbindungen kann nach den in den nachfolgenden Schemata skizzierten Wegen dargestellt werden. Dabei sind die einzelnen Syntheseschritte, wie beispielsweise C-C-Kupplungsreaktionen gemäß Suzuki, C-N-Kupplungsreaktionen gemäß Hartwig-Buchwald oder Cyclisierungsreaktionen, dem Fachmann prinzipiell bekannt. Weitere Informationen zur Synthese der erfindungsgemäßen Verbindungen können den Synthesebeispielen entnommen werden. Dabei ist in Schema 1 die Synthese von Verbindungen mit m = 1 dargestellt. In Schema 2 ist die Synthese von Verbindungen mit n = 1 und Y² = Einfachbindung dargestellt. In Schema 3 ist die Synthese von Verbindungen mit n = 1 und Y² = NR', C(R')₂, O oder S dargestellt.

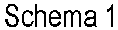

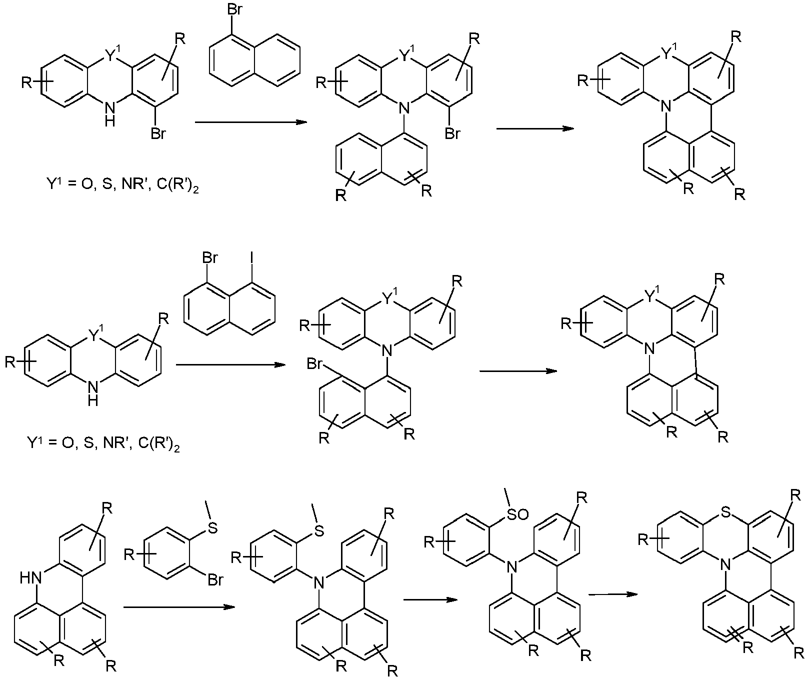

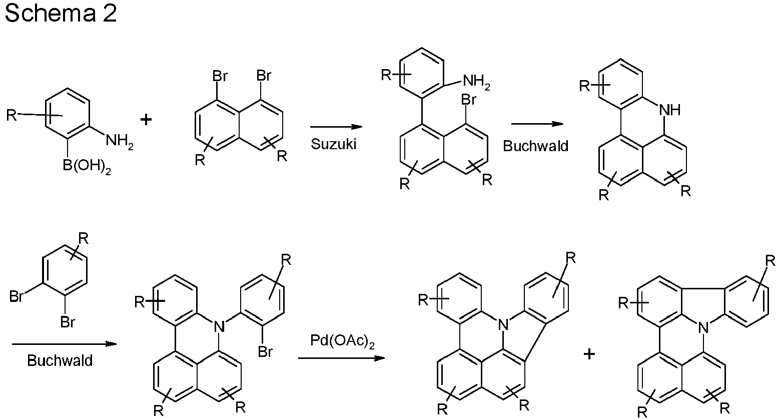

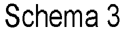

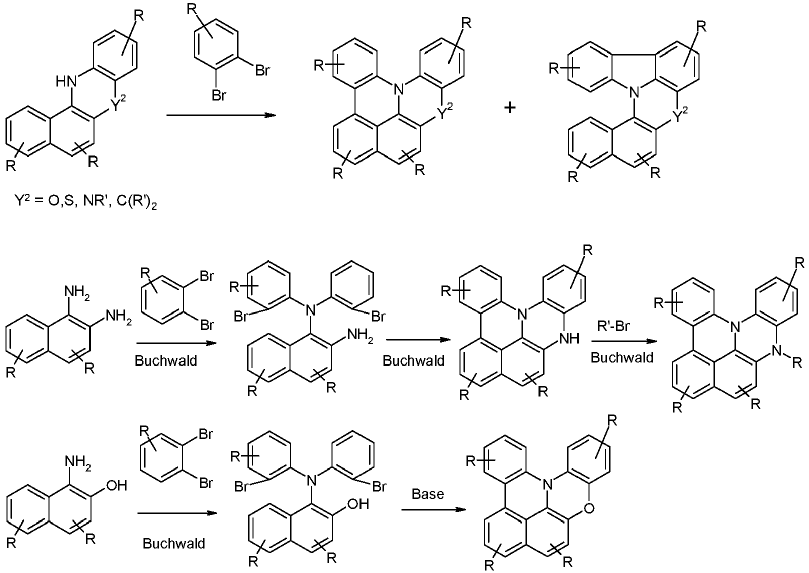

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, 2-Methylbiphenyl, 3-Methylbiphenyl, 1-Methylnaphthalin, 1-Ethylnaphthalin, Ethyloctanoat, Sebacinsäure-diethylester, Octyloctanoat, Heptylbenzol, Menthylisovalerat, Cyclohexylhexanoat oder Mischungen dieser Lösemittel.

Ein weiterer Gegenstand der vorliegenden Erfindung ist daher eine Formulierung bzw. eine Zusammensetzung, enthaltend mindestens eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Ein weiterer Gegenstand der vorliegenden Erfindung ist die Verwendung einer erfindungsgemäßen Verbindung in einer elektronischen Vorrichtung, insbesondere in einer organischen Elektrolumineszenzvorrichtung.

Ein nochmals weiterer Gegenstand der vorliegenden Erfindung ist eine elektronische Vorrichtung enthaltend mindestens eine erfindungsgemäße Verbindung.

Eine elektronische Vorrichtung im Sinne der vorliegenden Erfindung ist eine Vorrichtung, welche mindestens eine Schicht enthält, die mindestens eine organische Verbindung enthält. Das Bauteil kann dabei auch anorganische Materialien enthalten oder auch Schichten, welche vollständig aus anorganischen Materialien aufgebaut sind.

Die elektronische Vorrichtung ist bevorzugt ausgewählt aus der Gruppe bestehend aus organischen Elektrolumineszenzvorrichtungen (OLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices", bevorzugt aber organischen Elektrolumineszenzvorrichtungen (OLEDs), besonders bevorzugt phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Interlayer eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen. Es kann sich bei der erfindungsgemäßen organischen Elektrolumineszenzvorrichtung auch um eine Tandem-OLED handeln, insbesondere für weiß emittierende OLEDs.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) bzw. die oben ausgeführten bevorzugten Ausführungsformen in einer emittierenden Schicht als Matrixmaterial für phosphoreszierende Emitter oder für Emitter, die TADF (thermally activated delayed fluorescence) zeigen, insbesondere für phosphoreszierende Emitter. Weiterhin kann die erfindungsgemäße Verbindung auch in einer Elektronentransportschicht und/oder in einer Lochtransportschicht und/oder in einer Exzitonenblockierschicht und/oder in einer Lochblockierschicht eingesetzt werden. Besonders bevorzugt wird die erfindungsgemäße Verbindung als Matrixmaterial für rot, orange oder gelb phosphoreszierende Emitter, insbesondere für rot phosphoreszierende Emitter, in einer emittierenden Schicht oder als Elektronentransport- bzw. Lochblockiermaterial in einer Elektronentransport- bzw. Lochblockierschicht eingesetzt.

Wenn die erfindungsgemäße Verbindung als Matrixmaterial für eine phosphoreszierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit höherer Spinmultiplizität verstanden, also einem Spinzustand > 1, insbesondere aus einem angeregten Triplettzustand. Im Sinne dieser Anmeldung sollen alle lumineszierenden Komplexe mit Übergangsmetallen oder Lanthaniden, insbesondere alle Iridium-, Platin- und Kupferkomplexe als phosphoreszierende Verbindungen angesehen werden.

Die Mischung aus der erfindungsgemäßen Verbindung und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der erfindungsgemäßen Verbindung bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial.

Eine weitere bevorzugte Ausführungsform der vorliegenden Erfindung ist der Einsatz der erfindungsgemäßen Verbindung als Matrixmaterial für einen phosphoreszierenden Emitter in Kombination mit einem weiteren Matrixmaterial. Geeignete Matrixmaterialien, welche in Kombination mit den erfindungsgemäßen Verbindungen eingesetzt werden können, sind aromatische Ketone, aromatische Phosphinoxide oder aromatische Sulfoxide oder Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl) oder die in WO 2005/039246, US 2005/0069729, JP 2004/288381, EP 1205527, WO 2008/086851 oder WO 2013/041176, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Azacarbazolderivate, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2007/063754, WO 2008/056746, WO 2010/015306, WO 2011/057706, WO 2011/060859 oder WO 2011/060877, Zinkkomplexe, z. B. gemäß EP 652273 oder WO 2009/062578, Diazasilol- bzw. Tetraazasilol-Derivate, z. B. gemäß WO 2010/054729, Diazaphosphol-Derivate, z. B. gemäß WO 2010/054730, verbrückte Carbazol-Derivate, z. B. gemäß WO 2011/042107, WO 2011/060867, WO 2011/088877 und WO 2012/143080, Triphenylenderivate, z. B. gemäß WO 2012/048781, oder Dibenzofuranderivate, z. B. gemäß WO 2015/169412, WO 2016/015810, WO 2016/023608, WO 2017/148564 oder WO 2017/148565. Ebenso kann ein weiterer phosphoreszierender Emitter, welcher kürzerwellig als der eigentliche Emitter emittiert, als Co-Host in der Mischung vorhanden sein oder eine Verbindung, die nicht oder nicht in wesentlichem Umfang am Ladungstransport teilnimmt, wie beispielsweise in WO 2010/108579 beschrieben.

Insbesondere eignen sich in Kombination mit der erfindungsgemäßen Verbindung als Co-Matrix-Material Verbindungen, welche eine große Bandlücke aufweisen und selber nicht oder zumindest nicht in wesentlichem Maße am Ladungstransport der emittierenden Schicht teilnehmen. Es handelt sich bei solchen Materialien bevorzugt um reine Kohlenwasserstoffe. Beispiele für solche Materialien finden sich beispielsweise in der WO 2009/124627 oder in der WO 2010/006680.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439 und der noch nicht offen gelegten Anmeldung EP 16179378.1 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Explizite Beispiele für phosphoreszierende Dotanden sind in der folgenden Tabelle aufgeführt.

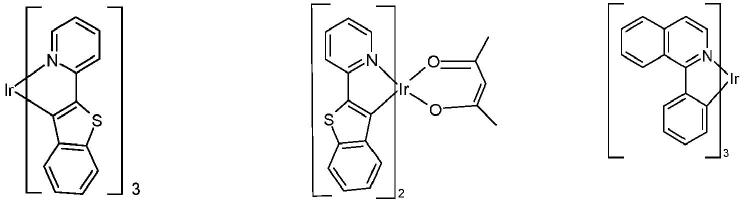

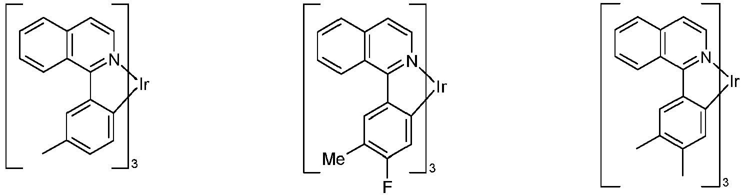

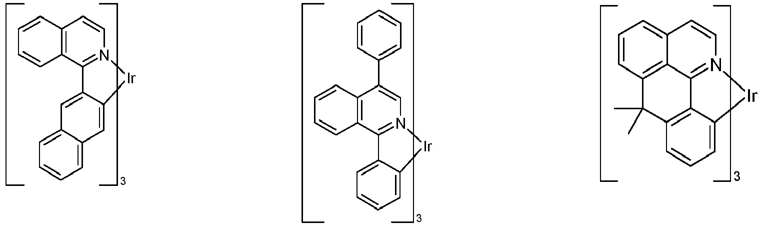

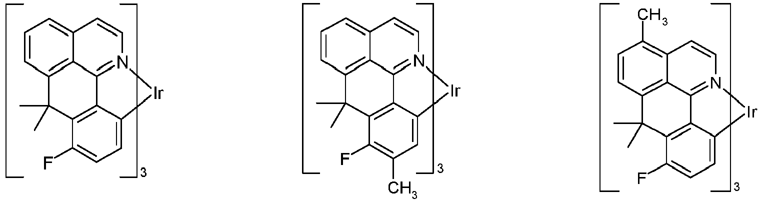

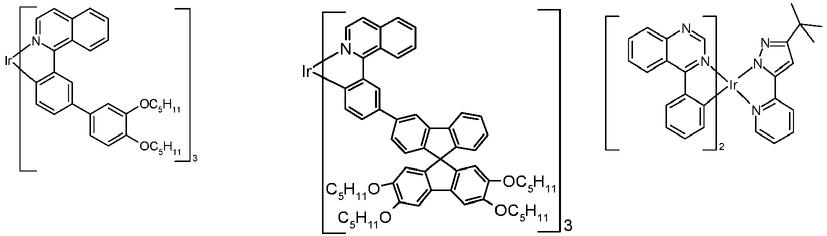

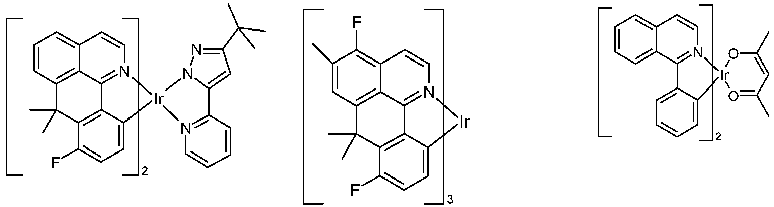

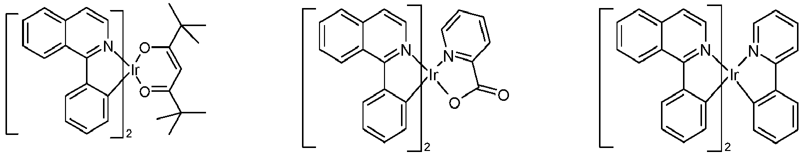

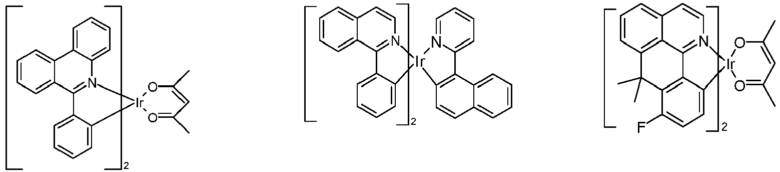

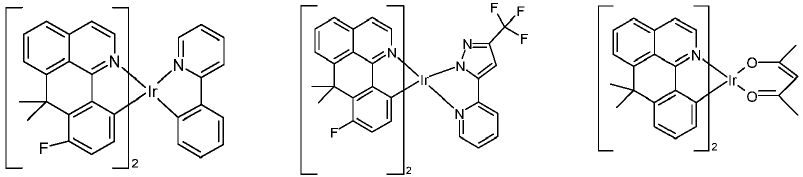

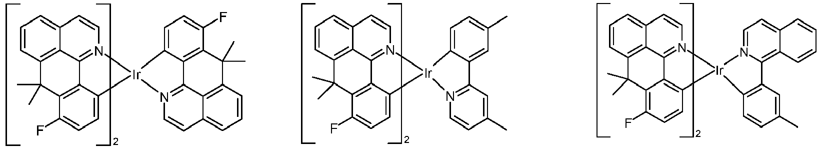

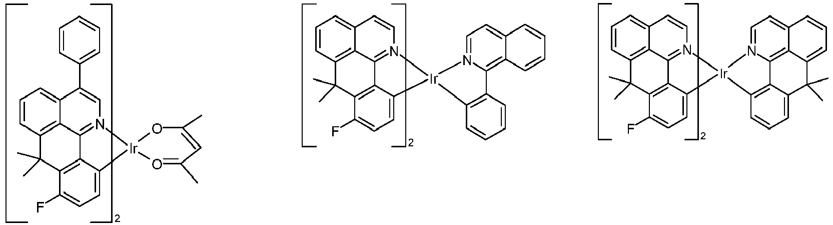

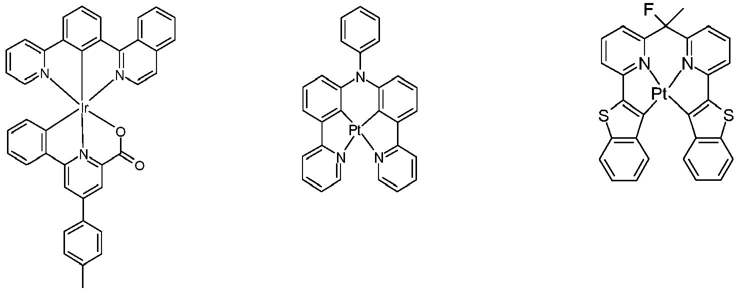

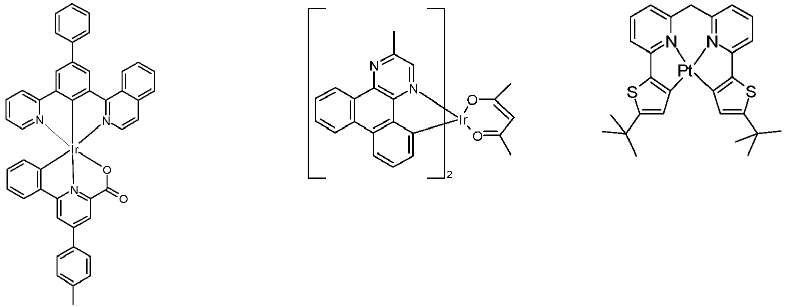

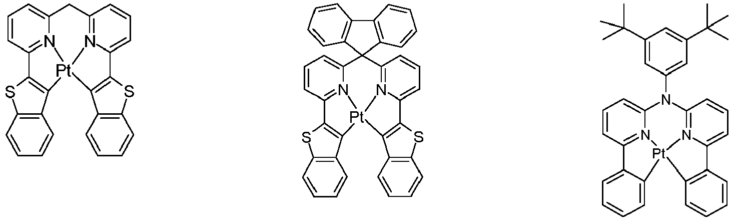

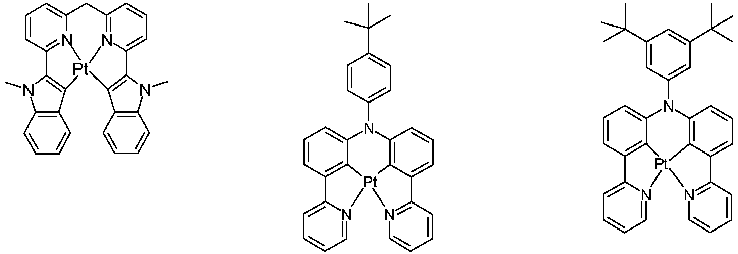

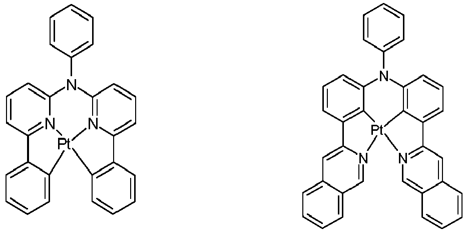

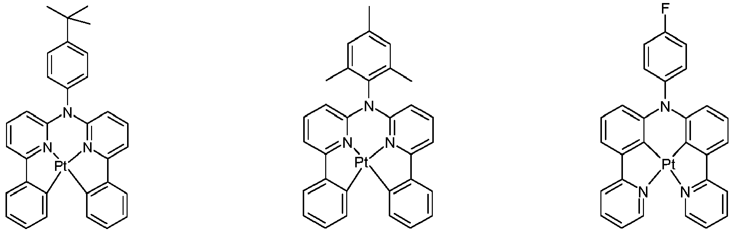

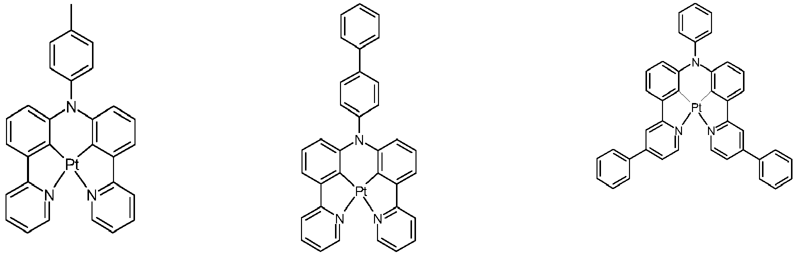

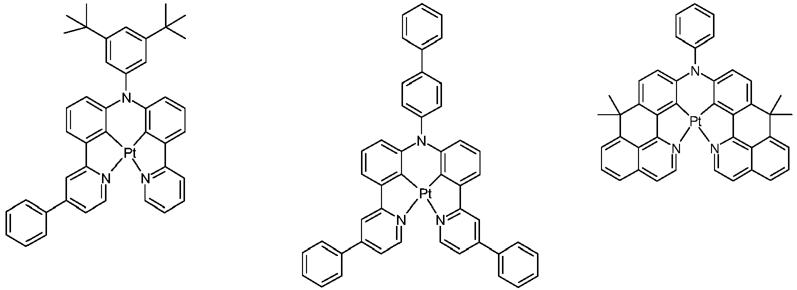

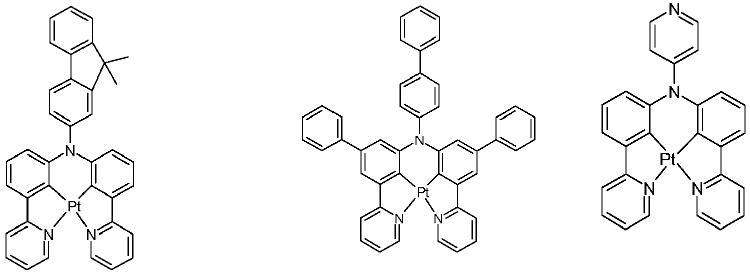

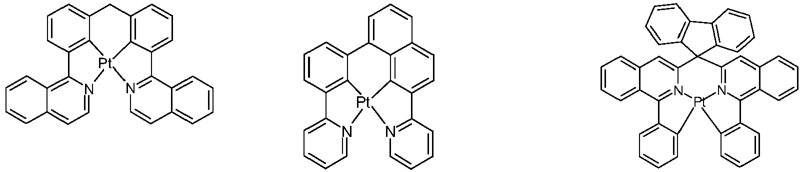

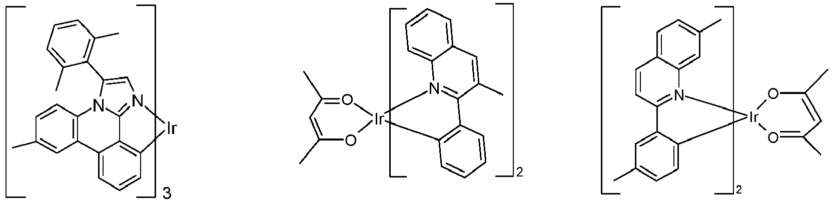

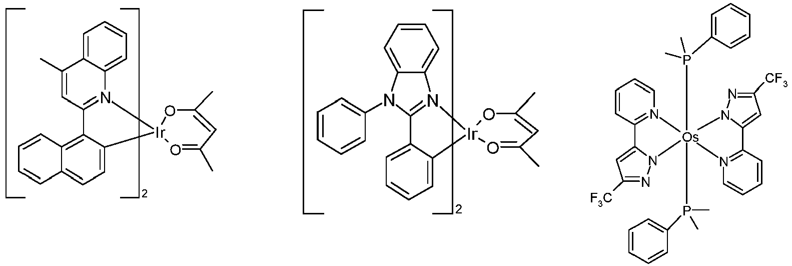

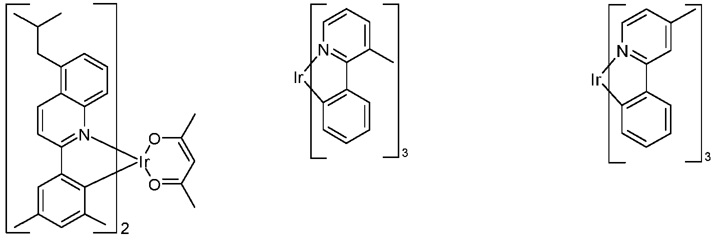

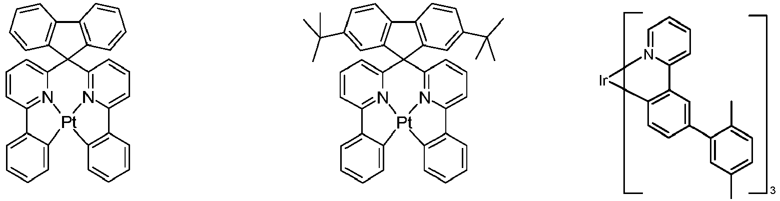

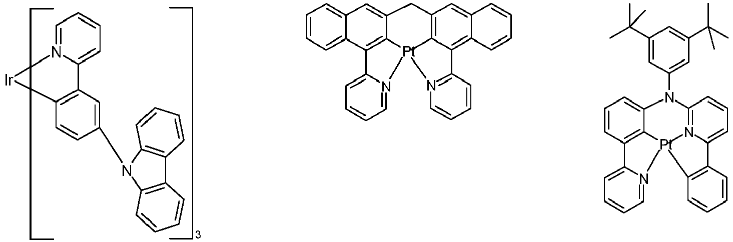

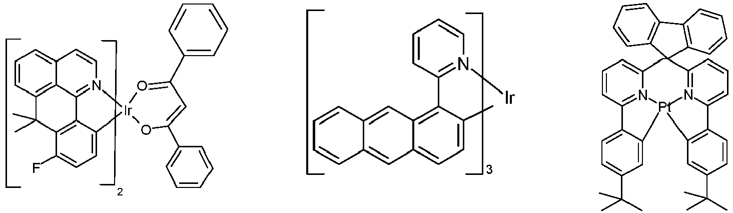

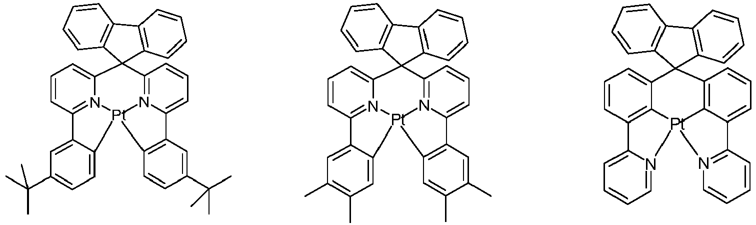

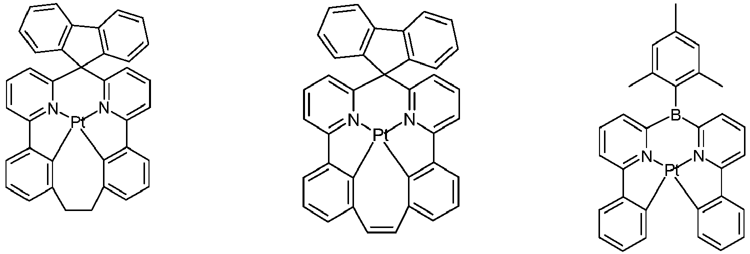

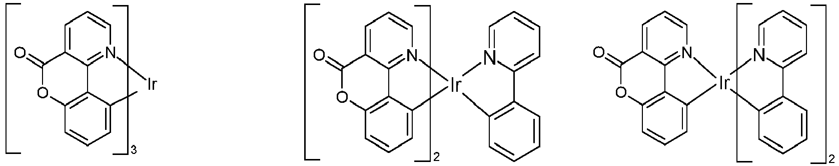

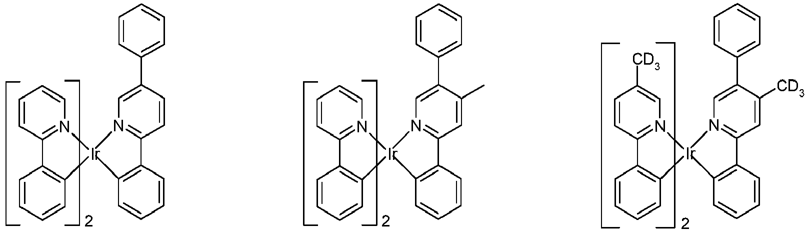

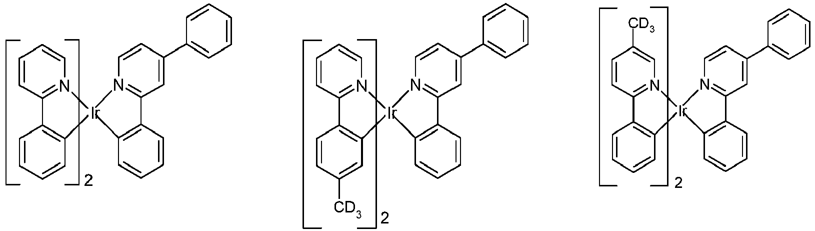

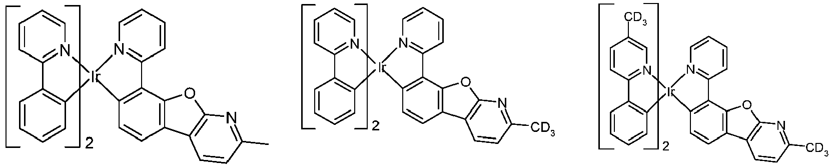

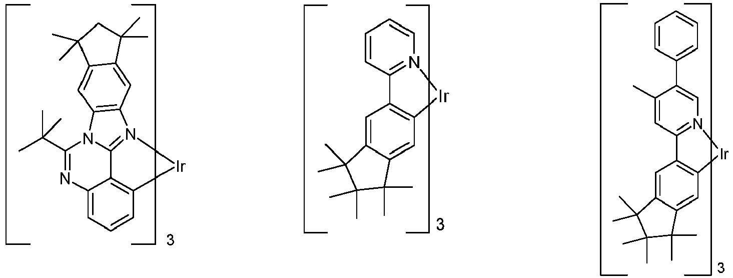

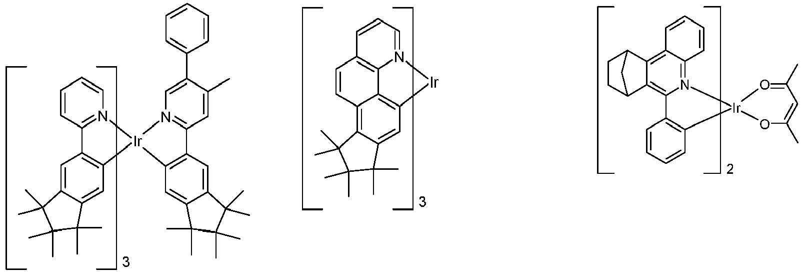

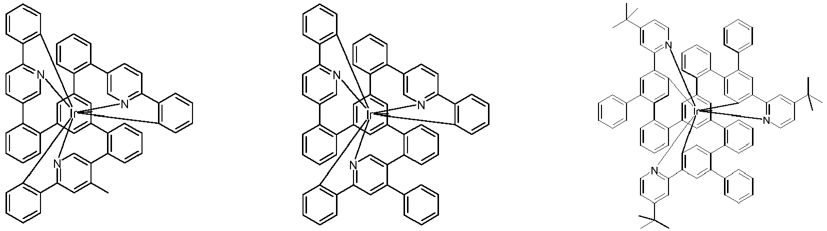

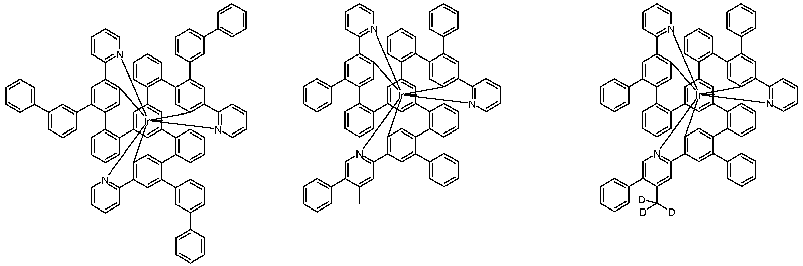

Die erfindungsgemäßen Verbindungen sind insbesondere auch geeignet als Matrixmaterialien für phosphoreszierende Emitter in organischen Elektrolumineszenzvorrichtungen, wie sie z. B. in WO 98/24271, US 2011/0248247 und US 2012/0223633 beschrieben sind. In diesen mehrfarbigen Display-Bauteilen wird eine zusätzliche blaue Emissionsschicht vollflächig auf alle Pixel, auch diejenigen mit einer von Blau verschiedenen Farbe, aufgedampft. Dabei wurde überraschend gefunden, dass die erfindungsgemäßen Verbindungen, wenn sie als Matrixmaterialien für das rote und/oder grüne Pixel, insbesondere für das rote Pixel, eingesetzt werden, zusammen mit der aufgedampften blauen Emissionsschicht zu weiterhin sehr guter Emission führen.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. den oben ausgeführten bevorzugten Ausführungsformen einsetzen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden.

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Offsetdruck, LITI (Light Induced Thermal Imaging, Thermotransferdruck), Ink-Jet Druck (Tintenstrahldruck) oder Nozzle Printing, hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen und die erfindungsgemäßen organischen Elektrolumineszenzvorrichtungen zeichnen sich durch einen oder mehrere der folgenden überraschenden Vorteile gegenüber dem Stand der Technik aus:
1. Die erfindungsgemäßen Verbindungen, eingesetzt als Matrixmaterial für phosphoreszierende Emitter, führen zu langen Lebensdauern.
2. Die erfindungsgemäßen Verbindungen führen zu hohen Effizienzen. Dies gilt insbesondere, wenn die Verbindungen als Matrixmaterial für einen phosphoreszierenden Emitter eingesetzt werden.
3. Die erfindungsgemäßen Verbindungen führen zu geringen Betriebsspannungen. Dies gilt insbesondere, wenn die Verbindungen als Matrixmaterial für einen phosphoreszierenden Emitter eingesetzt werden.

Diese oben genannten Vorteile gehen nicht mit einer Verschlechterung der weiteren elektronischen Eigenschaften einher.

Die Erfindung wird durch die nachfolgenden Beispiele näher erläutert, ohne sie dadurch einschränken zu wollen. Der Fachmann kann aus den Schilderungen die Erfindung im gesamten offenbarten Bereich ausführen und ohne erfinderisches Zutun weitere erfindungsgemäße Verbindungen herstellen und diese in elektronischen Vorrichtungen verwenden bzw. das erfindungsgemäße Verfahren anwenden.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können von ALDRICH bzw. ABCR bezogen werden. Die bei den nicht kommerziell erhältlichen Edukten angegeben Nummern sind die entsprechenden CAS-Nummern.

### a) 1-Chlor-7-(9-phenyl-9H-carbazol-3-yl)-10H-phenothiazin

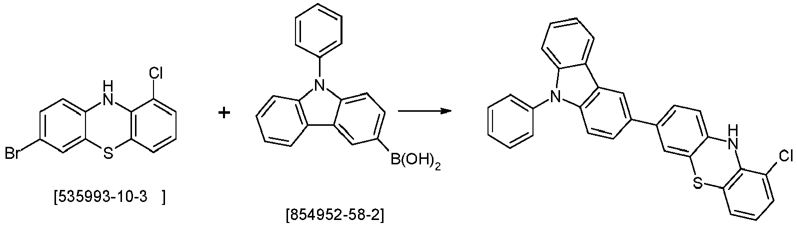

20.9 g (67 mmol) 7-Brom-1-chlor-10H-phenothiazin, 17 g (664 mmol) 2- N-Phenylcarbazol-3-boronsäure und 13.7 g (100 mmol) Natriumtetraborat werden in 100 mL THF und 60 ml Wasser gelöst und entgast. Es wird mit 0.9 g (1.3 mmol) Bis(triphenylphosphin)palladium(II)chlorid und 1 g (20 mmol) Hydraziniumhydroxid versetzt. Die Reaktionsmischung wird anschließend bei 70 °C für 48 h unter Schutzgasatmosphäre gerührt. Die abgekühlte Lösung wird mit Toluol aufgestockt, mehrmals mit Wasser gewaschen, getrocknet und eingeengt. Das Produkt wird via Säulenchromatographie an Kieselgel mit Toluol/Heptan (1:2) gereinigt. Ausbeute: 19.8 g (40 mmol), 62 % der Theorie.

Analog dazu werden die folgenden Verbindungen hergestellt:

| | **Edukt 1** | **Edukt 2** | **Produkt** | **Ausbeute** |
|---|---|---|---|---|
| 1a | 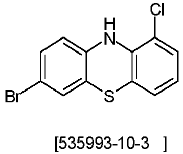 | 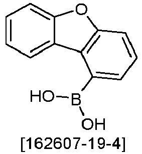 | 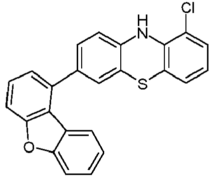 | 34% |
| 2a | 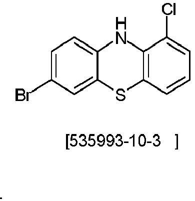 | 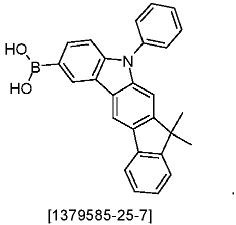 | 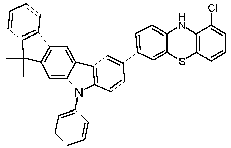 | 36% |
| 3a | 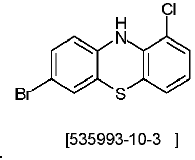 | 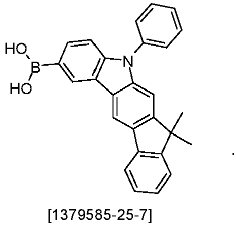 | 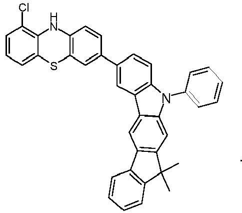 | 48% |
| 4a | 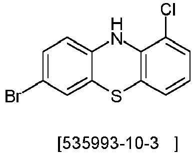 | 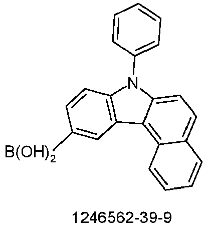 | 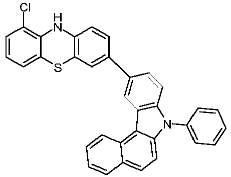 | 46% |
| 5a | 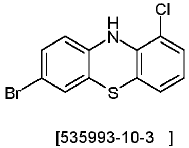 | 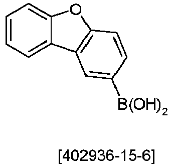 | 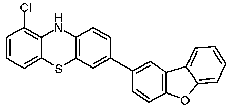 | 47% |
| 6a | 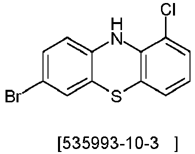 | 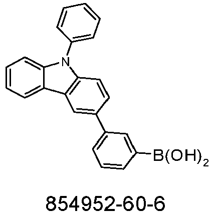 | 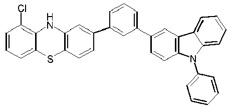 | 44% |
| 7a | 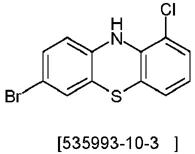 | 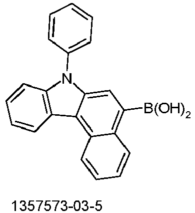 | 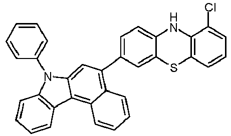 | 38% |
| 8a | 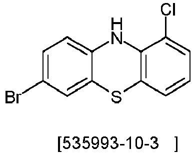 | 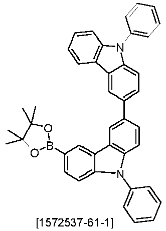 | 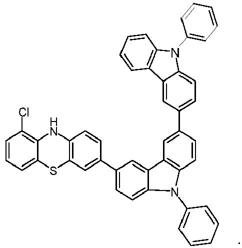 | |
| 9a | 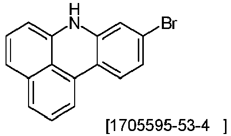 | 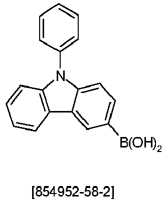 | 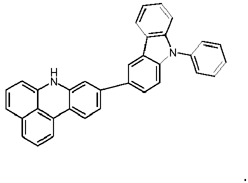 | 36% |
| 10a | 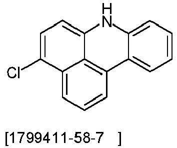 | 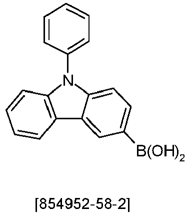 | 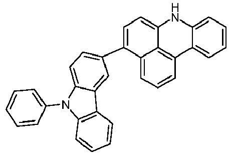 | 53% |
| 11a | 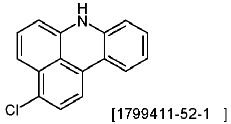 | 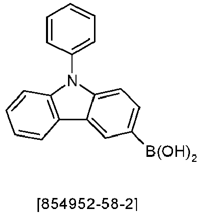 | 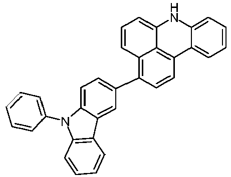 | 42% |
| 12a | 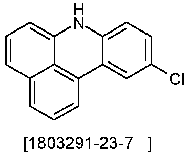 | 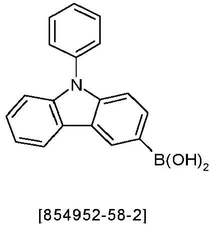 | 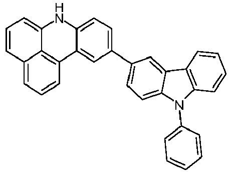 | 51% |
| 13a | 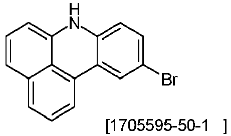 | 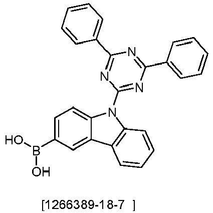 | 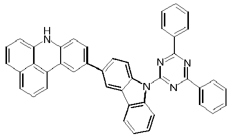 | 50% |
| 14a | 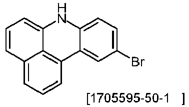 | 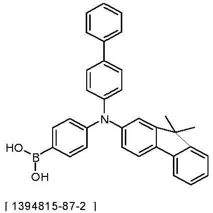 | 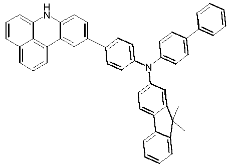 | 63% |
| 15a | 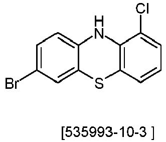 | 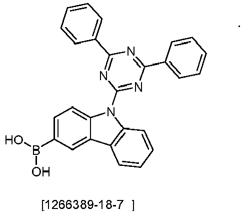 | 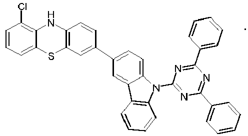 | 52% |
| 16a | 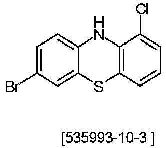 | 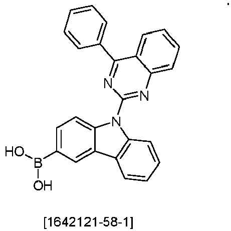 | 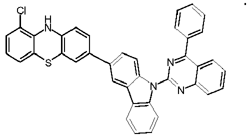 | 56% |
| 17a | 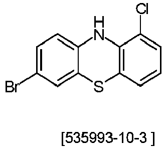 | 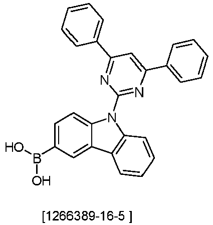 | 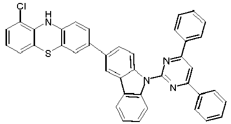 | 50% |
| 18a | 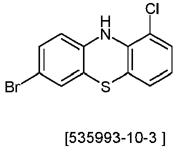 | 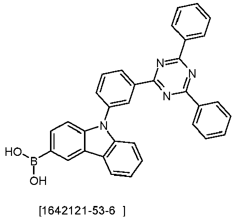 | 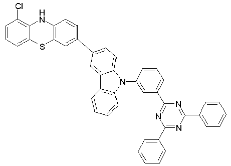 | 47% |
| 19a | 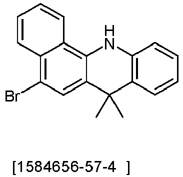 | 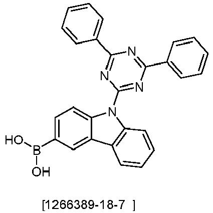 | 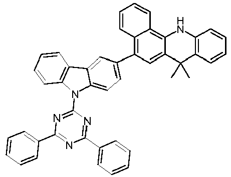 | 42% |
| 20a | 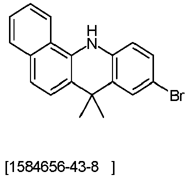 | 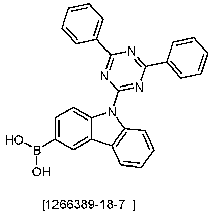 | 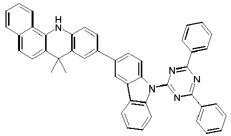 | 40% |

### b) 10-(8-Brom-naphth-1-yl)-10H-phenoxazin

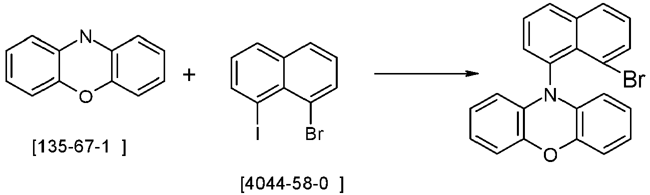

Unter Schutzgas werden 16.1 g (88 mmol) 10H-Phenoxazin, 29 g (88 mmol) 1-Brom-8-iodbenzol und 0.8 g (0.88 mmol) Tris(dibenzylidenaceton)-dipalladium in 500 ml Toluol suspendiert. Die Reaktionsmischung wird 8 h unter Rückfluss erhitzt. Nach Erkalten wird die organische Phase abgetrennt, dreimal mit 200 mL Wasser gewaschen und anschließend zur Trockene eingeengt. Das Produkt wird via Säulenchromatographie an Kieselgel mit Toluol/Heptan (2:2) gereinigt. Die Reinheit beträgt 98.0 %. Ausbeute: 26 g (67 mmol), 77 % der Theorie.

Analog dazu werden die folgenden Verbindungen hergestellt:

| | **Edukt &** | **Edukt 2** | **Produkt** | **Ausbeute** |
|---|---|---|---|---|
| 2b | 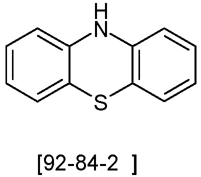 | 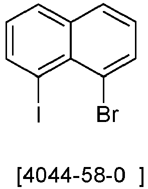 | 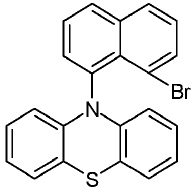 | 66% |
| 3b | 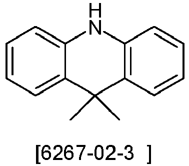 | 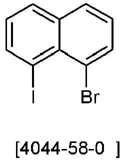 | 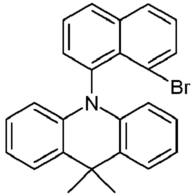 | 69% |
| 4b | 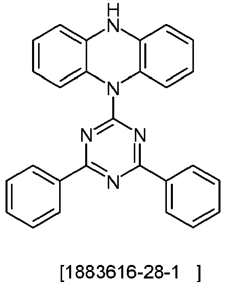 | 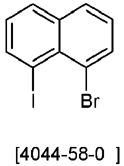 | 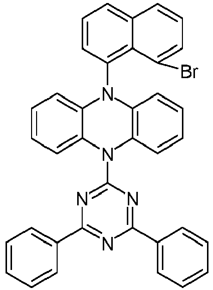 | 62% |
| 5b | 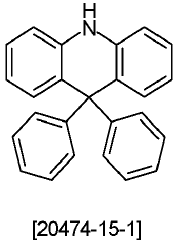 | 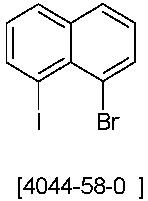 | 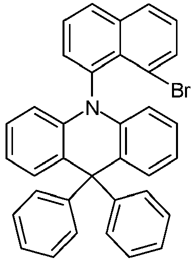 | 64% |
| 6b | 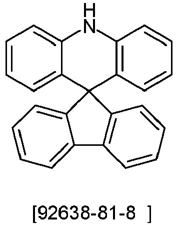 | 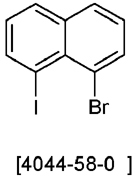 | 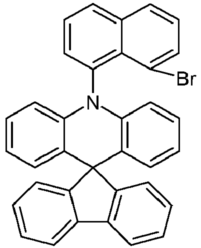 | 71% |
| 7b | 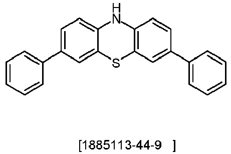 | 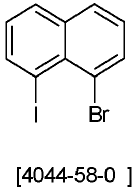 | 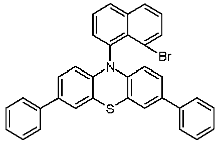 | 73% |
| 8b | 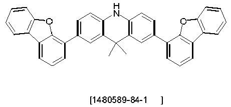 | 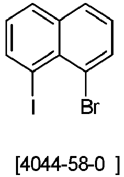 | 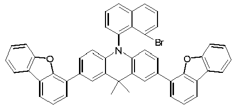 | 67% |
| 9b | 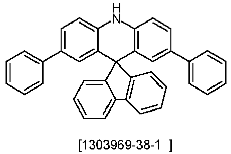 | 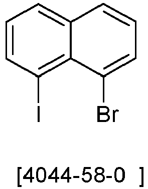 | 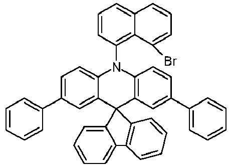 | 71% |
| 10b | 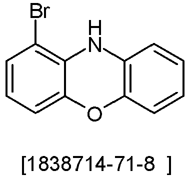 | 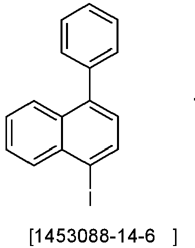 | 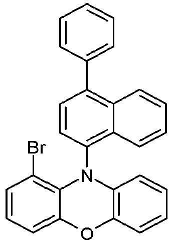 | 70% |
| 11b | 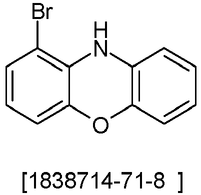 | 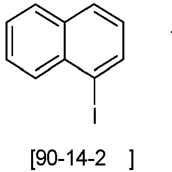 | 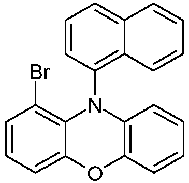 | 74% |
| 12b | 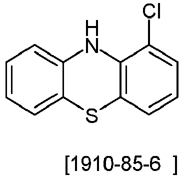 | 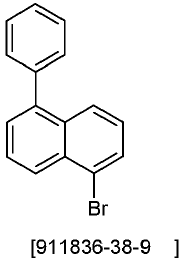 | 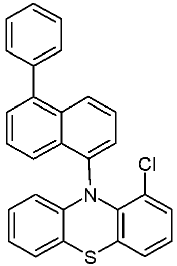 | 79% |
| 13b | 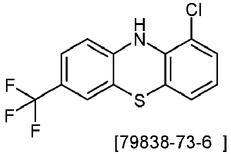 | 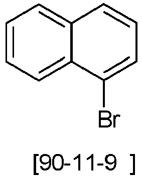 | 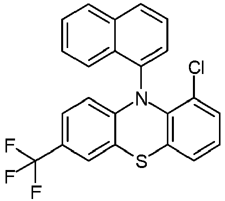 | 70% |
| 15b | 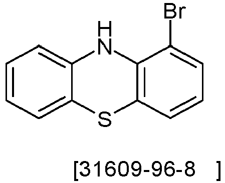 | 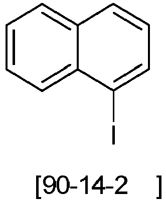 | 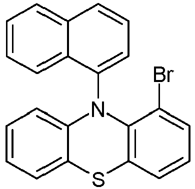 | 79% |
| 16b | 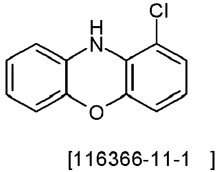 | 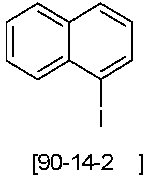 | 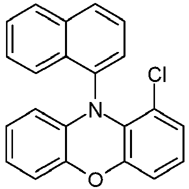 | 73% |
| 17b | 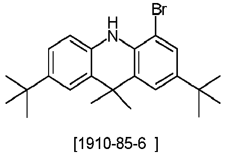 | 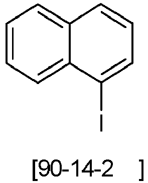 | 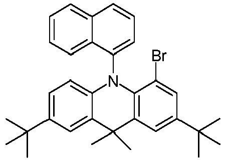 | 68% |
| 18b | 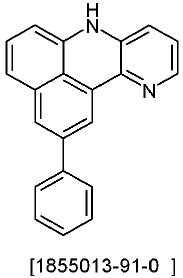 | 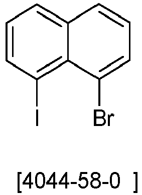 | 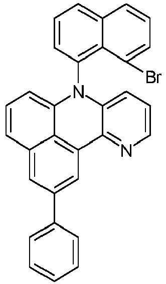 | 71% |
| 19b | 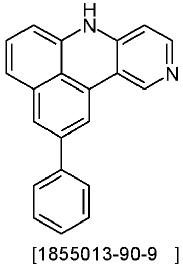 | 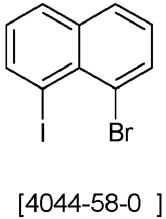 | 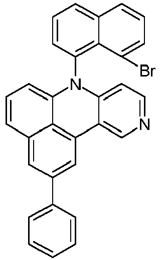 | 70% |
| 20b | 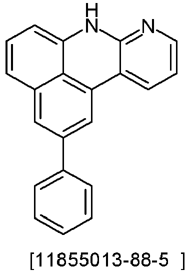 | 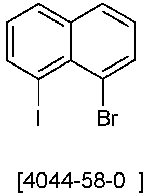 | 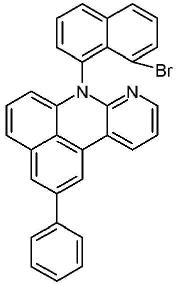 | 72% |
| 21b | 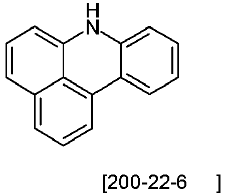 | 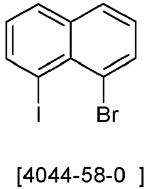 | 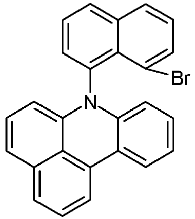 | 70% |
| 22b | 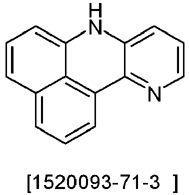 | 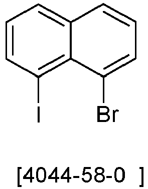 | 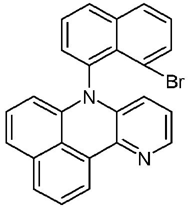 | 74% |
| 23b | 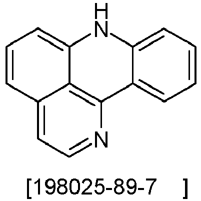 | 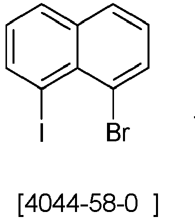 | 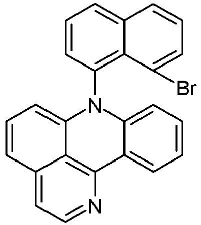 | 75% |
| 24b | 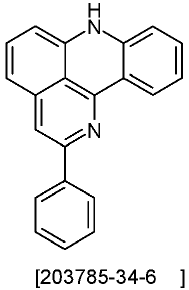 | 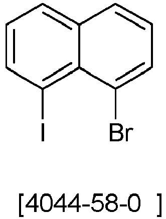 | 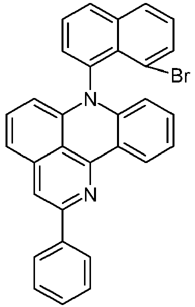 | 77% |
| 25b | 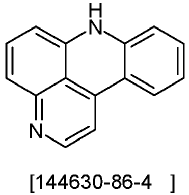 | 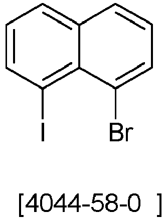 | 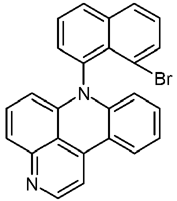 | 79% |
| 26b | 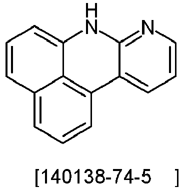 | 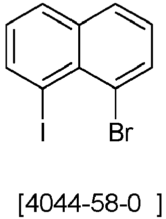 | 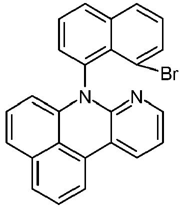 | 75% |
| 27b | 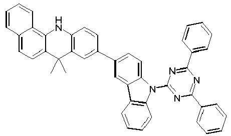 | 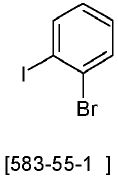 | 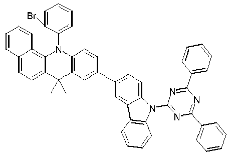 | 70% |
| 28b | 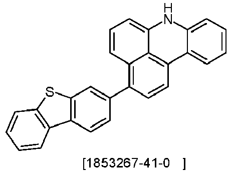 | 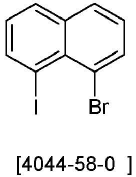 | 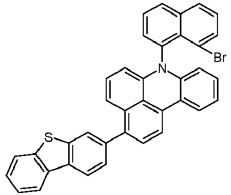 | 74% |
| 29b | 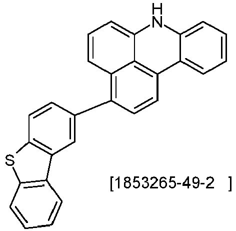 | 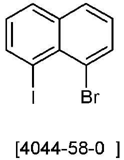 | 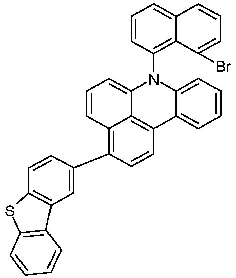 | 71% |
| 30b | 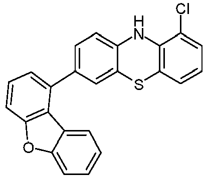 | 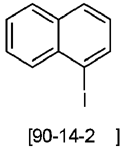 | 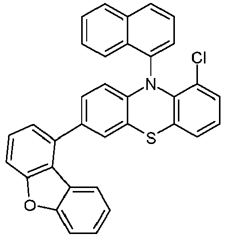 | 64% |
| 31b | 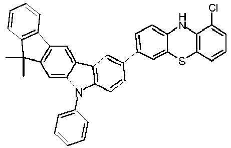 | 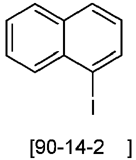 | 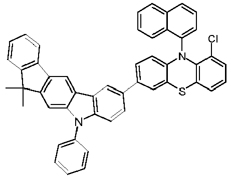 | 70% |
| 32b | 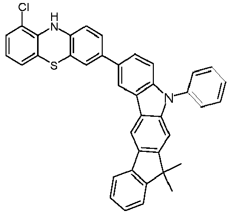 | 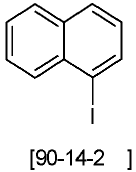 | 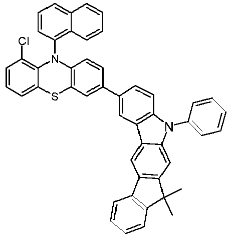 | 73% |
| 33b | 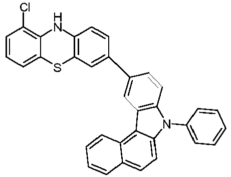 | 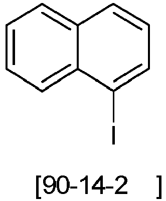 | 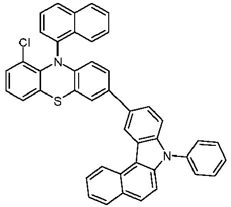 | 64% |
| 34b | 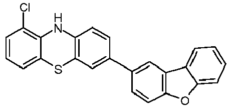 | 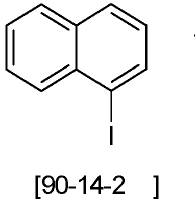 | 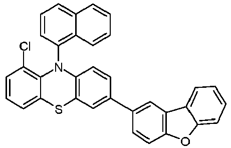 | 58% |
| 35b | 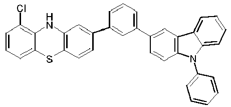 | 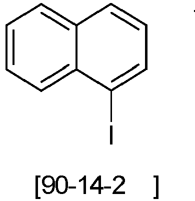 | 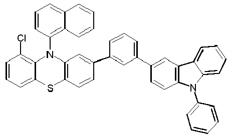 | 767% |
| 36b | 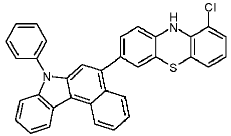 | 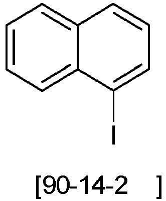 | 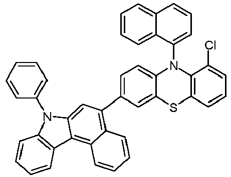 | 72% |
| 37b | 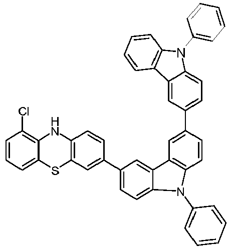 | 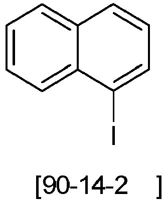 | 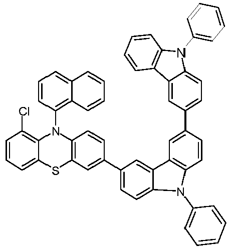 | 61% |
| 38b | 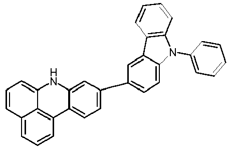 | 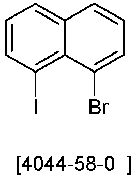 | 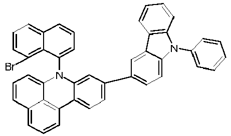 | 66% |
| 39b | 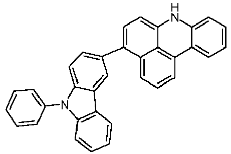 | 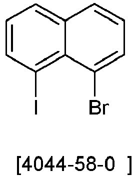 | 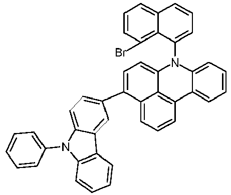 | 70% |
| 40b | 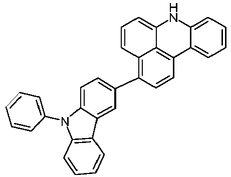 | 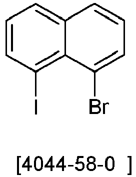 | 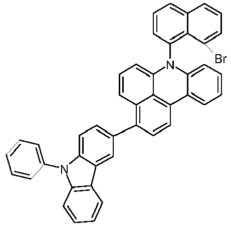 | 72% |
| 41b | 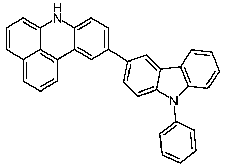 | 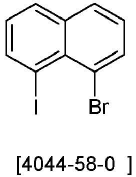 | 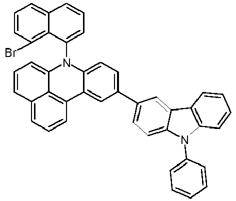 | 71% |
| 42b | 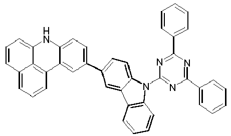 | 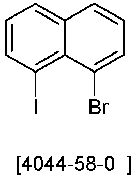 | 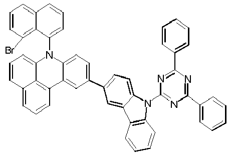 | 64% |
| 43b | 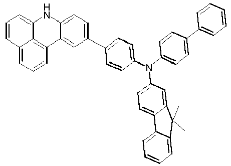 | 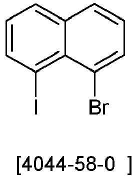 | 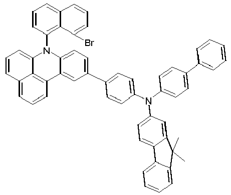 | 58% |
| 44b | 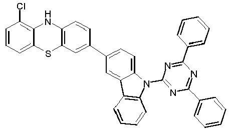 | 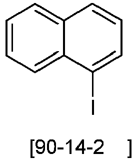 | 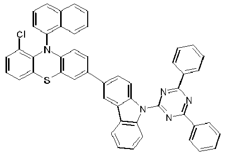 | 59% |
| 45b | 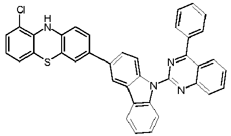 | 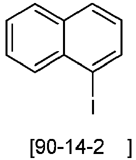 | 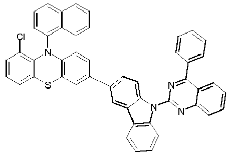 | 63% |
| 46b | 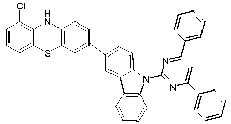 | 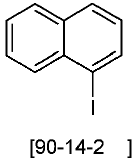 | 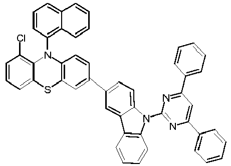 | 64% |
| 47b | 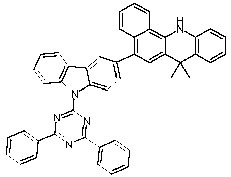 | 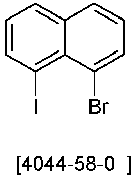 | 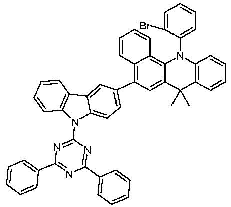 | 60% |
| 48b | 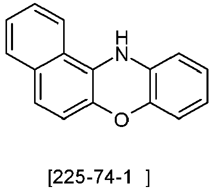 | 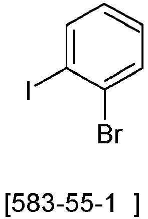 | 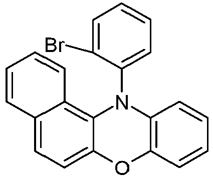 | 76% |
| 49b | 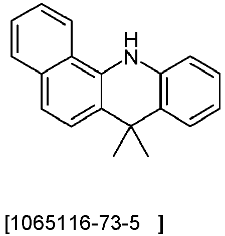 | 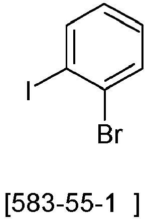 | 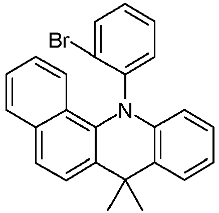 | 73% |
| 50b | 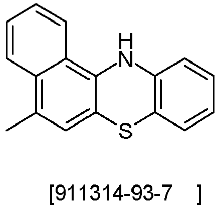 | 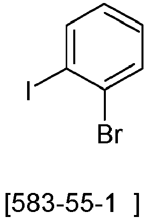 | 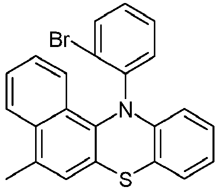 | 70% |
| 51b | 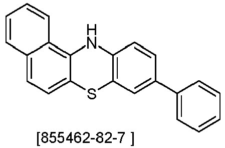 | 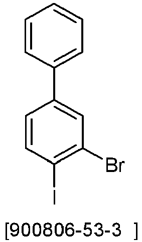 | 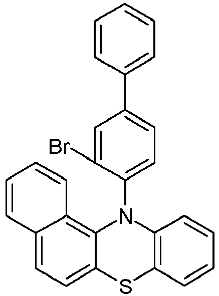 | 71% |
| 52b | 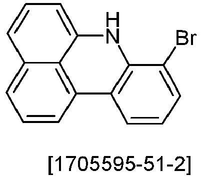 | 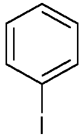 | 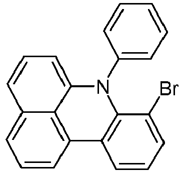 | 65% |
| 53b | 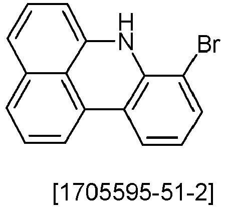 | 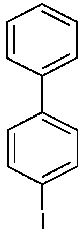 | 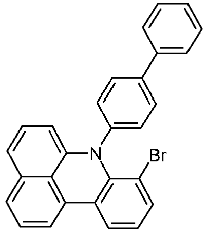 | 64% |
| 54b | 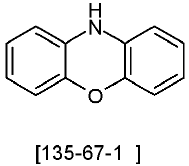 | 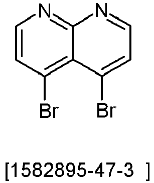 | 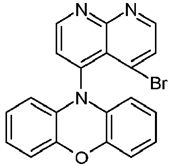 | 53% |
| 55b | 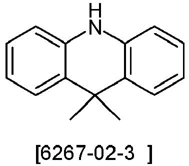 | 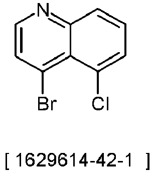 | 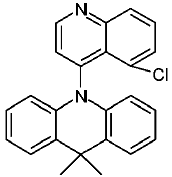 | 65% |

### c) 5-(8-Brom-naphth-1-yl)-5,10-dihydrophenazin

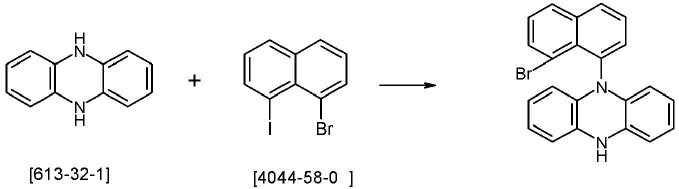

Unter Schutzgas werden 15.8 g (87.8 mmol) 9,10-Dihydrophenazin, 20 g (87 mmol) 1-Brom-8-iod-naphthalin und 0.8 g (0.88 mmol) Tris(dibenzylidenaceton)-dipalladium in 500 ml Toluol suspendiert. Die Reaktionsmischung wird 8 h unter Rückfluss erhitzt. Nach Erkalten wird die organische Phase abgetrennt, dreimal mit 200 mL Wasser gewaschen und anschließend zur Trockene eingeengt. Das Produkt wird via Säulenchromatographie an Kieselgel mit Toluol/Heptan (2:2) gereinigt. Die Reinheit beträgt 94.0 %. Ausbeute: 21 g (56 mmol), 65 % der Theorie.

Analog dazu werden die folgenden Verbindungen hergestellt:

| **Bsp.** | **Edukt 1** | **Edukt 1** | **Produkt** | **Ausbeute** |
|---|---|---|---|---|
| 1c | 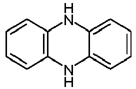 | 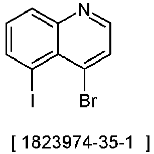 | 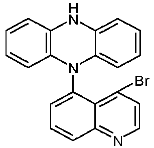 | 60% |
| 2c | 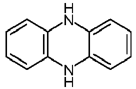 | 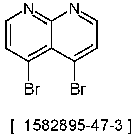 | 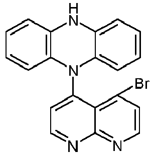 | 62% |
| 3c | 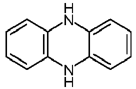 | 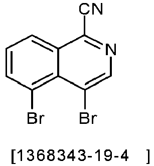 | 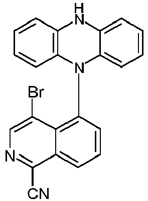 | 67% |
| 4c | 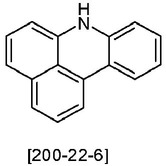 | 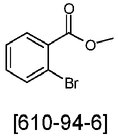 | 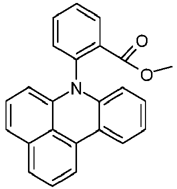 | 60% |
| 5c | 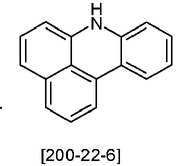 | 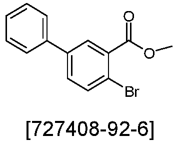 | 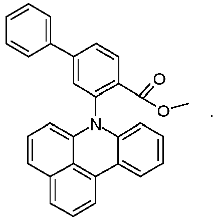 | 65% |
| 6c | 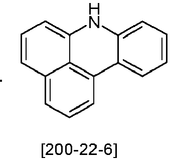 | 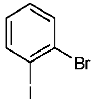 | 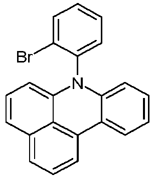 | 67% |

### d) 5-(8-Brom-naphth-1-yl)-10-(4,6-diphenyl-[1,3,5]triazin-2-yl)-5,10-dihydrophenazin

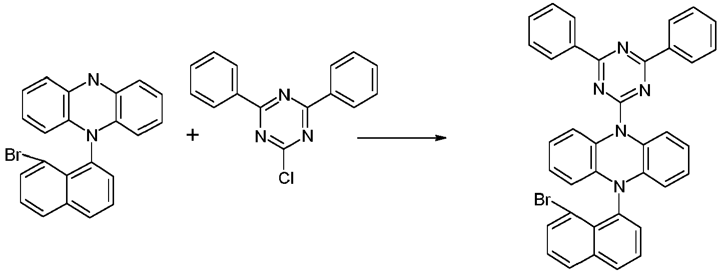

11.22 g (29 mmol) 5-(8-Brom-naphth-1-yl)-5,10-dihydrophenazin werden in 225 ml Dimethylformamid unter Schutzgasatmosphäre gelöst und mit 1.5 g (37.5 mmol) NaH, 60%ig in Mineralöl, versetzt. Nach 1 h bei Raumtemperatur wird eine Lösung von 2-Chlor-4,6-diphenyl-[1,3,5]-triazin (8.5 g, 31.75 mmol) in 75 mL Dimethylformamid zugetropft. Das Reaktionsgemisch wird 12 h bei Raumtemperatur gerührt, dann auf Eis gegossen und dreimal mit Dichlormethan extrahiert. Die vereinigten organischen Phasen werden über Na₂SO₄ getrocknet und eingeengt. Der Rückstand wird mit Toluol heiß extrahiert. Ausbeute: 14.3 g (23 mmol), 80 % der Theorie.

Analog dazu werden die folgenden Verbindungen hergestellt:

| | **Edukt 1** | **Edukt 2** | **Produkt** | **Ausbeute** |
|---|---|---|---|---|
| 1d | 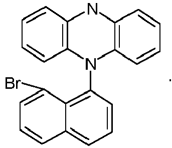 | 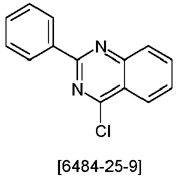 | 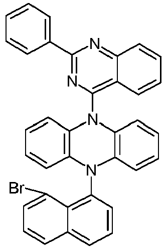 | 82% |
| 2d | 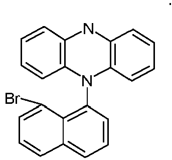 | 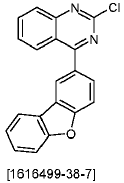 | 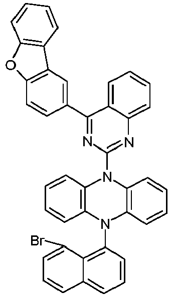 | 83% |
| 3d | 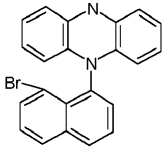 | 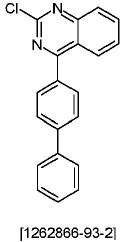 | 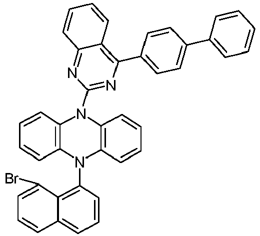 | 81% |
| 4d | 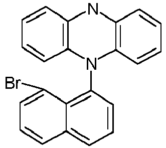 | 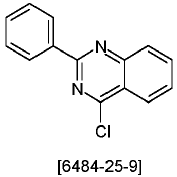 | 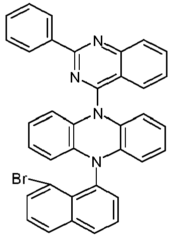 | 86% |
| 5d | 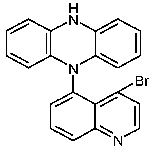 | 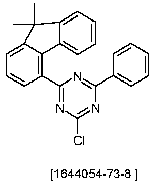 | 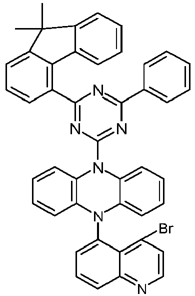 | 80% |
| 6d | 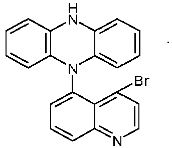 | 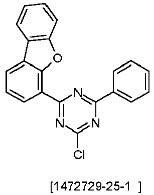 | 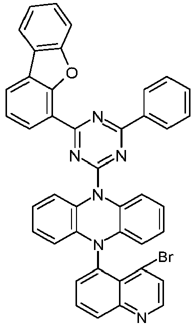 | 84% |
| 7d | 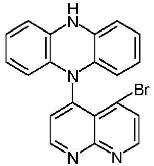 | 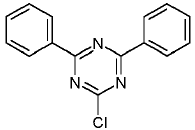 | 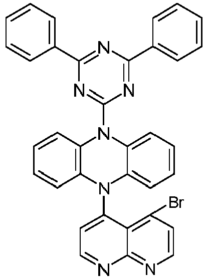 | 86% |
| 8d | 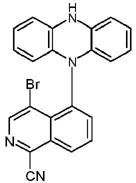 | 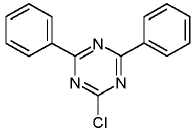 | 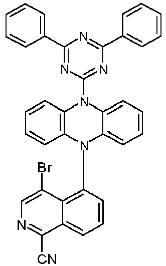 | 80% |

### e) Cyclisierung

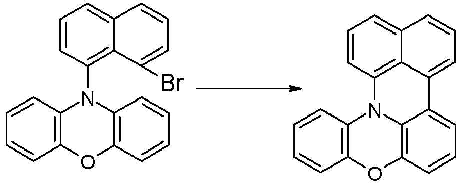

Unter Schutzgas werden 58 g (150 mmol) 10-(8-Brom-naphth-1-yl)-10H-phenoxazin in 500 mL Dimethylacetamid gelöst. Zur dieser Lösung werden 2.4 g (6.5 mmol) Tricyclohexyltetrafluoroborat und 701 mg (3.1 mmol) Pd(OAc)₂ zugegeben. Anschließend wird das Gemisch bei 120 °C für 9 h gerührt, dann auf Raumtemperatur gekühlt und mit Dichlormethan extrahiert. Die vereinigten organischen Phasen werden über Na₂SO₄ getrocknet und eingeengt. Der Rückstand wird mit Toluol heiß extrahiert, aus Toluol umkristallisiert und abschließend im Hochvakuum sublimiert. Die Ausbeute beträgt 33 g (107 mmol), 72% der Theorie.

Analog dazu werden die folgenden Verbindungen hergestellt:

| | **Edukt 1** | **Produkt** | **Ausbeute** |
|---|---|---|---|
| 2e | 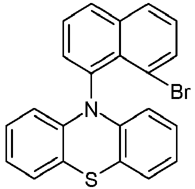 | 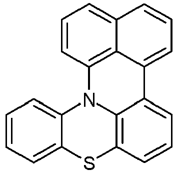 | 81% |
| 3e | 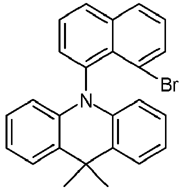 | 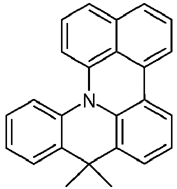 | 78% |
| 4e | 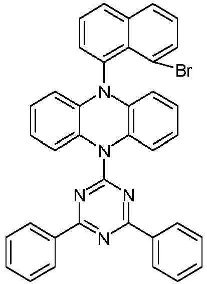 | 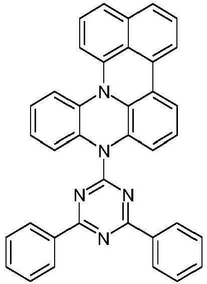 | 78% |
| 5e | 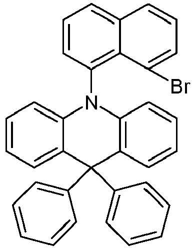 | 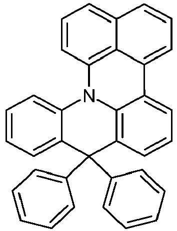 | 75% |
| 6e | 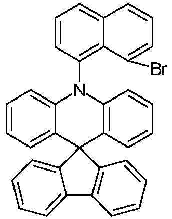 | 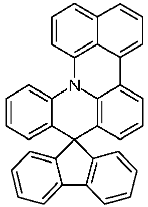 | 81% |
| 7e | 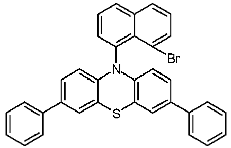 | 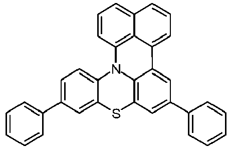 | 79% |
| 8e | 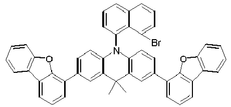 | 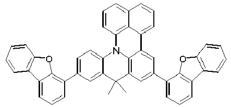 | 76% |
| 9e | 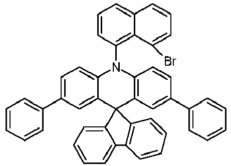 | 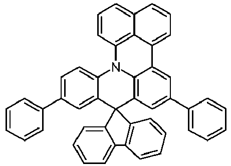 | 74% |
| 10e | 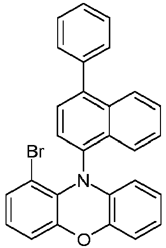 | 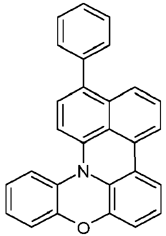 | 75% |
| 11e | 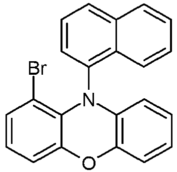 | 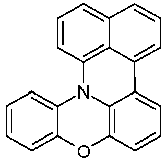 | 71% |
| 12e | 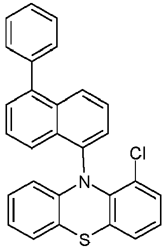 | 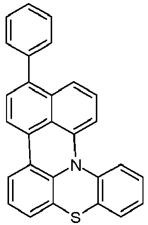 | 72% |
| 13e | 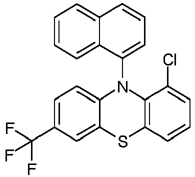 | 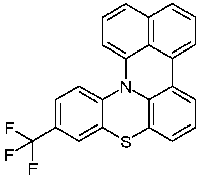 | 70% |
| 14e | 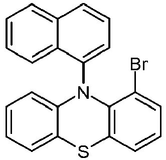 | 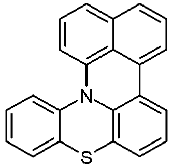 | 62% |
| 15e | 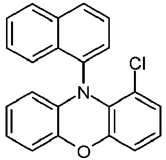 | 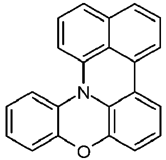 | 63% |
| 16e | 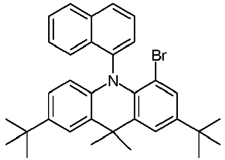 | 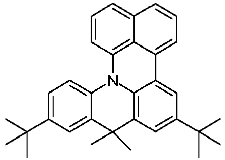 | 73% |
| 17e | 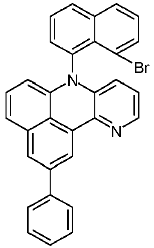 | 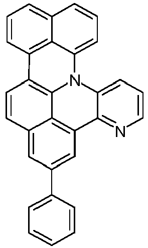 | 76% |
| 18e | 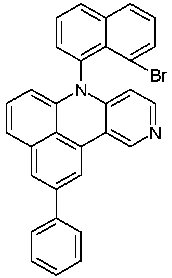 | 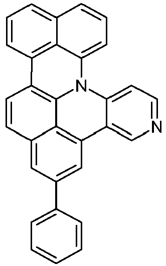 | 77% |
| 19e | 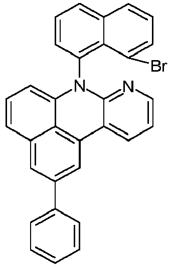 | 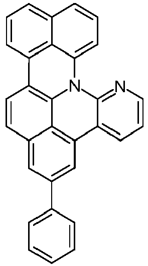 | 71% |
| 20e | 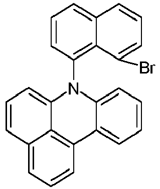 | 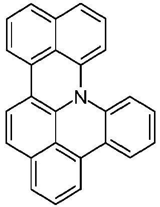 | 73% |
| 21e | 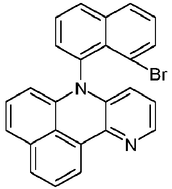 | 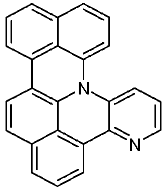 | 69% |
| 22e | 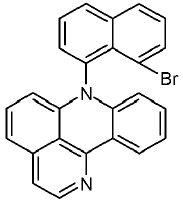 | 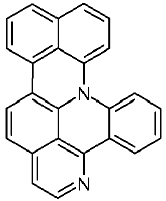 | 72% |
| 23e | 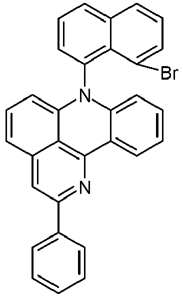 | 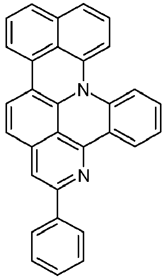 | 56% |
| 24e | 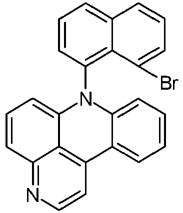 | 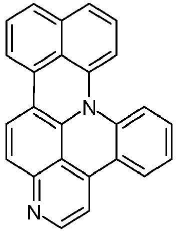 | 63% |
| 25e | 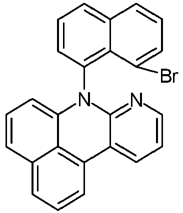 | 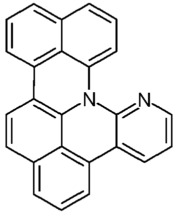 | 80% |
| 26e | 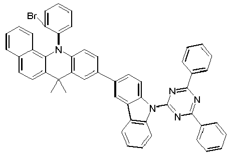 | 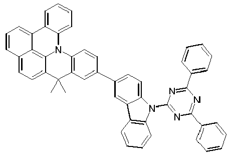 | 84% |
| 27e | 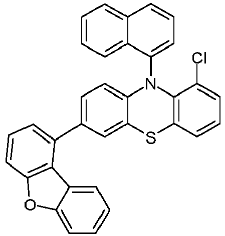 | 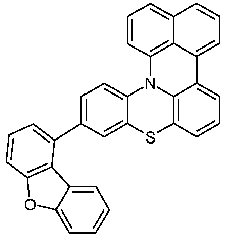 | 73% |
| 28e | 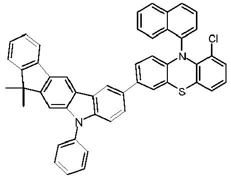 | 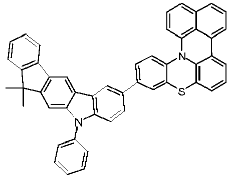 | 73% |
| 29e | 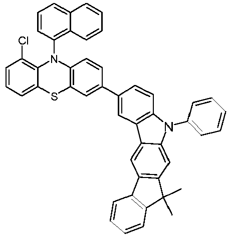 | 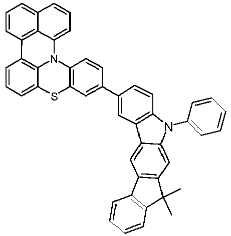 | 76% |
| 30e | 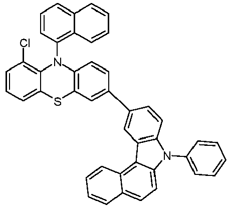 | 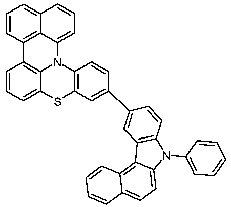 | 70% |
| 31e | 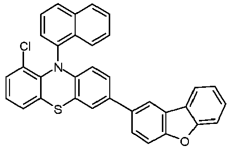 | 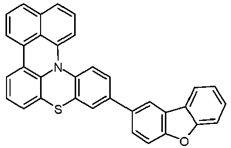 | 78% |
| 32e | 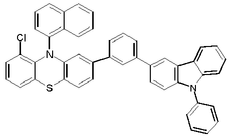 | 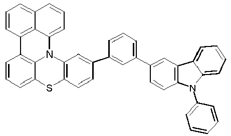 | 73% |
| 33e | 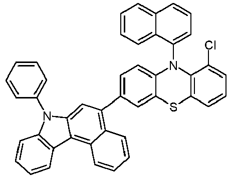 | 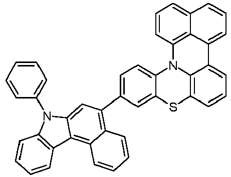 | 65% |
| 34e | 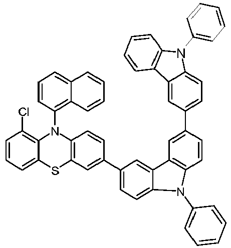 | 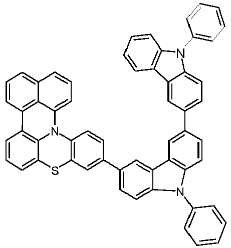 | 64% |
| 35e | 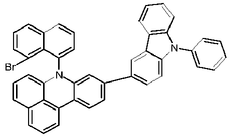 | 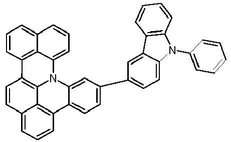 | 73% |
| 36e | 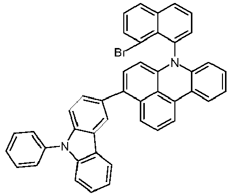 | 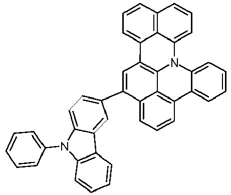 | 76% |
| 37e | 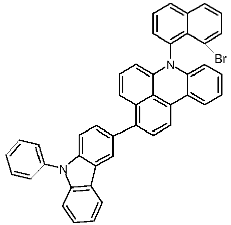 | 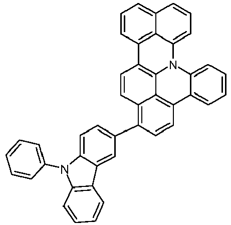 | 79% |
| 38e | 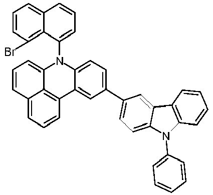 | 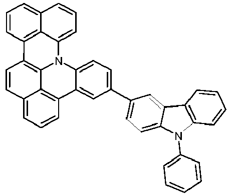 | 70% |
| 39e | 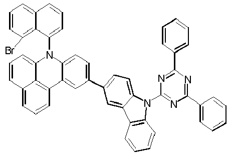 | 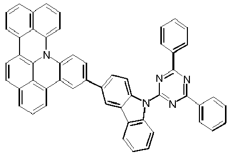 | 76% |
| 40e | 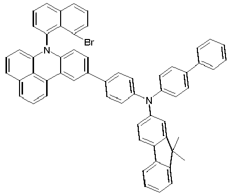 | 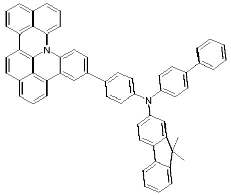 | 67% |
| 41e | 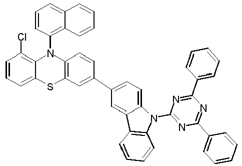 | 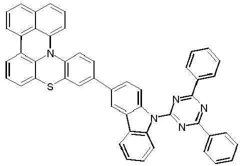 | 71% |
| 42e | 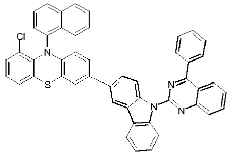 | 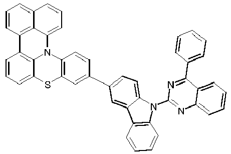 | 63% |
| 43e | 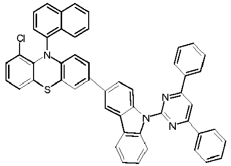 | 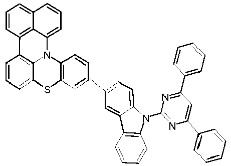 | 60% |
| 44e | 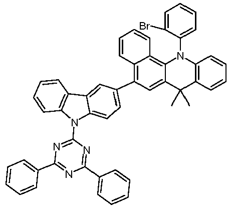 | 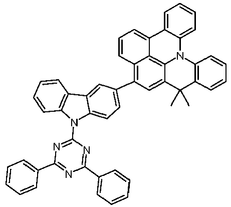 | 80% |
| 45e | 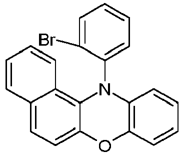 | 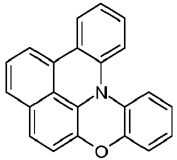 | 80% |
| 46e | 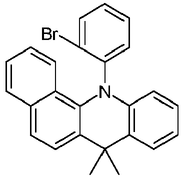 | 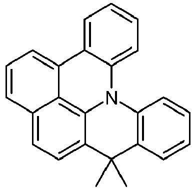 | 78% |
| 47e | 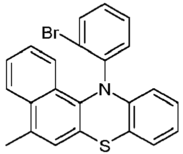 | 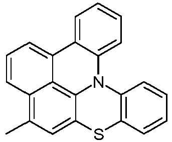 | 80% |
| 48e | 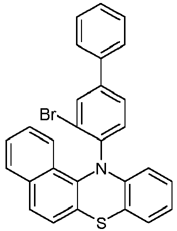 | 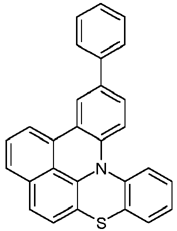 | 82% |
| 49e | 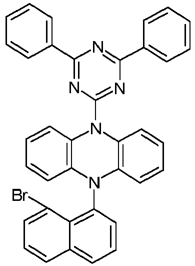 | 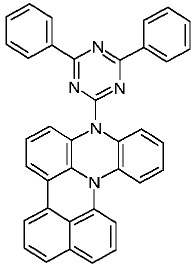 | 78% |
| 50e | 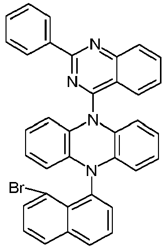 | 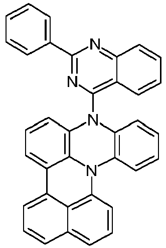 | 79% |
| 51e | 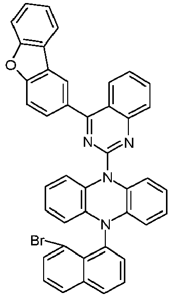 | 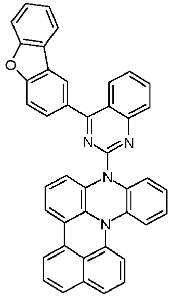 | 76% |
| 52e | 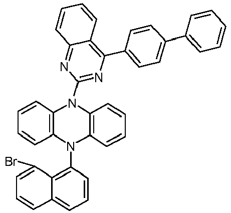 | 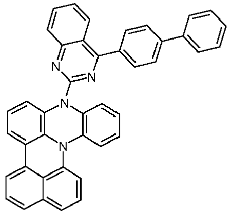 | 81% |
| 53e | 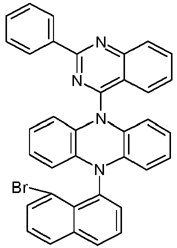 | 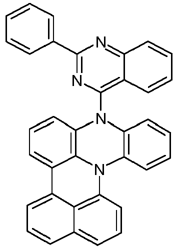 | 79% |
| 54e | 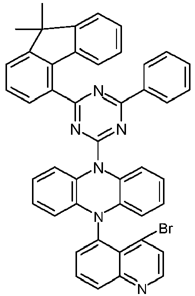 | 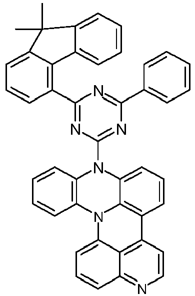 | 75% |
| 55e | 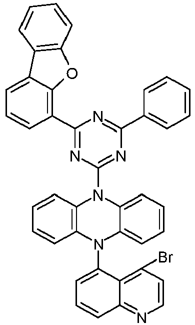 | 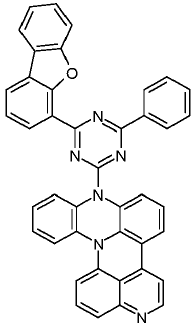 | 72% |
| 56e | 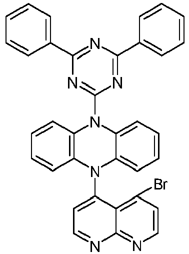 | 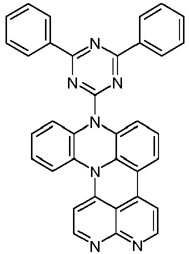 | 74% |
| 57e | 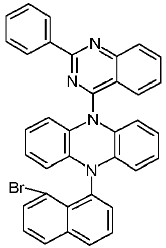 | 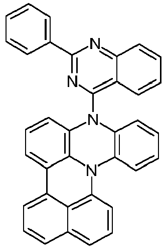 | 70% |
| 58e | 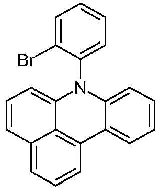 | 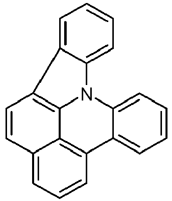 | 42% |
| 59e | 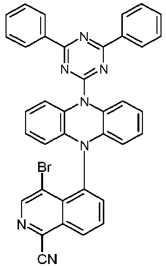 | 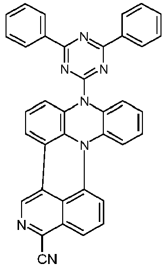 | 67% |
| 60e | 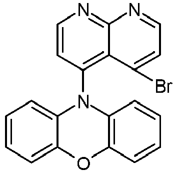 | 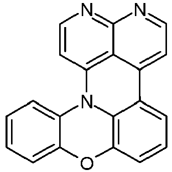 | 65% |
| 61e | 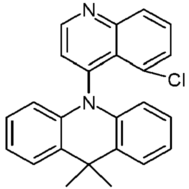 | 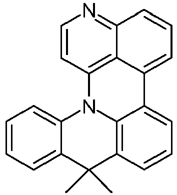 | 67% |

### f) 2-[2-(7-Aza-benzo[de]anthracen-7-yl)-phenyl]-propan-2-ol

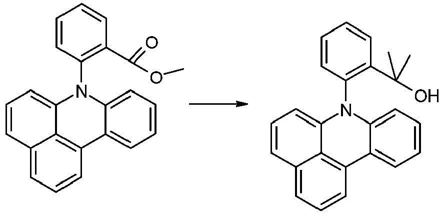

73 g (211 mmol) 2-(7-Aza-benzo[de]anthracen-7-yl)-benzoesäuremethyl-ester werden in 1500 mL getrocknetem THF gelöst und entgast. Es wird auf -78 °C gekühlt und innerhalb von 40 min. mit 569 mL (853 mmol) Methyllithium versetzt. Man lässt innerhalb 1 h auf -40 °C erwärmen und kontrolliert die Umsetzung via DC. Nach vollständiger Umsetzung wird bei -30 °C vorsichtig mit MeOH gequencht. Die Reaktionslösung wird auf ein Drittel des Volumens eingeengt, mit 1 L CH₂Cl₂ versetzt, gewaschen, die organische Phase über MgSO₄ getrocknet und eingeengt. Die Ausbeute beträgt 63 g (180 mmol), 87% der Theorie.

Analog dazu wird die folgende Verbindung hergestellt:

| | **Edukt 1** | **Produkt** | **Ausbeute** |
|---|---|---|---|
| 1f | 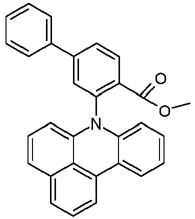 | 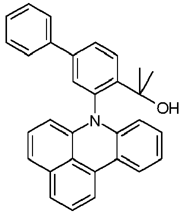 | 80% |

### g ) Cyclisierung

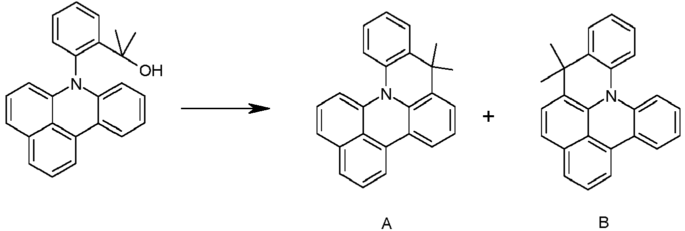

15.5 g (43.6 mmol) 2-[2-(7-Aza-benzo[de]anthracen-7-yl)-phenyl]-propan-2-ol werden in 1200 mL entgastem Toluol gelöst, mit einer Suspension aus 40 g Polyphosphorsäure und 28 mL Methansulfonsäure versetzt und für 1 h auf 60 °C erhitzt. Der Ansatz wird abgekühlt und mit Wasser versetzt. Es fällt ein Feststoff aus, der in CH₂Cl₂/THF (1:1) gelöst wird. Die Lösung wird mit 20%iger NaOH vorsichtig alkalisiert, die Phasen werden getrennt und über MgSO₄ getrocknet. Die Mischung aus A und B wird chromatographisch getrennt. Die Ausbeute beträgt 11.6 g (34 mmol), 64% der Theorie, A 31%, B 33%.

Analog dazu wird die folgende Verbindung hergestellt:

| | **Edukt 1** | **Produkt 1** | **Produkt 2** | **Ausbeute** |
|---|---|---|---|---|
| 1g | 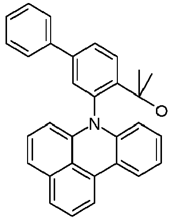 | 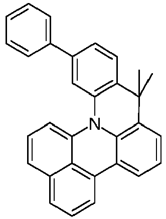 | 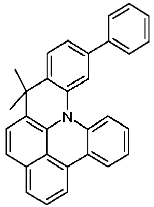 | 28%: 29% |

### h) Bromierung

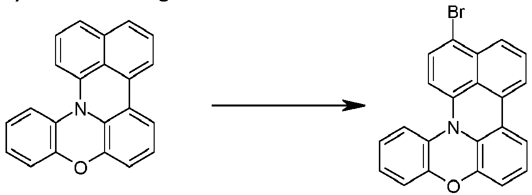

12.2 g (41 mmol) des Produktes e werden in 300 ml Chloroform gelöst. Diese Lösung wird portionsweise bei -50 °C unter Lichtausschluss mit 7 g (42 mmol) NBS versetzt und anschließend 1 h gerührt. Nach dem Entfernen des Lösungsmittels im Vakuum wird der Rückstand in Heptan/ Toluol 3:1 ausgerührt und heiß abfiltriert. Ausbeute: 12.5 g (32 mmol), 81% der Theorie.

Analog dazu werden die folgenden Verbindungen hergestellt:

| | **Edukt 1** | **Produkt** | **Ausbeute** |
|---|---|---|---|
| 2h | 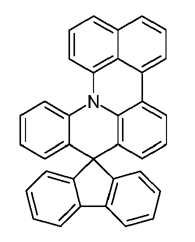 | 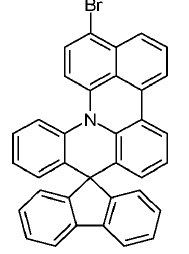 | 83% |
| 3h | 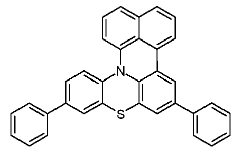 | 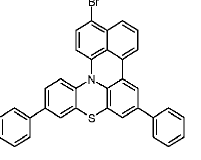 | 81% |
| 4h | 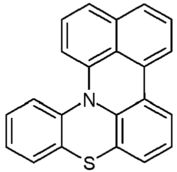 | 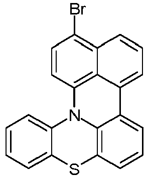 | 78% |
| 5h | 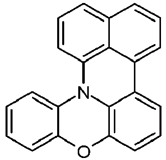 | 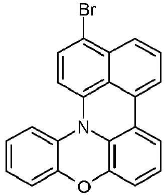 | 69% |
| 6h | 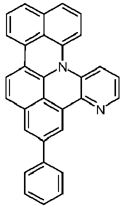 | 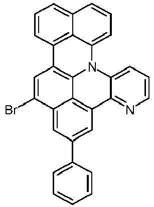 | 52% |
| 7h | 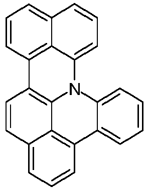 | 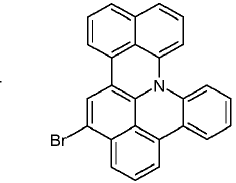 | 43% |
| 8h | 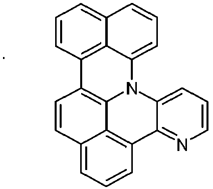 | 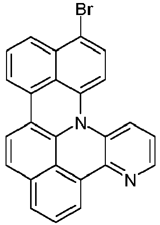 | 39% |
| 9h | 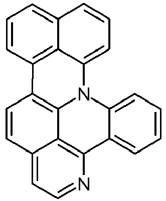 | 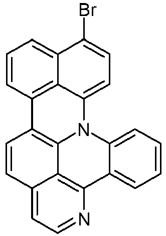 | 67% |
| 10h | 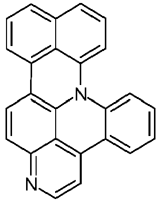 | 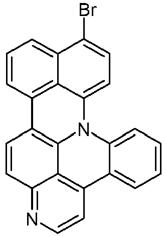 | 65& |
| 11h | 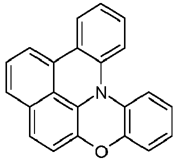 | 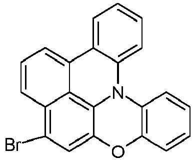 | 72% |
| 12h | 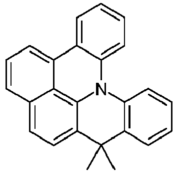 | 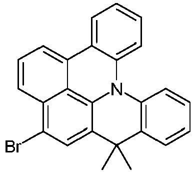 | 61% |
| 13h | 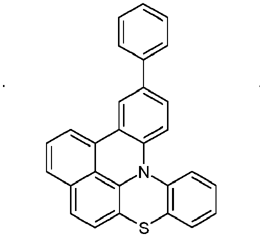 | 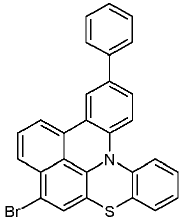 | 77% |
| 14h | 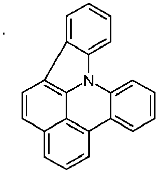 | 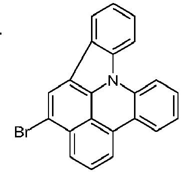 | 68% |

### j) Suzuki Kupplung

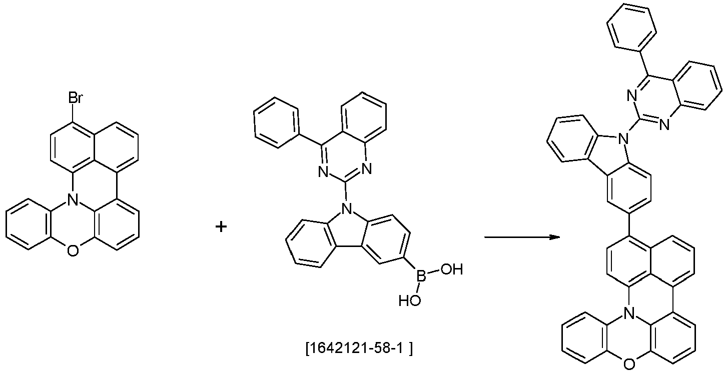

Unter Schutzgas werden 62 g (150 mmol) B-[9-(4-phenyl-2-chinazolinyl)-9H-carbazol-3-yl]-boronsäure, 55 g (145 mmol) des Produktes **h** und 36 g (340 mmol) Natriumcarbonat in 1000 mL Ethylenglycoldimethylether und 280 mL Wasser suspendiert. Zu dieser Suspension werden 1.8 g (1.5 mmol) Tetrakis(triphenylphosphin)-palladium(0) zugegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Das Produkt wird via Säulenchromatographie an Kieselgel mit Toluol/Heptan (1:2) gereinigt und abschließend im Hochvakuum (p = 5 × 10⁻⁷ mbar) sublimiert (Reinheit 99.9 %). Die Ausbeute beträgt 75 g (111 mmol), 60 % der Theorie.

Analog dazu werden die folgenden Verbindungen hergestellt:

| | **Edukt 1** | **Edukt 2** | **Produkt** | **Ausbeute** |
|---|---|---|---|---|
| 1j | 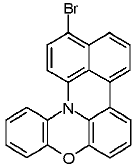 | 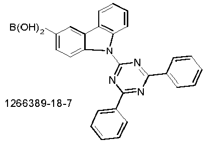 | 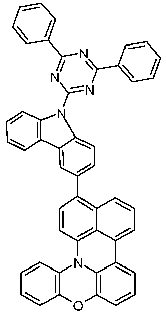 | 74% |
| 3j | 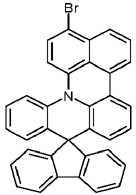 | 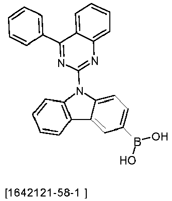 | 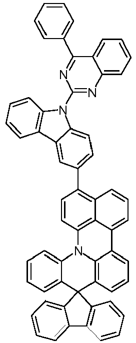 | 81% |
| 4j | 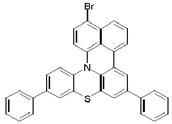 | 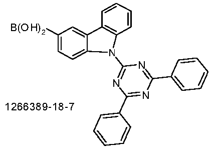 | 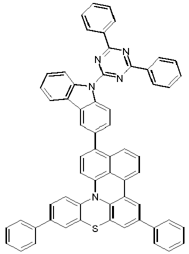 | 78% |
| 5j | 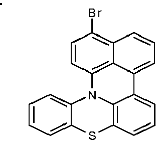 | 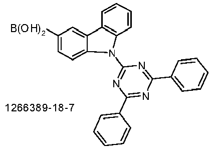 | 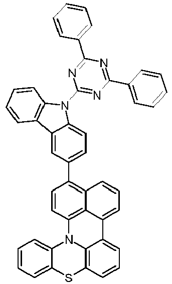 | 79% |
| 6j | 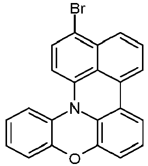 | 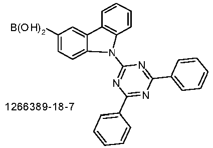 | 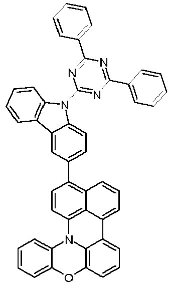 | 75% |
| 7j | 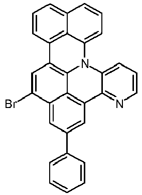 | 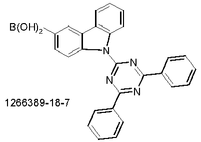 | 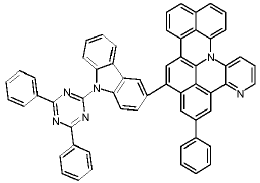 | 81% |
| 8j | 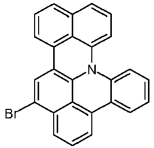 | 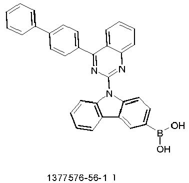 | 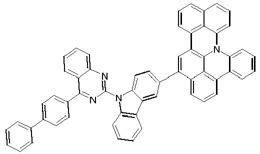 | 78% |
| 9j | 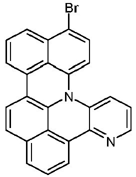 | 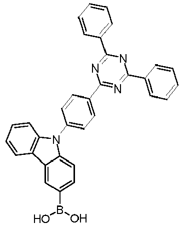 | 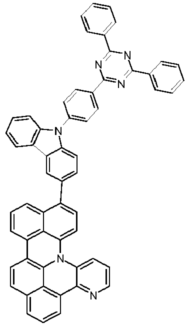 | 75% |
| 10j | 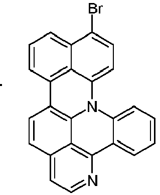 | 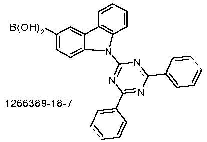 | 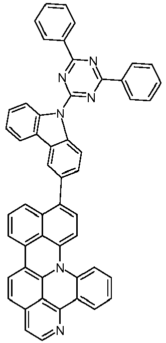 | 70% |
| 11j | 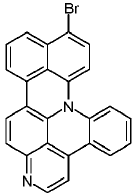 | 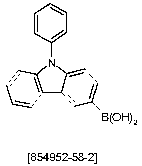 | 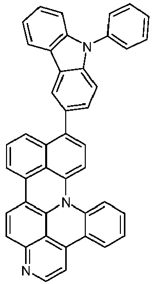 | 73% |
| 12j | 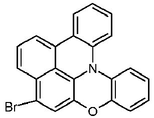 | 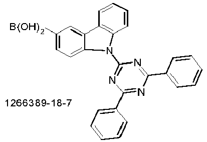 | 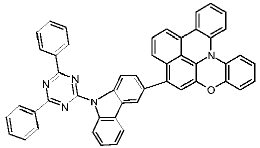 | 72% |
| 13j | 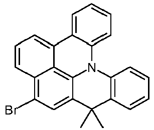 | 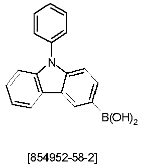 | 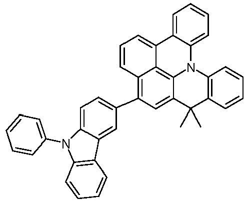 | 76% |
| 14j | 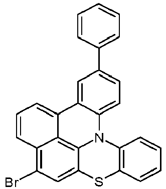 | 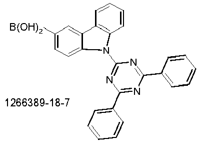 | 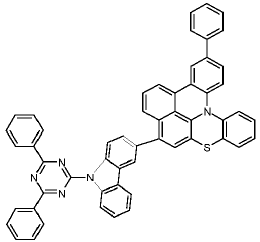 | 73% |
| 15j | 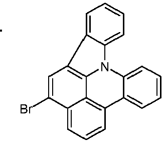 | 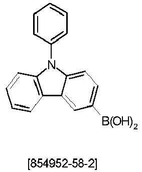 | 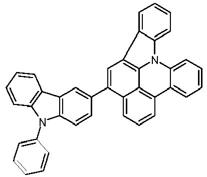 | 78% |
| 16j | 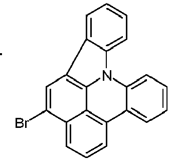 | 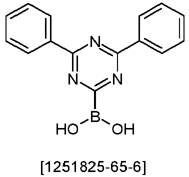 | 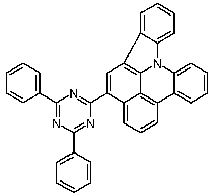 | 81% |
| 17j | 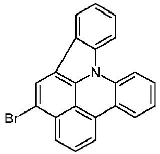 | 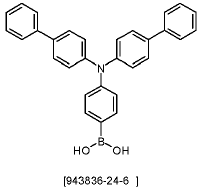 | 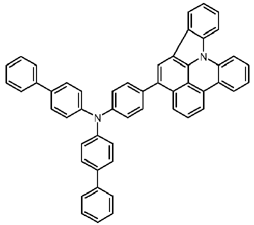 | 76% |
| 18j | 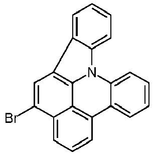 | 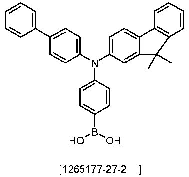 | 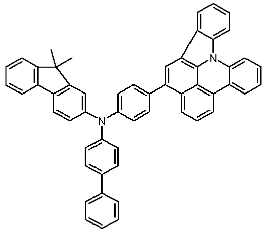 | 75% |
| 19j | 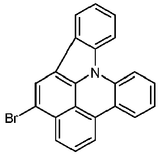 | 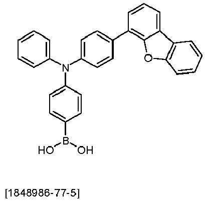 | 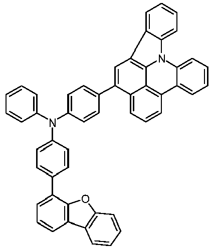 | 70% |
| 20j | 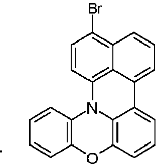 | 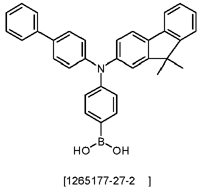 | 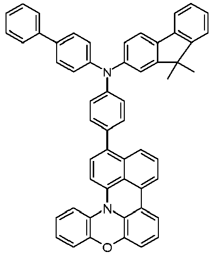 | 76% |

### Herstellung der OLEDs

In den folgenden Beispielen E1 bis E15 (siehe Tabelle 1) wird der Einsatz der erfindungsgemäßen Materialien in OLEDs vorgestellt.

**Vorbehandlung für die Beispiele E1-E15:** Glasplättchen, die mit strukturiertem ITO (Indium Zinn Oxid) der Dicke 50 nm beschichtet sind, werden vor der Beschichtung zunächst mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 2 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC1:EG1:TER1(50%:45%:5%) bedeutet hierbei, dass das Material IC1 in einem Volumenanteil von 50%, EG1 in einem Anteil von 45% und TER1 in einem Anteil von 5% in der Schicht vorliegt. Analog kann auch die Elektronentransportschicht aus einer Mischung von zwei Materialien bestehen.

Die OLEDs werden standardmäßig charakterisiert. Die Elektrolumineszenzspektren werden bei einer Leuchtdichte von 1000 cd/m² bestimmt und daraus die CIE 1931 x und y Farbkoordinaten berechnet.

### Verwendung von erfindungsgemäßen Mischungen in OLEDs

Die erfindungsgemäßen Materialien können in der Emissionsschicht in phosphoreszierenden roten OLEDs eingesetzt werden. Die erfindungsgemäßen Verbindungen EG1 bis EG7 werden in den Beispielen E1 bis E15 als Matrixmaterial in der Emissionsschicht eingesetzt. Die Farbkoordinaten der Elektrolumineszenzspektren der OLEDs liegen bei CIEx=0.67 und CIEy= 0.33. Somit eignen sich die Materialien für den Einsatz in der Emissionsschicht von roten OLEDs.

Des Weiteren lassen sich die erfindungsgemäßen Materialien in der Lochblockierschicht (HBL) oder Elektronenblockierschicht (EBL) erfolgreich einsetzen. Dies ist in den Beispielen E5 und E8 bzw. E2, E10, E13 und E15 gezeigt. Auch hier liegen die Farbkoordinaten des Spektrums der OLED jeweils bei CIEx=0.67 und CIEy= 0.33.

**Tabelle 1: Aufbau der OLEDs**

| Bsp. | HIL Dicke | HTL Dicke | EBL Dicke | EML Dicke | HBL Dicke | ETL Dicke |
|---|---|---|---|---|---|---|
| E1 | HATCN | SpMA1 | SpMA2 | IC1:EG1:TER1 | -- | ST1:LiQ (50%:50%) |
| | 5nm | 125nm | 10nm | (50%:45%:5%) 40nm | | 35nm |
| E2 | HATCN | SpMA1 | EG1 | IC1:EG1:TER1 | -- | ST1:LiQ (50%:50%) |
| | 5nm | 125nm | 10nm | (50%:45%:5%) 40nm | | 35nm |
| E3 | HATCN | SpMA1 | SpMA2 | EG2:TER1 | -- | ST1:LiQ (50%:50%) |
| | 5nm | 125nm | 10nm | (95%:5%) 40nm | | 35nm |
| E4 | HATCN | SpMA1 | SpMA2 | IC2:EG2:TER1 | -- | ST1:LiQ (50%:50%) |
| | 5nm | 125nm | 10nm | (50%:45%:5%) 40nm | | 35nm |
| E5 | HATCN | SpMA1 | SpMA2 | EG2:TER1 | EG2 | ST1:LiQ (50%:50%) |
| | 5nm | 125nm | 10nm | (95%:5%) 40nm | 5nm | 35nm |
| E6 | HATCN | SpMA1 | SpMA2 | EG3:TER1 | -- | ST1:LiQ (50%:50%) |
| | 5nm | 125nm | 10nm | (95%:5%) 40nm | | 35nm |
| E7 | HATCN | SpMA1 | SpMA2 | IC2:EG3:TER1 | -- | ST1:LiQ (50%:50%) |
| | 5nm | 125nm | 10nm | (50%:45%:5%) 40nm | | 35nm |
| E8 | HATCN | SpMA1 | SpMA2 | EG3:TER1 | EG3 | ST1:LiQ (50%:50%) |
| | 5nm | 125nm | 10nm | (95%:5%) 40nm | 5nm | 35nm |
| E9 | HATCN | SpMA1 | SpMA2 | IC1:EG4:TER1 | -- | ST1:LiQ (50%:50%) |
| | 5nm | 125nm | 10nm | (50%:45%:5%) 40nm | | 35nm |
| E10 | HATCN | SpMA1 | EG4 | IC1:EG4:TER1 | -- | ST1:LiQ (50%:50%) |
| | 5nm | 125nm | 10nm | (50%:45%:5%) 40nm | | 35nm |
| E11 | HATCN | SpMA1 | SpMA2 | EG5:TER1 | -- | ST1:LiQ (50%:50%) |
| | 5nm | 125nm | 10nm | (95%:5%) 40nm | | 35nm |
| E12 | HATCN | SpMA1 | SpMA2 | IC1:EG5:TER1 | -- | ST1:LiQ (50%:50%) |
| | 5nm | 125nm | 10nm | (50%:45%:5%) 40nm | | 35nm |
| E13 | HATCN | SpMA1 | EG1 | IC1:EG6:TER1 | -- | ST1:LiQ (50%:50%) |
| | 5nm | 125nm | 10nm | (50%:45%:5%) 40nm | | 35nm |
| E14 | HATCN | SpMA1 | SpMA2 | IC1:EG7:TER1 | -- | ST1:LiQ (50%:50%) |
| | 5nm | 125nm | 10nm | (50%:45%:5%) 40nm | | 35nm |
| E15 | HATCN | SpMA1 | EG1 | IC1:EG7:TER1 | -- | ST1:LiQ (50%:50%) |
| | 5nm | 125nm | 10nm | (50%:45%:5%) 40nm | | 35nm |

**Tabelle 2: Strukturformeln der Materialien für die OLEDs**

| | |
|---|---|
| 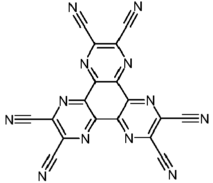 | 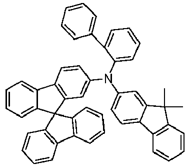 |
| HATCN | SpMA1 |
| 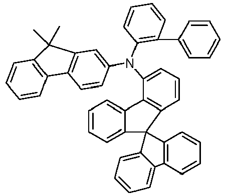 | 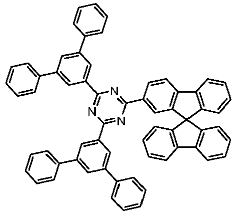 |
| SpMA2 | ST1 |
| 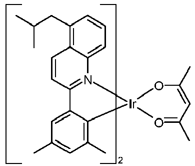 | 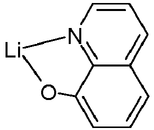 |
| TER1 | LiQ |
| 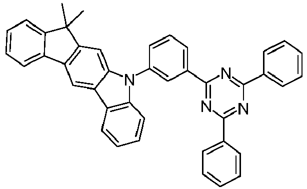 | 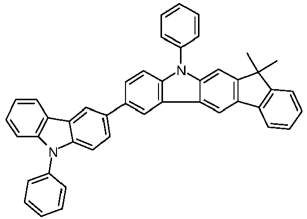 |
| IC1 | IC2 |
| 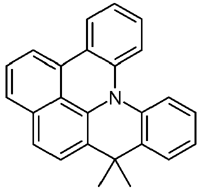 | 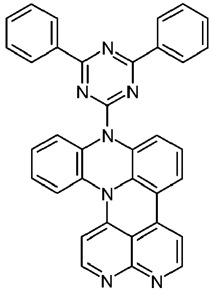 |
| EG1 | EG2 |
| 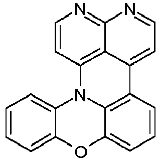 | 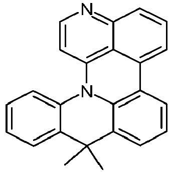 |
| EG3 | EG4 |
| 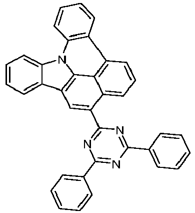 | 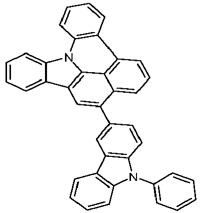 |
| EG5 | EG6 |
| 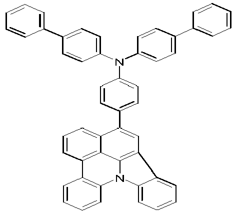 | |
| EG7 | |

## Patentansprüche

1. Verbindung gemäß Formel (1), 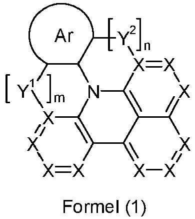 wobei für die verwendeten Symbole und Indizes gilt:
X ist bei jedem Auftreten gleich oder verschieden CR oder N, wobei X = C ist, wenn an dieses X eine Gruppe Y¹ bzw. Y² gebunden ist, mit der Maßgabe, dass kein, ein oder zwei Symbole X für N stehen;
Ar ist zusammen mit den explizit eingezeichneten Kohlenstoffatomen eine Aryl- oder Heteroarylgruppe mit 5 bis 14 aromatischen Ring-atomen, die durch einen oder mehrere Reste R substituiert sein kann;
Y¹ ist C(R')₂, NR', O, S oder BR';
Y² ist eine Einfachbindung, C(R')₂, C(=C(R")₂), NR', O, S oder BR';
R ist bei jedem Auftreten gleich oder verschieden H, D, F, N(Ar')₂, CN, OR¹, eine geradkettige Alkylgruppe mit 1 bis 10 C-Atomen oder eine Alkenylgruppe mit 2 bis 10 C-Atomen oder eine verzweigte oder cyclische Alkylgruppe mit 3 bis 10 C-Atomen, wobei die Alkyl- oder Alkenylgruppe jeweils mit einem oder mehreren Resten R¹ substituiert sein kann und wobei eine oder mehrere nicht benachbarte CH₂-Gruppen durch O ersetzt sein können, oder ein aromatisches oder heteroaromatisches Ringsystem mit 6 bis 30 aromatischen Ringatomen, das jeweils durch einen oder mehrere Reste R¹ substituiert sein kann;
R' ist für Y¹ bzw. Y² = NR' ein aromatisches oder heteroaromatisches Ringsystem mit 6 bis 24 aromatischen Ringatomen, das durch einen oder mehrere nicht-aromatische Reste R¹ substituiert sein kann; und ist für Y¹ bzw. Y² = C(R')₂ bei jedem Auftreten gleich oder verschieden eine geradkettige Alkylgruppe mit 1 bis 6 C-Atomen oder eine verzweigte oder cyclische Alkylgruppe mit 3 bis 6 C-Atomen oder ein aromatisches oder heteroaromatisches Ringsystem mit 6 bis 24 aromatischen Ringatomen, das jeweils durch einen oder mehrere Reste R¹ substituiert sein kann; dabei können zwei Reste R' auch miteinander ein aromatisches oder aliphatisches Ringsystem bilden;
R" ist für Y² = C(=C(R")₂) einmal H und das andere R" steht für CR¹=CR¹ und bildet zusammen mit Ar ein aromatisches oder heteroaromatisches Ringsystem;
Ar' ist bei jedem Auftreten gleich oder verschieden ein aromatisches oder heteroaromatisches Ringsystem mit 5 bis 40 aromatischen Ringatomen, das durch einen oder mehrere Reste R¹ substituiert sein kann;
R¹ ist bei jedem Auftreten gleich oder verschieden H, D, F, CN, OR², eine geradkettige Alkylgruppe mit 1 bis 10 C-Atomen oder eine Alkenylgruppe mit 2 bis 10 C-Atomen oder eine verzweigte oder cyclische Alkylgruppe mit 3 bis 10 C-Atomen, wobei die Alkyl- oder Alkenylgruppe jeweils mit einem oder mehreren Resten R² substituiert sein kann, wobei eine oder mehrere nicht benachbarte CH₂-Gruppen durch O ersetzt sein können, oder ein aromatisches oder heteroaromatisches Ringsystem mit 6 bis 30 aromatischen Ring-atomen, das jeweils durch einen oder mehrere Reste R² substituiert sein kann;
R² ist bei jedem Auftreten gleich oder verschieden H, eine Alkylgruppe mit 1 bis 4 C-Atomen oder eine Arylgruppe mit 6 bis 10 C-Atomen, die mit einer Alkylgruppe mit 1 bis 4 C-Atomen substituiert sein kann;
m, n ist unahbängig voneinander 0 oder 1 mit der Maßgabe, dass m + n = 1 ist; dabei bedeutet m = 0, dass die Gruppe Y¹ nicht vorhanden ist und an das Kohlenstoffatom in Ar, an das Y¹ gebunden wäre, ein Rest R gebunden ist; weiterhin bedeutet n = 0, dass die Gruppe Y² nicht vorhanden ist und an das Kohlenstoffatom in Ar, an das Y² gebunden wäre, ein Rest R gebunden ist;
dabei sind die folgenden Verbindungen von der Erfindung ausgenommen:

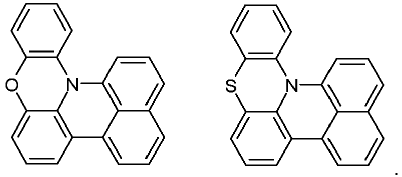

2. Verbindung nach Anspruch 1 gemäß einer der Formeln (2a), (2b), (2c), (2d), (2e), (2f), (2g) oder (2h), 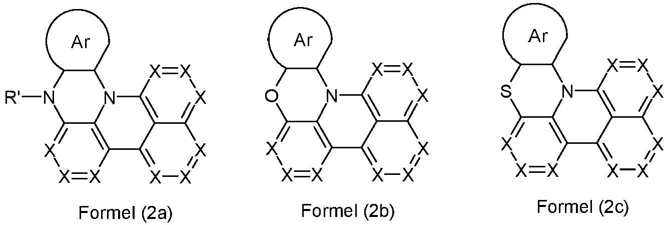 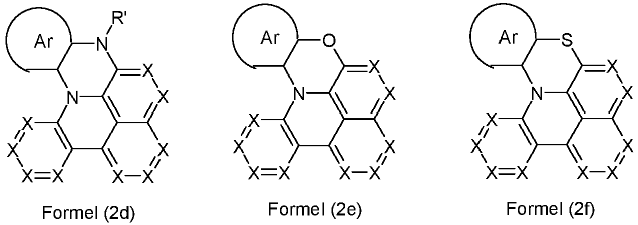 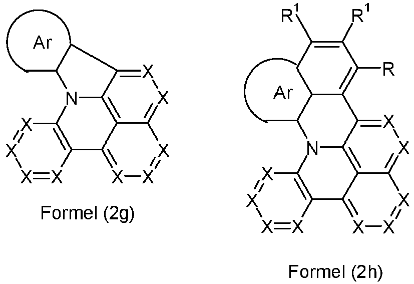 wobei die verwendeten Symbole die in Anspruch 1 genannten Bedeutungen aufweisen.

3. Verbindung nach Anspruch 1 oder 2, **dadurch gekennzeichnet, dass** Ar ausgewählt ist aus den Gruppen gemäß Formel (Ar-a), (Ar-b) oder (Ar-c), 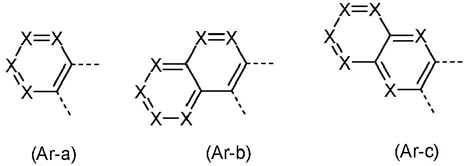 wobei eine der gestrichelten Bindungen die Bindung an das Stickstoffatom darstellt und die andere gestrichelte Bindung die Bindung an Y¹ bzw. Y² darstellt und X ist bei jedem Auftreten gleich oder verschieden CR oder N, wobei maximal ein Symbol X für N steht.

4. Verbindung nach einem oder mehreren der Ansprüche 1 bis 3 gemäß Formel (3), 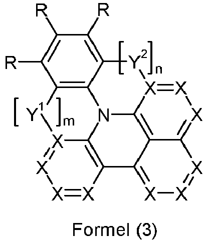 wobei die verwendeten Symbole und Indizes die in Anspruch 1 genannten Bedeutungen aufweisen und wobei an der Position, an der Y¹ bzw. Y² gebunden wäre, für m = 0 bzw. n = 0 ein Rest R gebunden ist.

5. Verbindung nach einem oder mehreren der Ansprüche 1 bis 4, gewählt aus den Verbindungen der Formeln (4a) bis (4h), 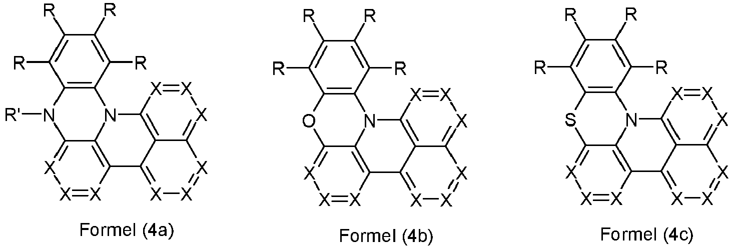 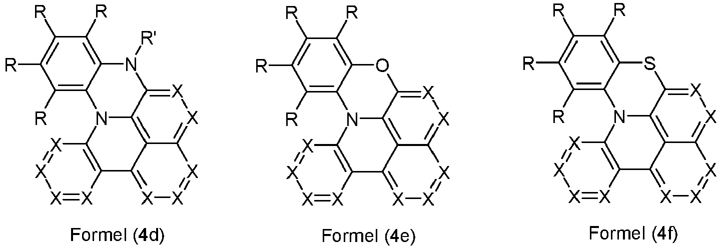 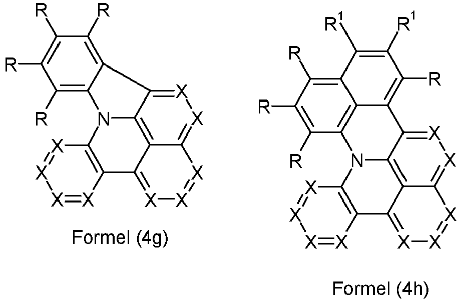 wobei die verwendeten Symbole die in Anspruch 1 genannten Bedeutungen aufweisen.

6. Verbindung nach einem oder mehreren der Ansprüche 1 bis 5 gewählt aus den Verbindungen der Formeln (5a) bis (5u), 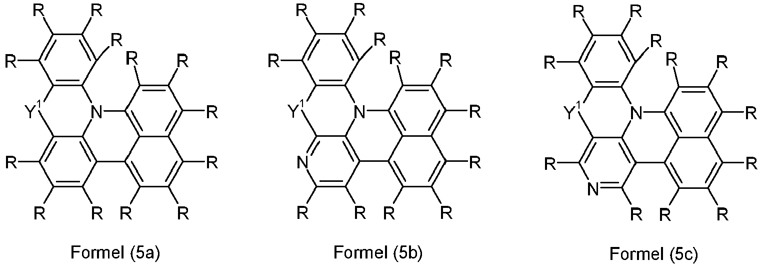 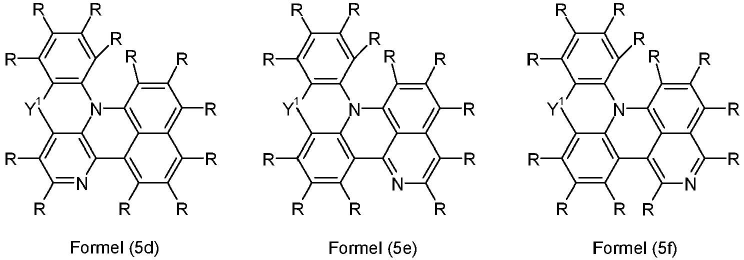 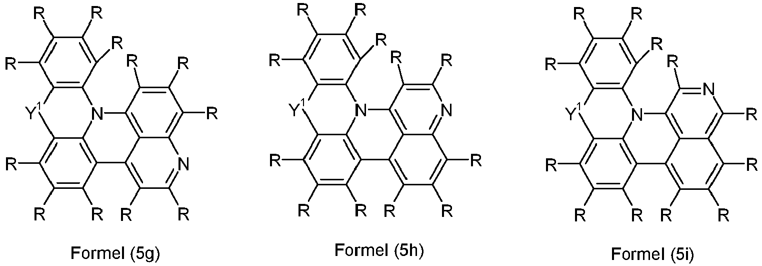 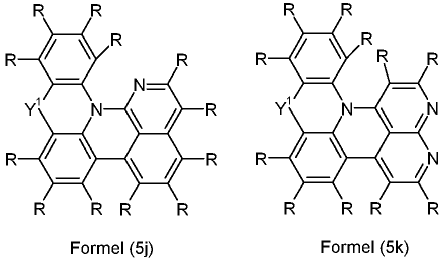 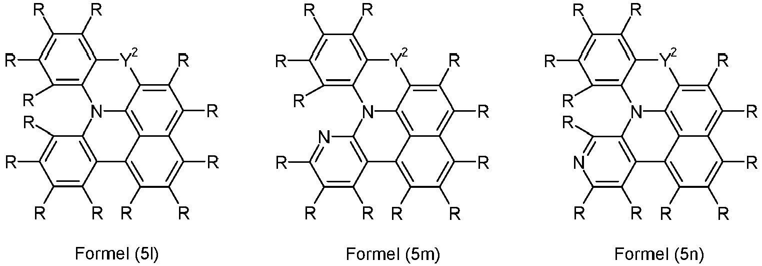 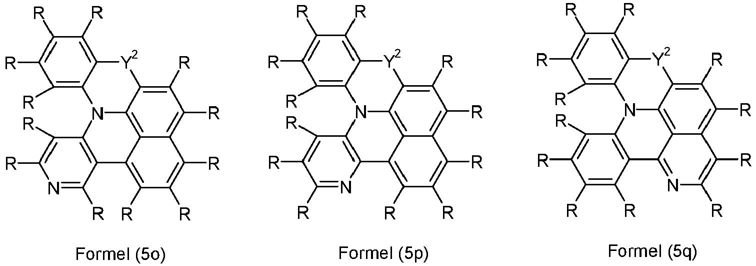 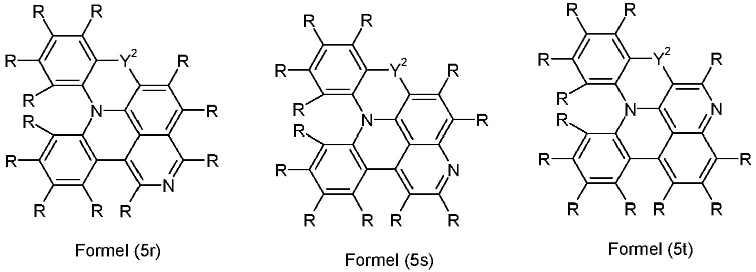 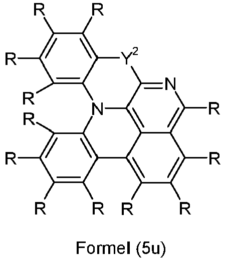 wobei die verwendeten Symbole die in Anspruch 1 genannten Bedeutungen aufweisen.

7. Verbindung nach einem oder mehreren der Ansprüche 1 bis 6, gewählt aus den Verbindungen der Formeln (6a) bis (6u), 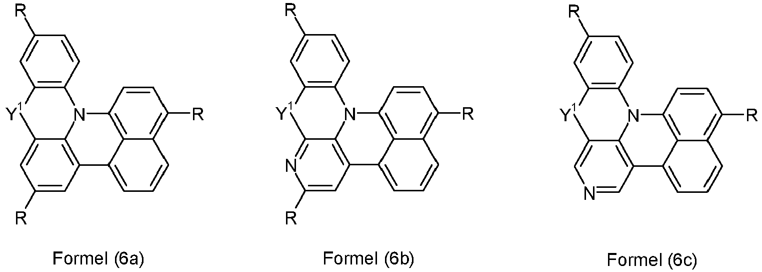 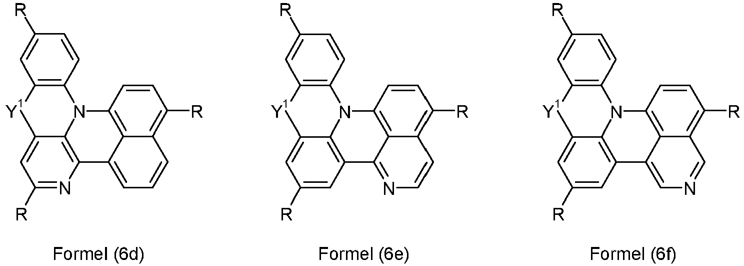 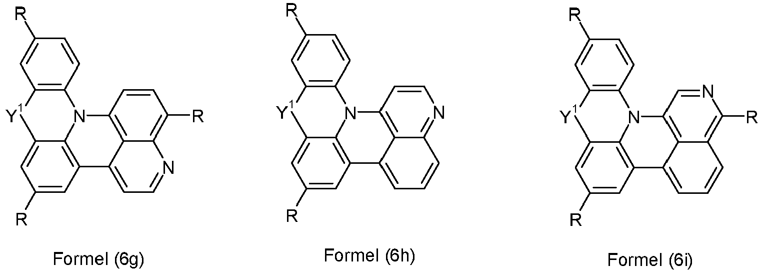 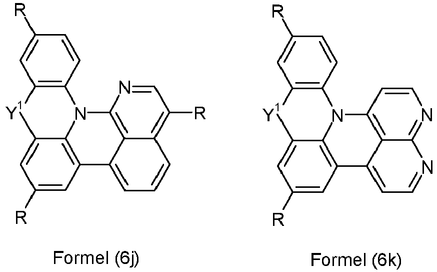 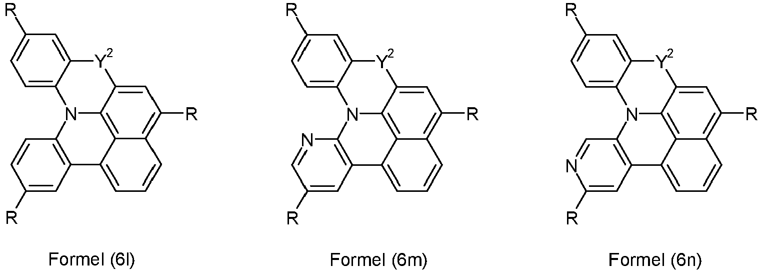 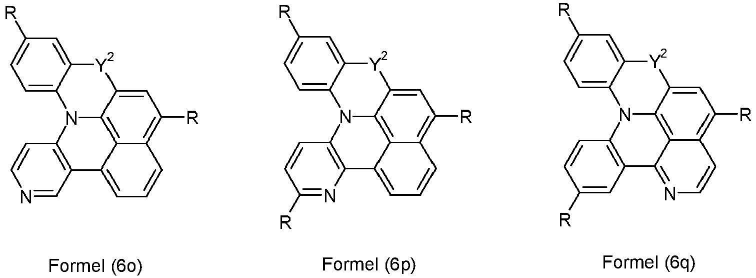 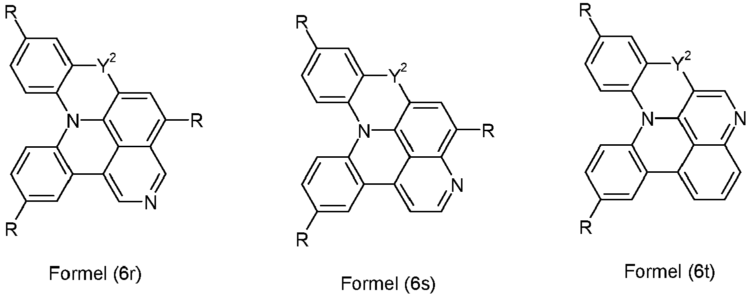 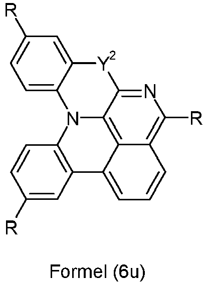 wobei die verwendeten Symbole die in Anspruch 1 genannten Bedeutungen aufweisen.

8. Verbindung nach einem oder mehreren der Ansprüche 1 bis 7, gewählt aus den Verbindungen der Formeln (7a) bis (7h), 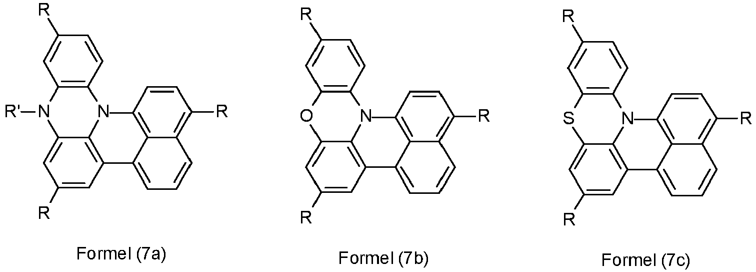 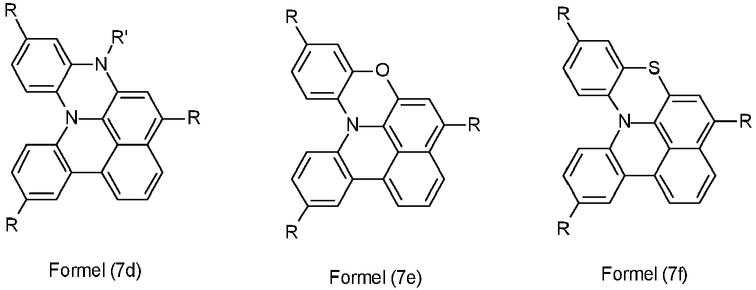 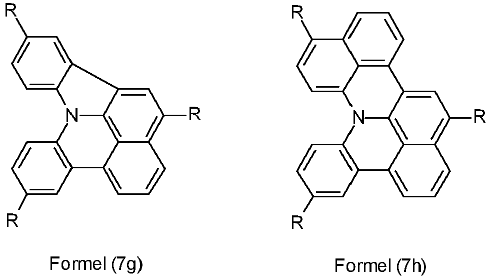 wobei die verwendeten Symbole die in Anspruch 1 genannten Bedeutungen aufweisen.

9. Verbindung nach einem oder mehreren der Ansprüche 1 bis 8, **dadurch gekennzeichnet, dass** die Verbindung mindestens einen Substituenten R enthält, der ausgewählt ist aus einem aromatischen oder heteroaromatischen Ringsystem mit 6 bis 30 aromatischen Ringatomen, welches durch einen oder mehrere Reste R¹ substituiert sein kann, oder N(Ar')₂, und/oder dass die Verbindung mindestens eine Gruppe Y¹ bzw. Y² enthält, die für NR' steht, wobei R' ausgewählt ist aus einem aromatischen oder heteroaromatischen Ringsystem mit 6 bis 30 aromatischen Ringatomen, welches mit einem oder mehreren Resten R¹ substituiert sein kann.

10. Formulierung, enthaltend eine Verbindung nach einem oder mehreren der Ansprüche 1 bis 9 und mindestens ein Lösemittel und/oder mindestens eine weitere organische oder anorganische Verbindung.

11. Verwendung einer Verbindung nach einem oder mehreren der Ansprüche 1 bis 9 in einer elektronischen Vorrichtung.

12. Elektronische Vorrichtung enthaltend mindestens eine Verbindung nach einem oder mehreren der Ansprüche 1 bis 9.

13. Elektronische Vorrichtung nach Anspruch 12, wobei es sich um eine organische Elektrolumineszenzvorrichtung handelt, **dadurch gekennzeichnet, dass** die Verbindung nach einem oder mehreren der Ansprüche 1 bis 11 eingesetzt wird in einer emittierenden Schicht als Matrixmaterial für phosphoreszierende oder fluoreszierende Emitter oder für Emitter, die TADF zeigen, oder in einer Elektronentransportschicht und/oder in einer Lochtransportschicht und/oder in einer Exzitonenblockierschicht und/oder in einer Lochblockierschicht.

## Claims

1. Compound of the formula (1), 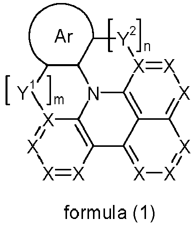 where the following applies for the symbols and indices used:
X is on each occurrence, identically or differently, CR or N, where X = C if a group Y¹ or Y² is bonded to this X, with the proviso that no, one or two symbols X stand for N;
Ar, together with the carbon atoms explicitly drawn in, is an aryl or heteroaryl group having 5 to 14 aromatic ring atoms, which may be substituted by one or more radicals R;
Y¹ is C(R')₂, NR', O, S or BR';
Y² is a single bond, C(R')₂, C(=C(R")₂), NR', O, S or BR';
R is on each occurrence, identically or differently, H, D, F, N(Ar')₂, CN, OR¹, a straight-chain alkyl group having 1 to 10 C atoms or an alkenyl group having 2 to 10 C atoms or a branched or cyclic alkyl group having 3 to 10 C atoms, where the alkyl or alkenyl group may in each case be substituted by one or more radicals R¹ and where one or more non-adjacent CH₂ groups may be replaced by O, or an aromatic or heteroaromatic ring system having 6 to 30 aromatic ring atoms, which may in each case be substituted by one or more radicals R¹;
R', for Y¹ or Y² = NR', is an aromatic or heteroaromatic ring system having 6 to 24 aromatic ring atoms, which may be substituted by one or more non-aromatic radicals R¹; and, for Y¹ or Y² = C(R')₂, is on each occurrence, identically or differently, a straight-chain alkyl group having 1 to 6 C atoms or a branched or cyclic alkyl group having 3 to 6 C atoms or an aromatic or heteroaromatic ring system having 6 to 24 aromatic ring atoms, which may in each case be substituted by one or more radicals R¹; two radicals R' may also form an aromatic or aliphatic ring system with one another;
R", for Y² = C(=C(R")₂), is H once and the other R" stands for CR¹=CR¹ and, together with Ar, forms an aromatic or heteroaromatic ring system;
Ar' is on each occurrence, identically or differently, an aromatic or heteroaromatic ring system having 5 to 40 aromatic ring atoms, which may be substituted by one or more radicals R¹;
R¹ is on each occurrence, identically or differently, H, D, F, CN, OR², a straight-chain alkyl group having 1 to 10 C atoms or an alkenyl group having 2 to 10 C atoms or a branched or cyclic alkyl group having 3 to 10 C atoms, where the alkyl or alkenyl group may in each case be substituted by one or more radicals R², where one or more non-adjacent CH₂ groups may be replaced by O, or an aromatic or heteroaromatic ring system having 6 to 30 aromatic ring atoms, which may in each case be substituted by one or more radicals R²;
R² is on each occurrence, identically or differently, H, an alkyl group having 1 to 4 C atoms or an aryl group having 6 to 10 C atoms, which may be substituted by alkyl group having 1 to 4 C atoms;
m, n are, independently of one another, 0 or 1, with the proviso that m + n = 1; m = 0 means that the group Y¹ is not present and a radical R is bonded to the carbon atom in Ar to which Y¹ would be bonded; furthermore, n = 0 means that the group Y² is not present and a radical R is bonded to the carbon atom in Ar to which Y² would be bonded;
the following compounds are excluded from the invention:

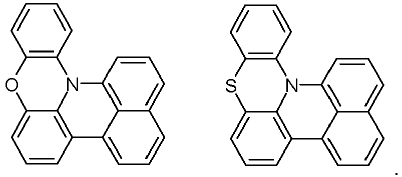

2. Compound according to Claim 1 of one of the formulae (2a), (2b), (2c), (2d), (2e), (2f), (2g) or (2h), 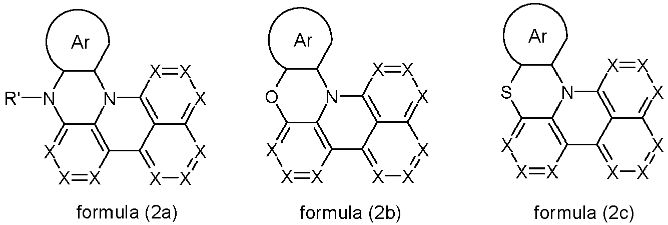 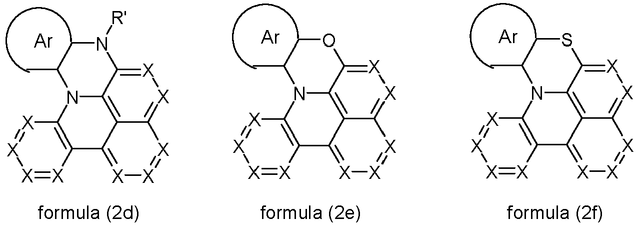 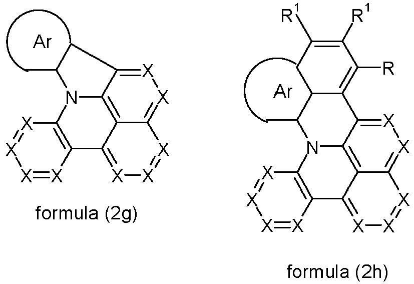 where the symbols used have the meanings given in Claim 1.

3. Compound according to Claim 1 or 2, **characterised in that** Ar is selected from the groups of the formula (Ar-a), (Ar-b) or (Ar-c), 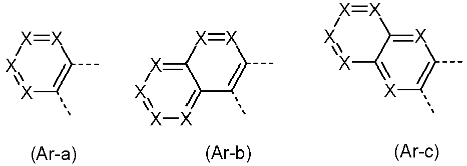 where one of the dashed bonds represents the bond to the nitrogen atom and the other dashed bond represents the bond to Y¹ or Y² and X is on each occurrence, identically or differently, CR or N, where a maximum of one symbol X stands for N.

4. Compound according to one or more of Claims 1 to 3 of the formula (3), 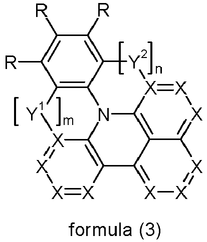 where the symbols and indices used have the meanings given in Claim 1 and where, for m = 0 or n = 0, a radical R is bonded at the position at which Y¹ or Y² respectively would be bonded.

5. Compound according to one or more of Claims 1 to 4, selected from the compounds of the formulae (4a) to (4h), 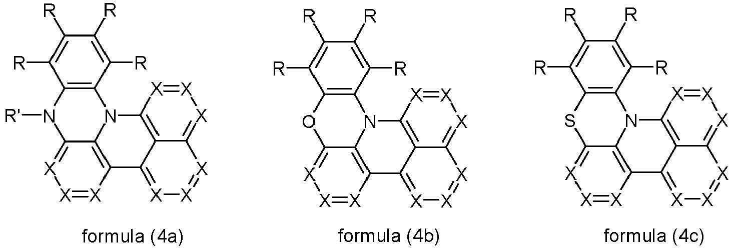 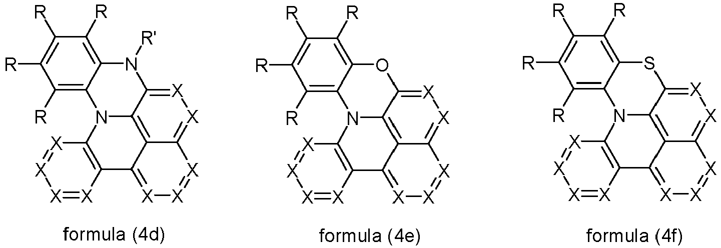 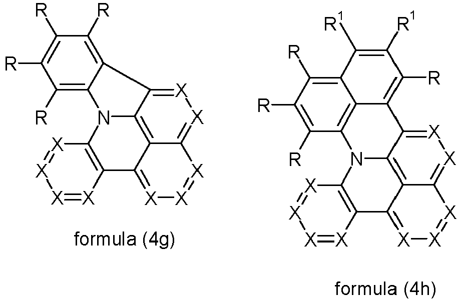 where the symbols used have the meanings given in Claim 1.

6. Compound according to one or more of Claims 1 to 5, selected from the compounds of the formulae (5a) to (5u), 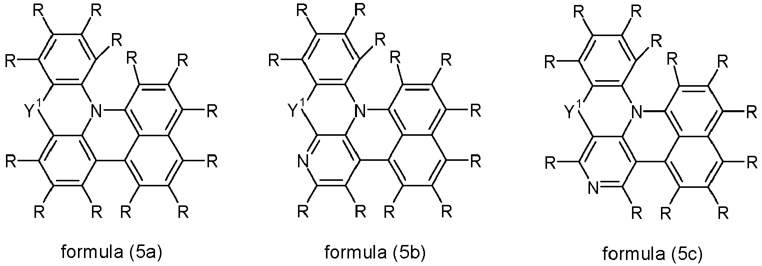 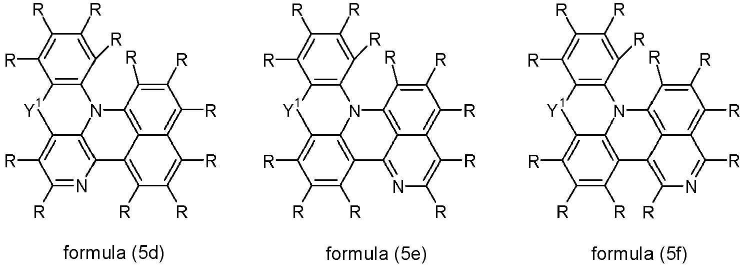 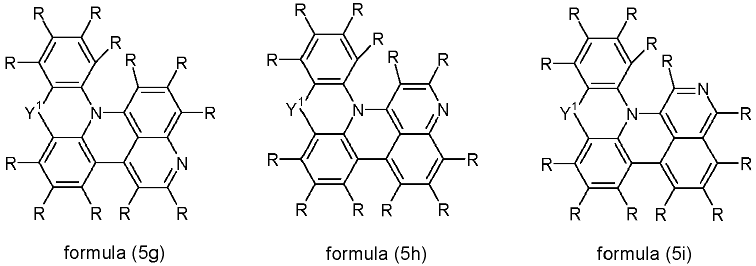 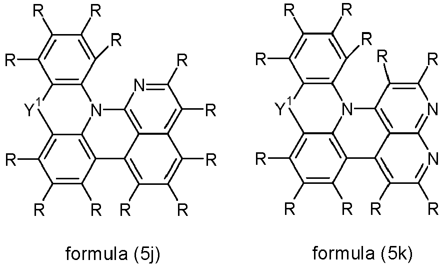 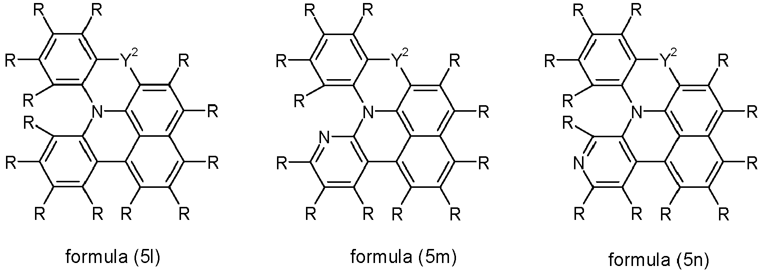 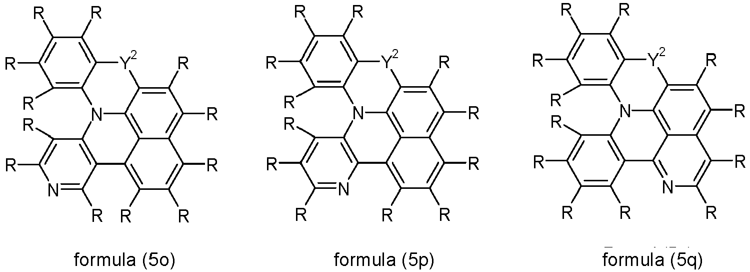 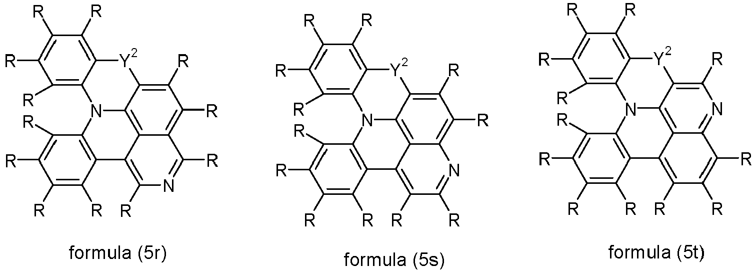 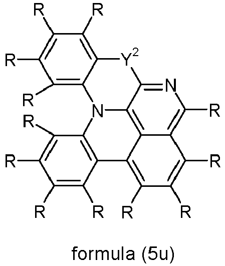 where the symbols used have the meanings given in Claim 1.

7. Compound according to one or more of Claims 1 to 6, selected from the compounds of the formulae (6a) to (6u), 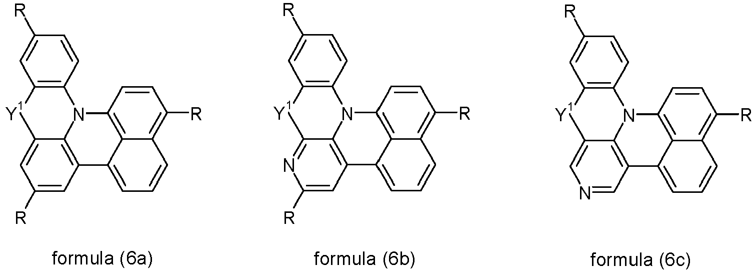 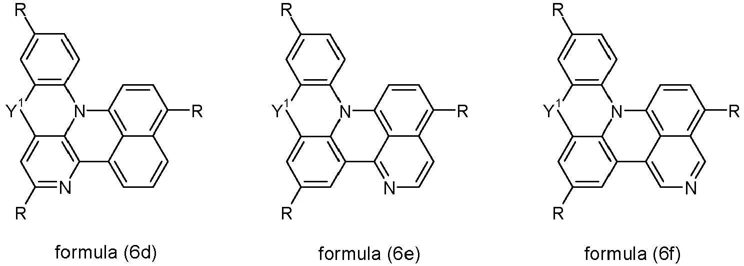 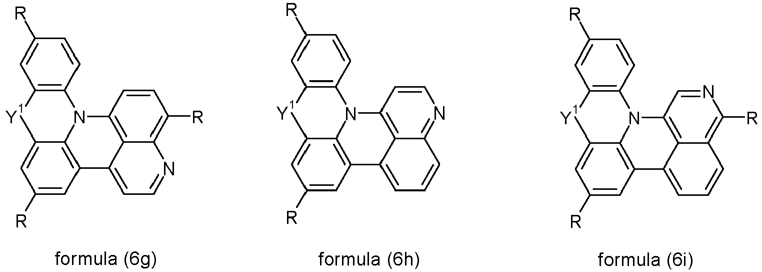 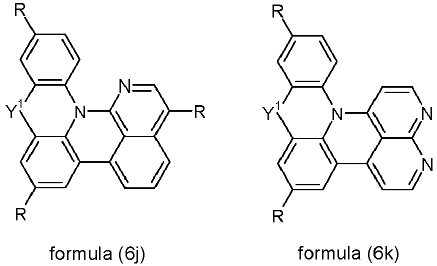 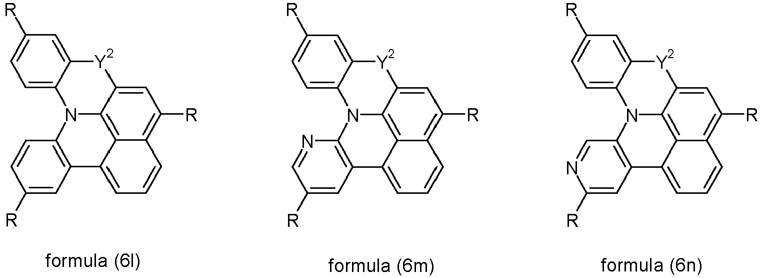 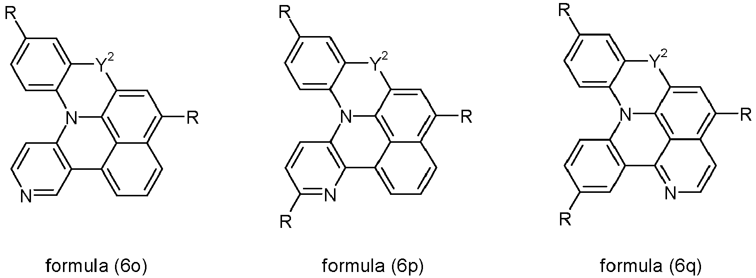 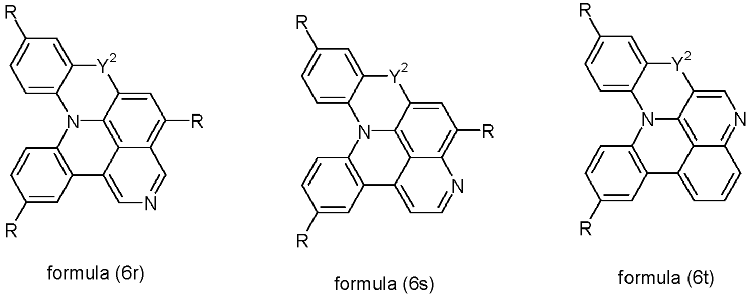 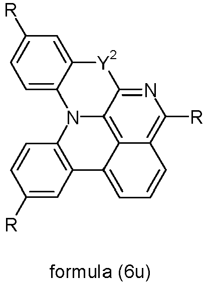 where the symbols used have the meanings given in Claim 1.

8. Compound according to one or more of Claims 1 to 7, selected from the compounds of the formulae (7a) to (7h), 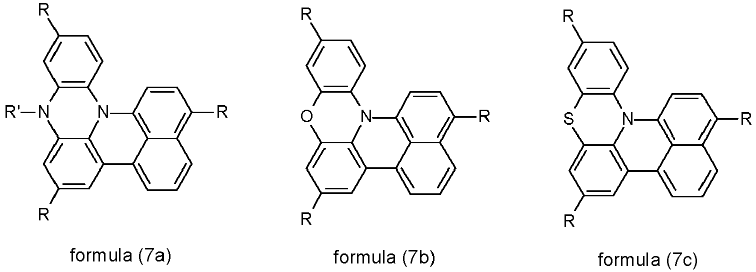 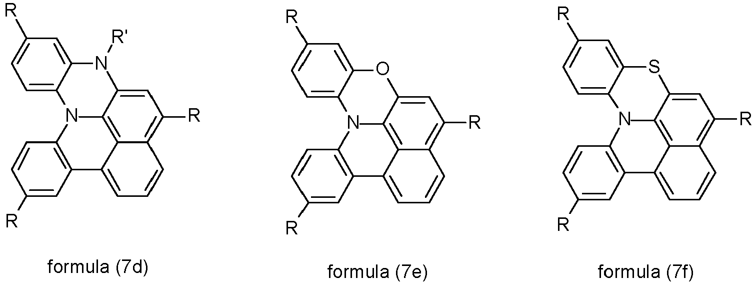 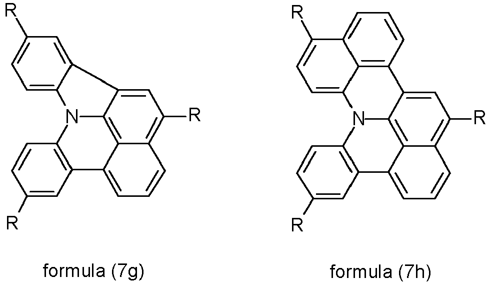 where the symbols used have the meanings given in Claim 1.

9. Compound according to one or more of Claims 1 to 8, **characterised in that** the compound contains at least one substituent R selected from an aromatic or heteroaromatic ring system having 6 to 30 aromatic ring atoms, which may be substituted by one or more radicals R¹, or N(Ar')₂, and/or **in that** the compound contains at least one group Y¹ or Y² that stands for NR', where R' is selected from an aromatic or heteroaromatic ring system having 6 to 30 aromatic ring atoms, which may be substituted by one or more radicals R¹.

10. Formulation comprising a compound according to one or more of Claims 1 to 9 and at least one solvent and/or at least one further organic or inorganic compound.

11. Use of a compound according to one or more of Claims 1 to 9 in an electronic device.

12. Electronic device containing at least one compound according to one or more of Claims 1 to 9.

13. Electronic device according to Claim 12, which is an organic electroluminescent device, **characterised in that** the compound according to one or more of Claims 1 to 11 is employed in an emitting layer as matrix material for phosphorescent emitters or for emitters which exhibit TADF, or in an electron-transport layer and/or in a hole-transport layer and/or in an exciton-blocking layer and/or in a hole-blocking layer.

## Revendications

1. Composé de formule (1), 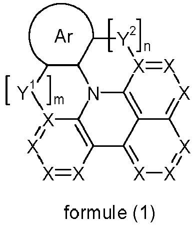 dans laquelle ce qui suit s'applique aux symboles et indices utilisés :
X est à chaque occurrence, de manière identique ou différente, CR ou N, où X = C si un groupement Y¹ ou Y² est lié à ce X, à condition qu'aucun, un ou deux symboles X représentent N ;
Ar, conjointement avec les atomes de carbone explicitement dessinés, est un groupement aryle ou hétéroaryle ayant de 5 à 14 atomes de cycle aromatique, qui peut être substitué par un ou plusieurs radicaux R ;
Y¹ est C(R')₂, NR', O, S ou BR' ;
Y² est une liaison simple, C(R')₂, C(=C(R")₂), NR', O, S ou BR' ;
R est à chaque occurrence, de manière identique ou différente, H, D, F, N(Ar')₂, CN, OR¹, un groupement alkyle à chaîne linéaire ayant de 1 à 10 atomes de C ou un groupement alcényle ayant de 2 à 10 atomes de C ou un groupement alkyle ramifié ou cyclique ayant de 3 à 10 atomes de C, où le groupement alkyle ou alcényle peut dans chaque cas être substitué par un ou plusieurs radicaux R¹ et où un ou plusieurs groupements CH₂ non adjacents peuvent être remplacés par O, ou un noyau aromatique ou hétéroaromatique ayant de 6 à 30 atomes de cycle aromatique, qui peut dans chaque cas être substitué par un ou plusieurs radicaux R¹ ;
R', pour Y¹ ou Y² = NR', est un noyau aromatique ou hétéroaromatique ayant de 6 à 24 atomes de cycle aromatique, qui peut être substitué par un ou plusieurs radicaux non aromatiques R¹ ; et, pour Y¹ ou Y² = C(R')₂, est à chaque occurrence, de manière identique ou différente, un groupement alkyle à chaîne linéaire ayant de 1 à 6 atomes de C ou un groupement alkyle ramifié ou cyclique ayant de 3 à 6 atomes de C ou un noyau aromatique ou hétéroaromatique ayant de 6 à 24 atomes de cycle aromatique, qui peut dans chaque cas être substitué par un ou plusieurs radicaux R¹ ; deux radicaux R' peuvent également former un noyau aromatique ou aliphatique l'un avec l'autre ;
R", pour Y² = C(=C(R")₂), est H une fois et les autres R" représentent CR¹=CR¹ et, conjointement avec Ar, forme un noyau aromatique ou hétéroaromatique ;
Ar' est à chaque occurrence, de manière identique ou différente, un noyau aromatique ou hétéroaromatique ayant de 5 à 40 atomes de cycle aromatique, qui peut être substitué par un ou plusieurs radicaux R¹ ;
R¹ est à chaque occurrence, de manière identique ou différente, H, D, F, CN, OR², un groupement alkyle à chaîne linéaire ayant de 1 à 10 atomes de C ou un groupement alcényle ayant de 2 à 10 atomes de C ou un groupement alkyle ramifié ou cyclique ayant de 3 à 10 atomes de C, où le groupement alkyle ou alcényle peut dans chaque cas être substitué par un ou plusieurs radicaux R², où un ou plusieurs groupements CH₂ non adjacents peuvent être remplacés par 0, ou un noyau aromatique ou hétéroaromatique ayant de 6 à 30 atomes de cycle aromatique, qui peut dans chaque cas être substitué par un ou plusieurs radicaux R² ;
R² est à chaque occurrence, de manière identique ou différente, H, un groupement alkyle ayant de 1 à 4 atomes de C ou un groupement aryle ayant de 6 à 10 atomes de C, qui peut être substitué par un groupement alkyle ayant de 1 à 4 atomes de C ;
m, n valent, indépendamment l'un de l'autre, 0 ou 1, à condition que m + n = 1 ; m = 0 signifie que le groupement Y¹ n'est pas présent et qu'un radical R est lié à l'atome de carbone dans Ar auquel Y¹ serait lié ; en outre, n = 0 signifie que le groupement Y² n'est pas présent et qu'un radical R est lié à l'atome de carbone dans Ar auquel Y² serait lié ;
les composés suivants sont exclus de l'invention :

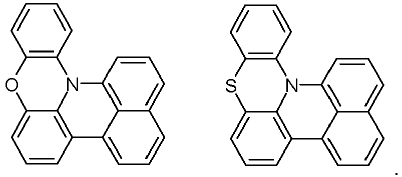

2. Composé selon la revendication 1 répondant à l'une des formules (2a), (2b), (2c), (2d), (2e), (2f), (2g) ou (2h), 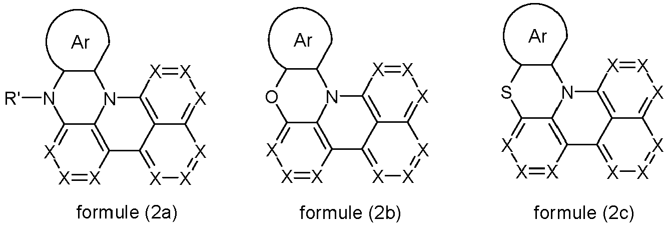 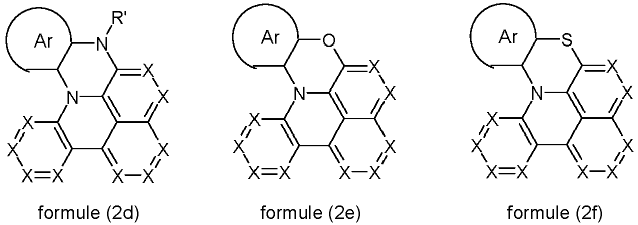 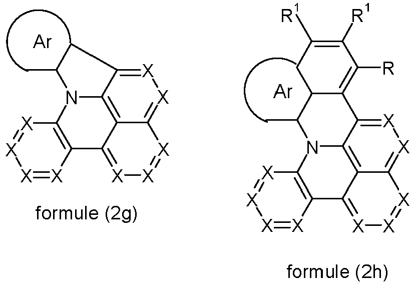 où les symboles utilisés revêtent les significations données selon la revendication 1.

3. Composé selon la revendication 1 ou 2, **caractérisé en ce que** Ar est choisi parmi les groupements de formule (Ar-a), (Ar-b) ou (Ar-c), 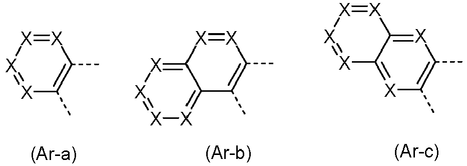 où l'une des lignes en pointillés représente la liaison à l'atome d'azote et l'autre ligne en pointillés représente la liaison à Y¹ ou Y² et X est à chaque occurrence, de manière identique ou différente, CR ou N, où un maximum d'un seul symbole X représente N.

4. Composé selon l'une ou plusieurs parmi les revendications 1 à 3 de formule (3), 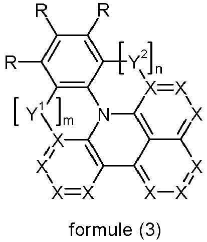 dans laquelle les symboles et indices utilisés revêtent les significations données selon la revendication 1 et où, pour m = 0 ou n = 0, un radical R est lié au niveau de la position à laquelle Y¹ ou Y² respectivement serait lié.

5. Composé selon l'une ou plusieurs parmi les revendications 1 à 4, choisi parmi les composés de formules (4a) à (4h), 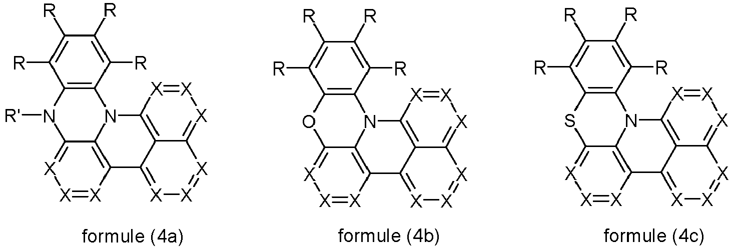 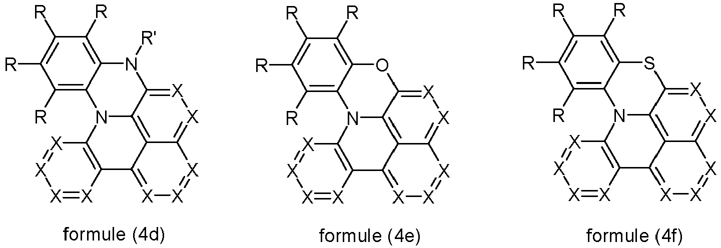 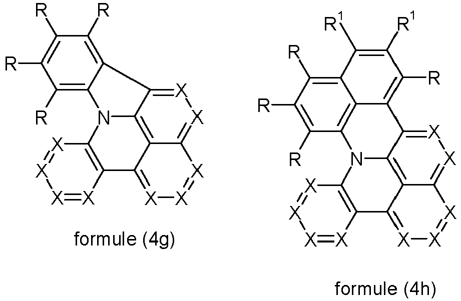 dans lesquelles les symboles utilisés revêtent les significations données selon la revendication 1.

6. Composé selon l'une ou plusieurs parmi les revendications 1 à 5, choisi parmi les composés de formules (5a) à (5u), 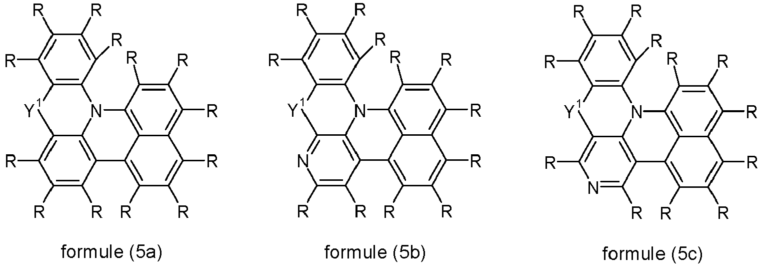 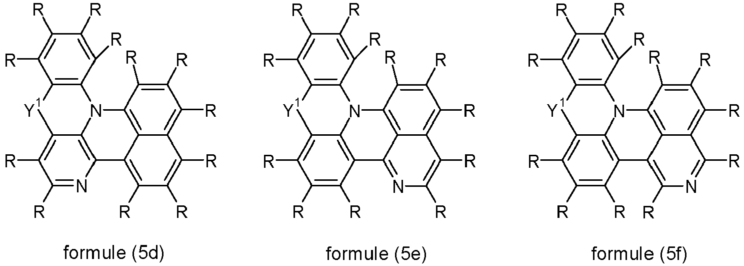 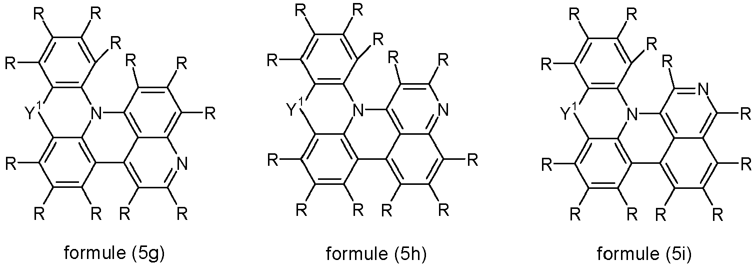 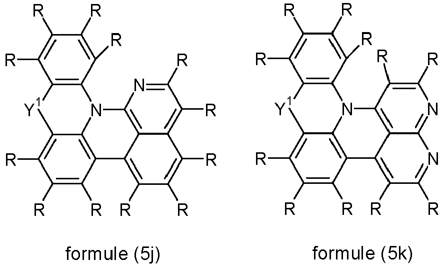 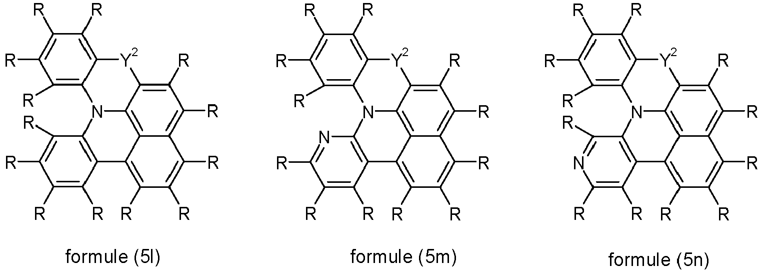 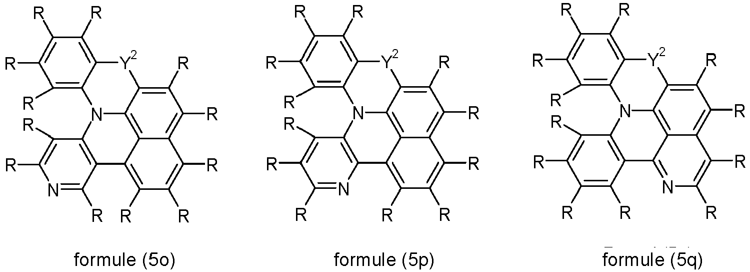 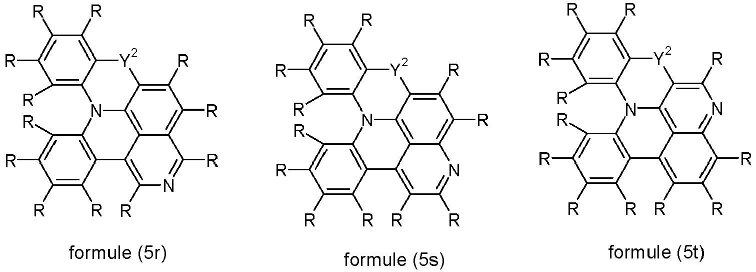 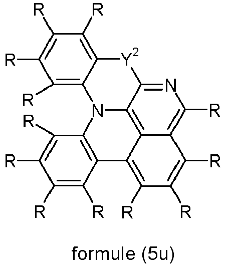 dans lesquelles les symboles utilisés revêtent les significations données selon la revendication 1.

7. Composé selon l'une ou plusieurs parmi les revendications 1 à 6, choisi parmi les composés de formules (6a) à (6u), 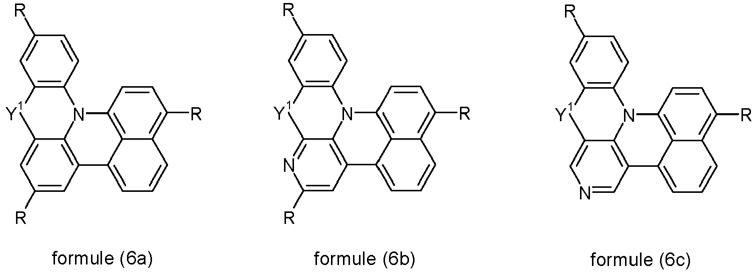 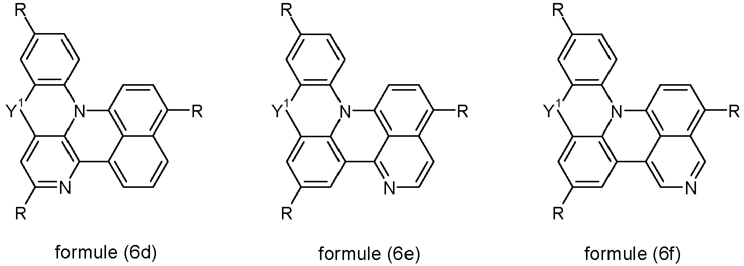 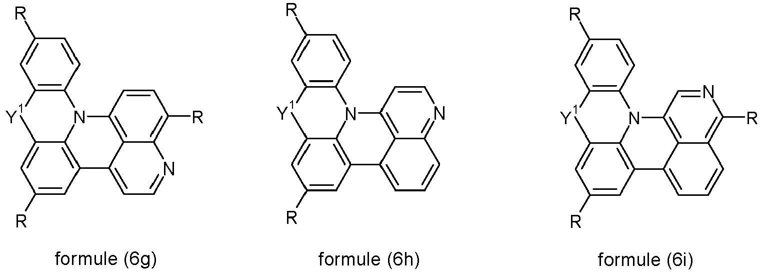 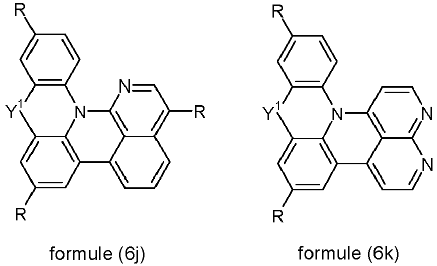 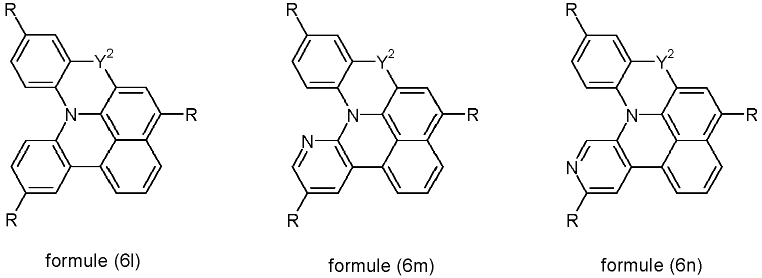 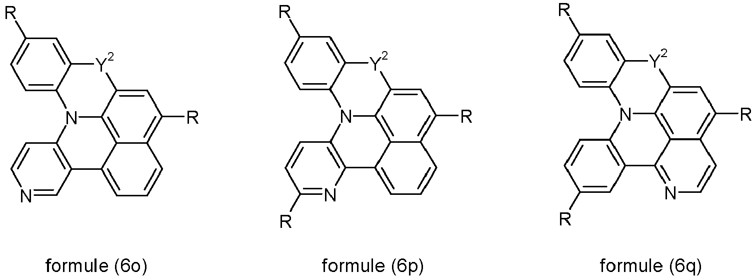 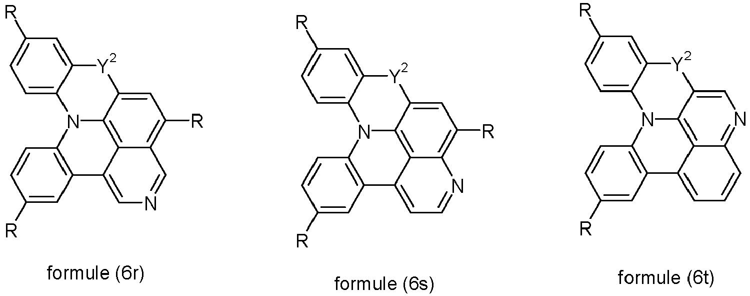 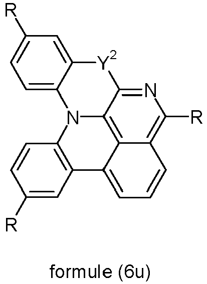 dans lesquelles les symboles utilisés revêtent les significations données selon la revendication 1.

8. Composé selon l'une ou plusieurs parmi les revendications 1 à 7, choisi parmi les composés de formules (7a) à (7h), 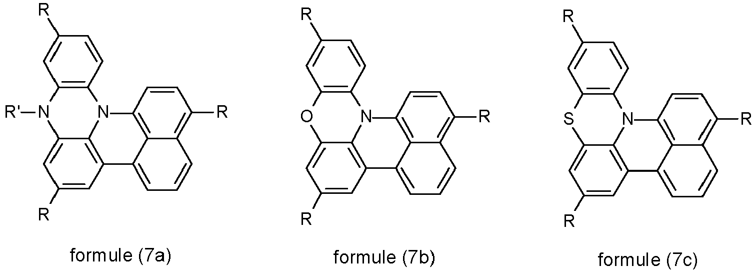 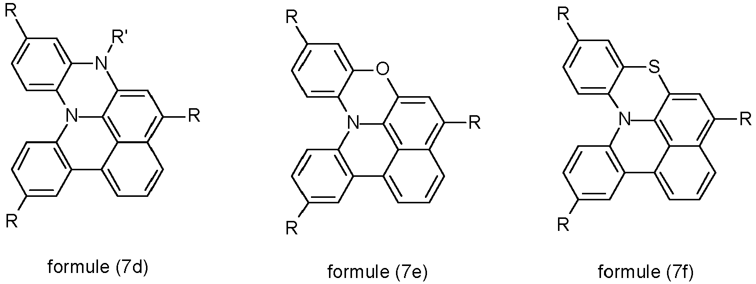 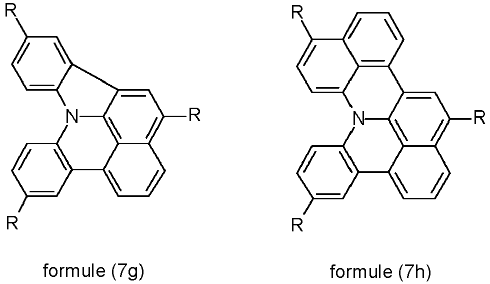 dans lesquelles les symboles utilisés revêtent les significations données selon la revendication 1.

9. Composé selon l'une ou plusieurs parmi les revendications 1 à 8, **caractérisé en ce que** le composé contient au moins un substituant R choisi parmi un noyau aromatique ou hétéroaromatique ayant de 6 à 30 atomes de cycle aromatique, qui peut être substitué par un ou plusieurs radicaux R¹, ou N(Ar')₂, et/ou **en ce que** le composé contient au moins un groupement Y¹ ou Y² qui représente NR', où R' est choisi parmi un noyau aromatique ou hétéroaromatique ayant de 6 à 30 atomes de cycle aromatique, qui peut être substitué par un ou plusieurs radicaux R¹.

10. Formulation comprenant un composé selon l'une ou plusieurs parmi les revendications 1 à 9 et au moins un solvant et/ou au moins un autre composé organique ou inorganique.

11. Utilisation d'un composé selon l'une ou plusieurs parmi les revendications 1 à 9, dans un dispositif électronique.

12. Dispositif électronique contenant au moins un composé selon l'une ou plusieurs parmi les revendications 1 à 9.

13. Dispositif électronique selon la revendication 12, qui est un dispositif électroluminescent organique, **caractérisé en ce que** le composé selon l'une ou plusieurs parmi les revendications 1 à 11 est employé dans une couche émettrice comme matériau de matrice pour des émetteurs phosphorescents ou pour des émetteurs présentant une TADF, ou dans une couche de transport d'électrons et/ou dans une couche de transport de trous et/ou dans une couche de blocage d'excitons et/ou dans une couche de blocage de trous.
